(19) Europäisches Patentamt
European Patent Office
Office européen des brevets

(11) **EP 1 778 296 B1**

(12) **EUROPEAN PATENT SPECIFICATION**

(45) Date of publication and mention
of the grant of the patent:
**06.06.2012 Bulletin 2012/23**

(51) Int Cl.:
*A61K 48/00* (2006.01)      *C07K 19/00* (2006.01)
*C12N 15/62* (2006.01)      *C12N 15/63* (2006.01)

(21) Application number: **05790332.0**

(22) Date of filing: **20.07.2005**

(86) International application number:
**PCT/US2005/025878**

(87) International publication number:
**WO 2006/012416 (02.02.2006 Gazette 2006/05)**

(54) **SPECIFIC INHIBITION OF AUTOIMMUNITY AND DISEASES ASSOCIATED WITH AUTOANTIGENS**

SPEZIFISCHE INHIBITION VON AUTOIMMUNITÄT UND KRANKHEITEN IM ZUSAMMENHANG MIT AUTOANTIGENEN

INHIBITION SPECIFIQUE D'AUTO-IMMUNITE ET MALADIES ASSOCIEES A DES AUTO-ANTIGENES

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.07.2004 US 589707 P**
**29.09.2004 US 614529 P**

(43) Date of publication of application:
**02.05.2007 Bulletin 2007/18**

(73) Proprietor: **Isogenis, Inc.**
**Denver, CO 80206 (US)**

(72) Inventors:
• **QI, Yan**
**Highlands Ranch, CO 80126 (US)**
• **STAERZ, Uwe, D.**
**Denver, CO 80207 (US)**

(74) Representative: **Dzieglewska, Hanna Eva**
**Dehns**
**St Bride's House**
**10 Salisbury Square**
**London**
**EC4Y 8JD (GB)**

(56) References cited:
**WO-A-99/21576      WO-A-99/23229**
**US-A- 5 601 828**

**Description**

FIELD OF THE INVENTION

The present invention relates to immunosuppressive therapy, and more specifically, to methods and compositions for preventing or treating autoimmune disorders.

BACKGROUND OF THE INVENTION

The immune response is surprisingly effective at eliminating microscopic pathogens and, to a lesser extent, neoplastic cells. In general, the complicated mechanisms for self-recognition are efficient and allow a strong response to be directed exclusively at eliminating foreign antigens. The regulation of self/non-self discrimination, which is a critical function of the immune system, involves multiple mechanisms during the development and lifespan of T and B lymphocytes. Unfortunately, the immune system occasionally malfunctions and turns against the cells of the host thereby provoking an autoimmune response. Autoimmunity typically occurs when antigen receptors on immune cells recognize specific self-antigens on host cells and initiate reactions that result in the destruction of the host cells. In some instances, the autoreactive lymphocytes survive longer and continue to induce apoptosis or otherwise eliminate host cells causing autoimmune diseases.

Different mechanisms have been described that prevent T lymphocytes from attacking self. These tolerance mechanisms act both on developing and mature T cells. For example, thymic positive selection skews the T cell repertoire to recognize self-MHC molecules and thus also enriches for auto-reactive T cells (Berg et al., J. Exp. Med. 194: 427-38 (1999)). Thymic negative selection, which follows positive selection, in turn removes auto-reactive T cells either by deletion or inactivation (Berg et al., J. Exp. Med. 194: 427-38 (1999); Zepp et al., Nature 336:473-5 (1988)). Whereas clonal deletion takes care of high affinity T cells at the CD4+CD8+TCR$^{high}$ maturation stage, clonal inactivation seems to work at lower affinity interaction possibly by the down-regulation of the TCR and the CD8 $\alpha$-chain (Berg et al., J. Exp. Med. 194: 427-38 (1999); Jordan et al., Nat. Immunol. 2:301-6 (2001); Stephens et al., Eur. J. Immunol. 33:1282-91 (2003)). However, these "anergic" T lymphocytes still have the ability to specifically respond to their antigen. It has been suggested that they actually might represent a population of regulatory T cells since they release IL-10 and TGF-$\beta$ upon stimulation (Asseman et al., J. Exp. Med. 190:995-1004 (1999); Seddon et al., J. Exp. Med. 189:279-88 (1999)). Thus, it is well-established that thymic (central) tolerance mechanisms do not eliminate all autoreactive T cells. Indeed, T cells reactive with self-antigens, such as myelin basic protein (MBP), insulin and glutamic acid decarboxylase (GAD), can be readily found in the periphery.

Both CD4+ helper T cells as well as CD8+ cytotoxic T cells (CTLs) play important roles in the autoantigen immune response. In the well-accepted NOD mouse model of spontaneous diabetes, for example, both CD4+ and CD8+ T cells are crucial for disease development. Wicker et al., Ann. Rev. Immunol. 12:179-200 (1995); Mora et al., J. Immuno/. 162:4576-88 (1996). Directly- and indirectly-primed CD4+ T cells help in the production of autoantibody and provide the signals required for induction of CD8+ CTLs, both of which are capable of injuring the cells expressing the autoantigen. Thus, the success of any immunosuppressive strategy directed against an autoimmune response depends on the effective inhibition of both major subsets of T cells.

Existing treatments for autoimmune diseases have had only limited success. For example, it is often possible to correct organ-specific autoimmune disease through metabolic control. Where function is lost and cannot be restored, mechanical substitutes or tissue grafts may be appropriate. While it may be possible to alleviate some of the symptoms using this approach, no effective long-term curative treatment exists for several of the most disabling autoimmune disorders, including multiple sclerosis and insulin-dependent diabetes mellitus (IDDM). While a number of compounds, including insulin, corticosteroids and modified beta interferon can ameliorate some of the symptoms of autoimmune diseases, they can have serious side effects and/or require long-term use. General immunosuppressive drug therapies, such as chronic treatment with cyclosporin A, FK506 and rapamycin have also been unable to provide a cure for these diseases, and their use is accompanied by a host of deleterious side effects.

More recently, immune modulatory strategies have been proposed based on the systemic administration of DNA vaccines encoding autoantigens, either alone or in combination with T-helper type 2 (Th2) cytokines such as IL-4 and IL-10. See, e.g., Ruiz et al., J. Immunol. 162:3336-3341 (1999); Garren et al., Immunity 15:15-22 (2001); U.S. Patent Publication No. US 2003/0148983 A1, the disclosures of which are expressly incorporated by reference herein. The preliminary data reported by these researchers suggested that DNA vaccines encoding the autoantigen alone could potentially anergize autoreactive T cells, while tolerizing vaccines in combination with IL-4 could help induce Th2 responses. Robinson et al., Nature Biotech. 21:1033-39 (2003). Data from another group of researchers suggested that the presence of IL-4 was critical for protection against disease development induced by the tolerizing vaccine. Bot et al., J. Immuno/. 167:2950-55 (2001). Thus, the success of this therapeutic strategy likely hinges on the co-administration of Th2-associated cytokines or vectors encoding the same along with the tolerizing vaccine to bias a pro-inflammatory T-helper type 1 (Th1) response to more protective Th2 response. Although this vaccine-based strategy has been somewhat effective in a prophylactic setting, it may prove much more difficult to treat an active autoimmune response already heavily biased towards an inflammatory Th1 response.

Accordingly, novel therapeutic compositions and protocols are sought that can inhibit the function of autoreactive T cells, including Th1-type T cells, in a highly specific fashion. It is an object of the present invention to inhibit and/or eliminate autoreactive CD4+ and CD8+ T lymphocytes to prevent the development of, as well as the progression of, autoimmune diseases.

SUMMARY OF THE RELEVANT LITERATURE

It is known that the activity of MHC class I-restricted T cells (e.g., CD8+ CTLs) can be suppressed when a CTL that has received a signal through its T cell receptor complex also receives a signal through the α3 domain of its class I MHC molecule. This so-called veto signal may be delivered by a CD8 molecule expressed by the stimulator or "veto" cell. Sambhara and Miller, Science 252:1424-1427 (1991). The resulting immune suppression is both antigen-specific and MHC-restricted, and results from the unidirectional recognition of the veto cell by the responding CTL, but not vice versa. Rammensee et al., Eur. J. Immunol. 12:930-934 (1982); Fink et al., J. Exp. Med. 157:141-154 (1983); Rammensee et al., J. Immuno/. 132:668-672 (1984). Veto activity has since been linked to the presence of the CD8 α-chains, such that the veto function is lost if expression of CD8 is deleted and established when the CD8 α-chain is expressed. Hambor et al, J. Immunol. 145:1646-1652 (1990); Hambor et al, Intern. Immuno/. 2:8856-8879 (1990); Kaplan et al., Proc. Natl. Acad. Sci. USA 86:8512-8515 (1989).

Numerous strategies have been proposed to exploit this antigen-specific suppressive pathway to eliminate unwanted cytotoxic T cell responses. One such strategy involves the use of polypeptide conjugates covalently linking CD8 or a functional domain thereof to secondary ligands that direct CD8's veto activity to specific target cells. See, *e.g.* U.S. Patent Nos. 5,242,687, 5,601,828 and 5,623,056. Alternatively, hybrid antibody molecules have been investigated having a monoclonal antibody binding site with specificity to MHC class I molecules linked to the extracellular domain of the CD8 α-chain. Qi et al., J. Exp. Med. 183:1973-1980 (1996). Such molecules, however, have several shortcomings and have yet to find actual clinical utility. Significantly, for treatment of autoimmune disease a hybrid antibody approach using both CD4 and CD8 hybrid antibodies was proposed based on the belief that the CD4 hybrid was needed to impact the responding CD4+ helper T-cell population. Staerz et el., Immunol Today 21 (4):172-6 (2000).

More recently, International PCT Publication No. WO 02/102852 describes the inhibition of CTL using soluble CD8 α-chain variants having amino acid modifications designed to increased affinity for MHC class I. Significantly, and consistent with the above prior art, it is taught therein that the proposed CD8α compositions are specific for class I MHC molecules and are therefore expected to inhibit only the response of CD8+ CTL. *Id.* at p. 27. It is further suggested that combinations with other immunosuppressive agents will be required in situations involving other elements of the cellular and humoral immune responses, *e.g.,* MHC class II-restricted T cells such as CD4+ T cells. *Id.* at p. 28.

SUMMARY OF THE INVENTION

The present invention is based on the surprising discovery that the targeted expression of CD8α in conjunction with induced autoantigen expression or presentation can effectively and specifically suppress an autoimmune response directed to the autoantigen. Thus, using the compositions and methods described herein one may selectively inhibit the full scope of a host immune response directed against an autoantigen without the need for chronic administration of general immunosuppressive agents, effectively resulting in specific immunological tolerance to the autoantigen. Using the compositions and methods described herein a specific autoreactive T cell population can be inhibited either as a prophylactic measure or for treatment of an ongoing autoimmune response.

A method for preventing the development of and for treating autoimmune diseases comprises contacting a target cell *ex vivo* or *in vivo* with an autoantigen and an expression vector encoding a CD8 polypeptide, preferably a human CD8 polypeptide, still more preferably the CD8 α-chain, wherein the combination of CD8 polypeptide expression and autoantigen presentation by a contacted target cell specifically inhibits an autoreactive immune response directed against the autoantigen. The present invention thus provides a therapeutic composition for inhibiting an autoreactive T cell response against a target cell comprising an expression vector encoding a CD8 polypeptide comprising all or a functional portion of a CD8 α-chain, wherein said CD8, α-chain includes a transmembrane domain for associating said CD8 α-chain on a surface of said target cell, and at least one epitope of an autoantigen associated with said autoreactive T cell response; wherein said expression of the CD8 polypeptide is separate from said expression of the autoantigen. The invention also provides an *ex vivo* method for conditioning a target cell to specifically inhibit an autoreactive immune response directed against an autoantigen expressed by the target cell comprising contacting the target cell *ex vivo* with a therapeutic composition according to any one of Claims 4 to 9.

Also provided is a polynucleotide comprising: a) a first nucleic acid sequence encoring a CD8 polypeptide comprising all or a functional portion of a CD8 α-chain, wherein said CD8 α-chain includes a transmembrane domain for associating said CD8 α-chain on a surface of said target cell; b) a second nucleic acid sequence encoding at least one epitope of an autoantigen associated with an autoreactive T cell response; and c) separate control sequences operably linked with said first and second nucleic acids for expression of said CD8 polypeptide and said autoantigen separately in the target cell.

The target cell may be, for example, a muscle cell, a hematopoietic cell, a stem cell or a cell subjected to or at risk of an autoimmune response. In a preferred embodiment, the target cell is an antigen-presenting cell, *e.g.,* a dendritic cell. When administered to or present in a host, both the contacted target cell as well as other host cells expressing the same autoantigen are protected and their survival prolonged through the elimination of both CD4+ and CD8+ T cells responsive to the autoantigen. Preferably, the subject compositions and methods are capable of inhibiting both the humoral and cellular immune responses to such autoantigens.

Methods for specifically inhibiting immune responses to an autoantigen may comprise contacting a target cell *ex vivo* or *in vivo* with an expression vector encoding a CD8 polypeptide, preferably a human CD8 polypeptide, still more preferably the human CD8 α-chain, and an autoantigen whereby the CD8 polypeptide and autoantigen are expressed by the target cell and whereby the autoimmune response directed against the autoantigen is specifically inhibited. In one embodiment of the invention, the CD8 polypeptide and autoantigen are encoded by the same expression vector. Alternatively, although not claimed herein, the CD8 polypeptide and autoantigen can be encoded by separate expression vectors. The autoantigen may be a full length protein or fragment thereof, or may comprise one or more functionally-relevant epitopes. In a further embodiment, the autoimmune response includes both a humoral component and a cellular component. In a preferred embodiment, the autoimmune response is effectively inhibited without the need for general immunosuppressive agents.

Methods for specifically inhibiting immune responses to an autoantigen may comprise contacting a target cell *ex vivo* or *in vivo* with an autoantigen protein or peptide and an expression vector encoding a CD8 polypeptide, preferably a human CD8 polypeptide, still more preferably the human CD8 α-chain, whereby the CD8 polypeptide is expressed by the target cell in conjunction with presentation of the autoantigen, and whereby the autoimmune response directed against the autoantigen is specifically inhibited. The autoantigen may be a full length protein or fragment thereof, or may comprise one or more functionally-relevant epitopes. Preferably, the target cell is a hematopoietic cell, particularly a lymphocyte or antigen presenting cell. Particularly preferably, the contacting is done *ex vivo*.

Compositions for inhibiting an autoimmune response against a target cell may comprise an expression vector encoding a CD8 polypeptide, preferably a human CD8 polypeptide, still more preferably the human CD8 α-chain and an autoantigen associated with the target cell. In one embodiment, the CD8 polypeptide and autoantigen are encoded by the same expression vector.

Methods for prolonging the survival of a cell expressing an autoantigen comprise contacting the cell with an expression vector encoding a CD8 polypeptide and an autoantigen, wherein the CD8 polypeptide and autoantigen are expressed by cells and whereby the survival time of the cell is extended.

Preferred CD8 polypeptides for use in the methods and compositions will generally comprise the CD8 α-chain, more preferably the extracellular domain of the CD8 α-chain, and still more preferably the Ig-like domain of the CD8 α-chain. In preferred embodiments of the invention, the CD8 polypeptides may comprise or consist essentially of the extracellular domain of the CD8 α-chain and a transmembrane domain, or more preferably the Ig-like domain of the CD8 α-chain and a transmembrane domain. In a particularly preferred embodiment, the transmembrane domain is the transmembrane domain of the CD8 α-chain. Given the nature of the subject expression methods, as well as the apparent inadequacies of the prior art soluble forms of CD8 α-chain described above, the presence of the CD8 α-chain transmembrane domain or a suitable alternative transmembrane region is deemed essential.

The CD8 α-chain may be co-expressed with an autoantigen or expression vectors comprising the CD8 polypeptide may be co-administered with an autoantigen protein or peptide. Preferred autoantigens are those associated with an autoimmune disease, *e.g.,* (pro)insulin (β-chain) and glutamic acid decarboxylase (GAD) in type I diabetes mellitus, myelin basic protein in multiple sclerosis, MMP-1 in rheumatoid arthritis, cholinergic receptor α-chain in myasthenia gravis, thyoglobulin in autoimmune thyroiditis, etc.

Preferred target cells of the compositions and methods described herein include, *e.g.,* muscle cells, cells of hematopoietic origin such as antigen-presenting cells (*e.g.,* dendritic cells), macrophages, granulocytes, erythrocytes, leukocytes, B lymphocytes, T lymphocytes, etc. and Langerhans cells of the skin, stem cells such as hematopoietic stem cells as well as tissue specific stem cells, *i.e.* dedicated stem cells for neurons, liver, etc.; and cells at risk of or subjected to an autoimmune attack such as pancreatic islet cells, glial cells of the brain, and the other cells and tissues described herein.

Suitable expression vectors contemplated for use in the subject methods and compositions include recombinant and non-recombinant vectors, and viral vectors (*e.g.,* adenoviral, retroviral, adeno-associated viral vectors and the like) as well as non-viral vectors (*e.g.,* bacterial plasmids, phages, liposomes and the like). In a preferred embodiment, modification of target cells may be achieved with a liposome-mediated nucleic acid transfer vehicle, or a viral-mediated nucleic acid transfer vehicle, or with naked DNA.

Target cells may be induced *ex vivo* to express a functional portion of an immunomodulatory molecule of the invention along with an autoantigen or biologically active fragment thereof and the induced cells may be infused into a patient. The autoantigen may be delivered to the target cell in the form of an expression vector or alternatively as a protein or polypeptide for processing via the Class I or Class II MHC pathway for presentation on the surface of the target cell.

While multiple embodiments are disclosed, still other embodiments of the present invention will become apparent to those skilled in the art from the following detailed description, which shows and describes illustrative embodiments of the invention. As will be realized, the invention is capable of modifications in various obvious aspects. Accordingly, the drawings and detailed description are to be regarded as illustrative in nature and not restrictive.

BRIEF DESCRIPTION OF THE DRAWINGS

FIGS. 1A-B depict the amino acid and nucleic acid sequences for the wild-type human CD8 $\alpha$-chain, including a demarcation of the different domains of the protein for human and mouse, respectively.

FIGS. 2A-B depict Balb/c spleen cells that were stimulated with C57BL/6 spleen cells. Cultures were supplemented with normal fibroblasts, medium or fibroblasts with CD8 of mouse (A) or human (B) origin. Cultures were harvested and tested for their lytic ability towards C57BL/6-derived target cells.

FIG. 3 depicts Balb/c (H-2d) mice that were injected with control fibroblasts (■ and A) or mCD8-transfected C57BL/6-(H-2$^b$) derived (O and •) fibroblasts. After two weeks animals were sacrificed, spleen cells were harvested, stimulated with C57BL/6 (H-2$^b$) (■ and O) or CBA/J (H-2$^k$) (• and A) spleen cells and tested for their lytic ability on EL4 (H-2b) (■ and O) or S.AKR (H-2$^k$) (• and A) target cells.

FIGS. 4A-B depict target cells (A) or CD8-expressing targets (■) that were tested for their susceptibility to lysis by alloreactive T cells (A) or by antigen-specific CTLs (B).

FIGS. 5A-B depict MLCs (Balb/c anti-C57B/6) that were set up in the presence of normal fibroblasts (•) and fibroblasts transduced with mAdCD8 (A, A) or HAdCD8 (B, ▲). No fibroblasts were added to control cultures (■). The lytic activity of these cultures towards an C57BL/6-derived target was determined at the end of the culture period.

FIG. 6 depicts immunization with an adenoviral veto transfer vector, mAdCD8. C57BL/6 mice were infected with the vectors indicated above. After 10 days, spleen cells were harvested and cultured in the presence of the Adbgal virus. The number of blast cells is given.

FIGS. 7A-B depict negative immunization with mAdCD8 (A) C57BL/6 mice were once immunized i.v. with Ad$\beta$gal or mAdCD8. (B) Animals treated as in (A) were re-immunized with Ad$\beta$gal after 5 days. Seven days after the last injection animals were sacrificed, and their spleen cells were cultured in the presence of Ad$\beta$gal. After 5 days of culture, cells were tested for their lytic ability of Ad$\beta$gal-infected syngeneic target cells.

FIG. 8 depicts 3x106 C7BI/6 spleen cells that were incubated with 1x106 (or no) stimulator cells, transduced as indicated. After 4 days the cultures were analyzed for presence CD4+ T lymphoblasts by immunofluorescence.

FIGS. 9A-D depict surface expression of mouse and human CD8 $\alpha$-chains after infection with the different virus constructs. A. Infected cells: MC57T Fibroblasts; Panel 1: Mock-Infection; Panel 2: Infection with hAdCDS. B. Infected cells: MC57T Fibroblasts; Panel 1: Mock Infection; Panel 2: Infection with mAdCD8. C. Infected cells: Balbc unselected bone marrow cells; Panel 1: Infection with lacZ Adenoviral Vector (AdLacZ); Panel 12: Infection with mAdCD8. D. Infected Cells: MC57T Fibroblasts; Panel 1: Mock-Infection; Panel 2: Infection with pAAV-mCD8; Panel 3: Infection with pAAV-hCD8.

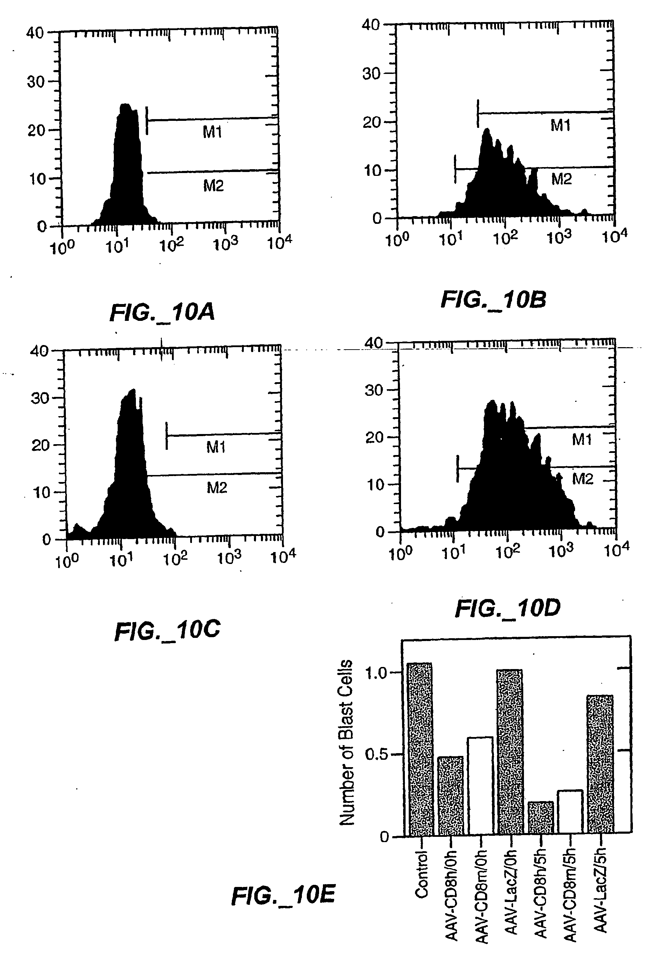

FIGS. 10A-E depict MLCs (Balb/c anti-C57BL/6) were set up in the presence of these fibroblasts that had been cultured for 0 or 5 hours after transduction before they were added to the MLCs. At the end of the cultures, the number of lymphoblasts was determined on a fluorescence activated cell analyzer.

FIG. 11 depicts *in vitro* inhibition with veto transfer vector. A Balb/c antiC57BL/6 mixed lymphocyte culture (MLC) was established in the absence or presence of uninfected or mAdCD8-infected MC57 fibroblasts (H-2b) (X). CTL responses were measured in EL4 (H-2b) target cells.

FIG. 12 depicts Balb/c mice that were immunized with AdLacZ (A) or mAdCD8 (■). Their spleen cells were cultured in the presence of AdLacZ and tested for specific lytic activity against AdLacZ-infected syngeneic P815 target cells.

FIGS. 13A-B depict (A) C57BL/6 animals that were immunized with AdLacZ (■) or mAdCD8 (▲). The lytic activity of their spleen cells towards syngeneic AdLacZ EL4 target cells was tested. (B) Such animals were re-immunized with AdLacZ prior to testing their lytic activity against AdLaz-infected EL4 targets.

FIG. 14 depicts the reduction in percentage of diabetic mice in mice injected with the pBudCE4.1 / CD8 $\alpha$-chain / Insulin 2 veto vector (■), comparing to the control group that was not treated (♦).

DETAILED DESCRIPTION

Effective and sustainable treatments for autoimmune diseases have been elusive. The success of the present invention stems from the surprising discovery that co-expression of an immunomodulatory molecule such as CD8, and particularly the CD8 $\alpha$-chain, and an autoantigen will effectively and specifically inhibit T cells responsive to the autoantigen, and thus both the auto reactive immune response can be inhibited.

In accordance therewith, and as defined herein the present invention provides polynucleotides, compositions and methods for inhibiting immune responses directed against a host cell expressing an autoantigen. Thus a conditioning step may comprise contacting host cells with an expression vector encoding for a CD8 polypeptide as described herein and at least one epitope of an autoantigen. An alternative conditioning method for modulating expression levels of CD8 in a target cell to effectively and specifically inhibit an immune response against the target cell may comprise e.g., providing transcriptional activators that result in increased CD8 expression. *See, e.g.,* Mortlock et al., Nuc. Acids. Res. 31 :152 (2003); Mizuguchi et al., Hum. Gene Ther. 14:1265-77 (2003). The specificity and selectivity of the immune inhibition achieved by the subject methods and compositions provides a significant improvement over conventional immunosuppressive strategies, which typically produce a generalized and non-specific inhibition of the immune system thereby leaving the host highly susceptible to adventitious infections and, in some cases, mutagenicity and cancer.

The methods described herein can be used alone or in combination with other methods, such as the administration of other active agents, *e.g.,* therapeutic or prophylactic agents and/or general immunosuppressive agents (*e.g.,* cyclosporin, FK506), T cell depletion antibodies *(e.g.,* OKT3, polyclonal anti-thymocyte preparations), etc. as are known in the art. Preferably, the use of such agents is unnecessary in view of the autoantigenspecific immunosuppression obtained using the subject compositions and methods.

By "inhibiting" is meant the direct or indirect, partial or complete, inhibition and/or reduction of an innate or acquired immune response, whether cellular (*e.g.,* leukocyte recruitment) or humoral, to autoantigens. Inhibiting can include preventing an immune response by signaling a change to the immune cell such that it no longer targets the autoantigen. Alternatively, inhibiting can include providing a signal resulting in anergy and/or death, *e.g.* apoptosis, of the immune cell.

By "immune response" is preferably meant an acquired immune response, such as a cellular or humoral immune response.

By "specific immune inhibition" or "antigen-specific immune inhibition" is meant the inhibition of immune responses directed against antigens such as autoantigens, as opposed to general immune inhibition that is not antigen-specific. Thus, by way of example, the absence of a cellular and/or humoral autoimmune response to an autoantigen that previously was recognized by the patient, combined with evidence of *in vivo* immune competence to other antigens, would demonstrate specific immune inhibition of autoantigen.

By "expression vector" is meant any vehicle for delivery of a nucleic acid to a target cell. Expression vectors can be generally divided into viral vectors and non-viral vectors. By viral vectors is meant, but not limited to adenoviral vectors, adeno-associated vectors, retroviral vectors, lentiviral vectors, and the like. By non-viral vectors is meant plasmid vectors, naked DNA, naked DNA coupled to different carriers, or DNA associated with liposomes or other lipid preparation. Generally, expression vectors are recombinant, although in some embodiments, for example when liposomes or cell ablation, *e.g.* biolistic techniques, are used, they are not. Preferred recombinant vectors for use herein are plasmid vectors as well as viral vectors selected from the group consisting of an adenoviral vector, an adeno-associated viral vector, a herpes viral vector, a retroviral vector and a lentiviral vector. In some embodiments utilizing recombinant viral vectors, and in particular adenoviral vectors, the immunogenicity of the capsid, e.g., the hexon protein of an adenoviral capsid, may be reduced in accordance with methods known in the art, although such modifications are no longer a necessity in view of the improvements detailed herein.

By "contacting" is meant administering the expression vector in such a manner and in such an amount as to effect physical contact between the vector and cell. If the vector is a recombinant viral particle, desirably, attachment to and infection of the cell by the viral vector is effected by such physical contact. If the viral vector is other than a recombinant viral particle, such as a nonencapsulated viral nucleic acid or other nucleic acid, desirably, entry into the cell by the nucleic acid is effected.

Such "contacting" can be done by any means known to those skilled in the art, and described herein, by which the apparent touching or mutual tangency of the vector with the target cell can be effected. Optionally, the vector, such as an adenoviral vector, can be further complexed with a bispecific or multispecific molecule (*e.g.,* an antibody or fragment thereof), in which case "contacting" involves the apparent touching or mutual tangency of the complex of the vector and the bispecific or multispecific molecule with the target cell. For example, the vector and the bispecific (multispecific) molecule can be covalently joined, *e.g.,* by chemical means known to those skilled in the art, or other means. Preferably, the vector and the bispecific (multispecific) molecule can be linked by means of noncovalent interactions (*e.g.,* ionic bonds, hydrogen bonds, Van der Waals forces, and/or nonpolar interactions). Although the vector and the bispecific (multispecific) molecule can be brought into contact by mixing in a small volume of the same solution, the target cell and the complex need not necessarily be brought into contact in a small volume, as, for instance, in cases where the complex is administered to a host (*e.g.,* a human), and the complex travels by the bloodstream to the target cell to which it binds selectively and into which it enters. The contacting of the vector with a bispecific (multispecific) molecule preferably is done before the target cell is contacted with the complex of the vector and the bispecific (multispecific) molecule.

### AUTOANTIGENS

By "autoantigen" is meant a "self" antigen that is the target of one's own immune system. Autoantigens include any self antigen that the host or patient immune system recognizes and responds against as foreign including, *e.g.,* self antigens associated with an autoimmune disorder including, *e.g.,* myelin basic protein (MBP), proteolipid protein PLP-1, myelin oligodendrocyte glycoprotein, pro-insulin/insulin, glutamic acid decarboxylase (GAD), matrix metalloproteinase (MMP-1), type II collagen, thyroglobulin, and the like. In addition, autoantigens may be present in the host at levels different (e.g. elevated or reduced) from 11

levels present in well or non-afflicted patients. These include Alzheimer's disease, Parkihson's disease, Huntington's disease and Prion disease. Several other diseases associated with autoantigens present in a host at levels different from levels present in well or non-afflicted patients include obesity, osteoarthritis, spinal cord injury, hypertension, peptic ulcer disease, aging depression, gout, migraine headaches, hyperlipidemia and coronary artery disease.

The expression vector may comprise one or more transgenes encoding at least one epitope of an autoantigen along with at least one transgene encoding for an immunomodulatory molecule as described herein. Alternatively, autoantigen proteins or peptides may be employed in conjunction with DNA-based expression of a CD8 polypeptide. The autoantigen may be a full length protein or fragment thereof, or may comprise one or more functionally-relevant epitopes. Diseases that may be treated by the present invention include, but are not limited to, multiple sclerosis, type I diabetes mellitus, myasthenia gravis, autoimmune thyroiditis and rheumatoid arthritis, and the like. Additional autoantigens are described in Nature Medicine 7:8 (Aug.2001) 899-905. Autoantigens associated with such diseases are set forth in Table 1 below.

### TABLE 1

| Autoimmune Disease | Self-Antigen | Number mRNA | Reference |
|---|---|---|---|
| Multiple Sclerosis | proteolipid protein PLP-1 | NM_000533 | J. Neurosci Res. 2001 Feb 1;63(3):290-302 |
| | myelin basic protein | NM_002385 | J Neurosci Res. 2001 Feb 63(3):290-302 |
| | Myelin associated oligodendrocytic basic protein | NM_006501 | J. Neurochem. 88 (5), 1211-1219 (2004); J. Biol. Chem. 269 (50), 31725-31730(1994) |
| | Myelin-associated glycoprotein | NM_002361; NM_080600 | J. Neuropathol. Exp. Neurol. 62 (1), 25-33 (2003); J. Neurosci. 24 (2), 137-142 (1989) |
| | Alpha-B-crystallin | NM_001885 | J. Neurosci. Res. 75 (4), 516-523 (2004) |
| | Cyclic nucleotide phosphodiesterase | NM_033133 | |
| | myelin oligodendrocyte glycoprotein | NM_002433 | J Neurosci Res. 2001 Feb 63(3):290-302 |

(continued)

| Autoimmune Disease | Self-Antigen | Number mRNA | Reference |
|---|---|---|---|
| Type 1 Diabetes Mellitus | Pre-proinsulin/pro-insulin / insulin | NM_000207 | Proc Natl Acad Sci USA 2003 Sep 2;100(18): 10376-81. Epub 2003 Aug 18 |
| | glutamic acid decarboxylase 2 (65 kDa and 67 kDa forms) | NM_000818 | J Autoimmun. 2003 May; 203):203-6 |
| | Tyrosine phosphatase IA2, IA-2$\beta$ | NM_002846 | |
| | Carboxypeptidase H | | |
| | Heat shock proteins | | |
| | Glima 38 | | J. Clin. Invest. 1996 Jun; 15:97(12); 2772-83 |
| | Islet cell antigen 69 kDA | | J. Biol. Chem; 2003 Jul; 11: 278(28): 26166-73 |
| | P52 | | J. Autoimmun. 1997 Aug; 1-(4): 387-94 |
| | Islet cell glucose transporter GLUT-2 | J03810 | |
| Guillian Barrre Syndrome | Peripheral myelin protein I (and others) | | |
| Myasthenia Gravis | cholinergic receptor $\alpha$-chain | NM_005199 | Ann NY Acad Sci. 2003 Sep;998:284-307 |
| Autoimmune Thyroiditis | Thyoglobulin | NM_003235 | Int Rev Immunol. 2000;19 (6):501-33 |
| | Thyroid peroxidase | AF439430 | |
| Rheumatoid Arthritis | Type I, II, III, IV, V, IX and XI collagens | BM_001844 | J Rheumatol. 2000 Mar;27 (3):589-93 |
| | Immunoglobulin | | |
| | fibrin | | |
| | filaggrin | XM_048104 | |
| | GP-39 f | NM_001276 | Genomics 43 (2), 221-225 (1997) |
| | hnRNPs | | |
| | matrix metalloproteinase MMP-1 | NM_002412 | J Rheumatol. 2003 Jun;30 (6):1147-56 |
| Autoimmune Uveitis | S-antigen | | |
| | Interphotoreceptor retinoid binding protein (IRBP) | M22453 | |
| | rhodopsin | | |
| | recoverin | | |
| Primary Biliary Cirrhosis | Pyruvte dehydrogenase complexes (2-oxoacid dehydrogenase) | | |

(continued)

| Autoimmune Disease | Self-Antigen | Number mRNA | Reference |
|---|---|---|---|
| Autoimmune hepatitis | Liver hepatocyte antigens | | |
| | Cytochrome p450 | | |
| Pemphigus vulgaris | Desmoglein-1, -3 (and others) | NM_001942 NM_001944 | |
| Autoimmune gastritis | H+/K+ ATPASE | | Eksp Klin Gastroenterol. 2003;(3):5-6, 116. |
| | Intrinsic factor | | |
| Pernicious Anemia | Intrinsic factor | | |
| Polymyositis | Histidyl tRNA synthetase, | NM_002109 | |
| | Other synthetases | | |
| | Other nuclear antigens | | |
| Grave's disease | Thyroid-stimulating hormone receptor | NM_000369 | |
| Psoriasis | | | |
| Vitiligo | Tyrosinase | NM_000372 | |
| | Tyrosinase-related protein-2 | NM_000550 | |
| Systemic Lupus Eryth. | Systemic nuclear antigens | | |
| | DNA | | |
| | histones | | |
| | ribonucleoproteins | | |
| Celiac disease | Transglutaminase | NM_198951 | J. Biol. Chem. 277 (37), 34109-34116 (2002) |

Several examples of autoimmune diseases associated with autoantigens and noting target tissues are set forth below in Table 2.

### TABLE 2

| Autoimmune Disease | Tissue Targeted |
|---|---|
| Multiple Sclerosis | Central nervous system |
| Guillian Barre Syndrome | Peripheral nervous system |
| Insulin Dependent Diabetes Mellitus | β cells in islets of pancreas |
| Rheumatoid Arthritis | Synovial joints |
| Autoimmune Uveitis | Eye, uvea |
| Primary Biliary Cirrhosis | Bilary tree of liver |
| Autoimmune Hepatitis | Bilary tree of liver |
| Pemphigus Vulgaris | Skin nerve-muscle junct. |
| Autoimmune Gastritis | Stomach/parietal cells |
| Pernicious Anemia Polymyositis | Stomach muscle |
| Autoimmune Thyroiditis | Thyroid |
| Grave's Disease | Thyroid |

(continued)

| Autoimmune Disease | Tissue Targeted |
|---|---|
| Psoriasis | Skin |
| Vitiligo | Skin |
| Systemic Lupus Eryth. | |
| Celiac Disease | Small bowel |

*Multiple Sclerosis*

Multiple sclerosis (MS) is the most common demyelinating disorder of the CNS and affects 350,000 Americans and one million people worldwide. Onset of symptoms typically occurs between 20 and 40 years of age and manifests as an acute or sub-acute attack of unilateral visual impairment, muscle weakness, paresthesias, ataxia, vertigo, urinary incontinence, dysarthria, or mental disturbance (in order of decreasing frequency). Such symptoms result from focal lesions of demyelination which cause both negative conduction abnormalities due to slowed axonal conduction, and positive conduction abnormalities due to ectopic impulse generation (e.g. Lhermitte's symptom). Diagnosis of MS is based upon a history including at least two distinct attacks of neurologic dysfunction that are separated in time, produce objective clinical evidence of neurologic dysfunction, and involve separate areas of the CNS white matter. Laboratory studies providing additional objective evidence supporting the diagnosis of MS include magnetic resonance imaging (MRI) of CNS white matter lesions, cerebral spinal fluid (CSF) oligoclonal banding of IgG, and abnormal evoked responses. Although most patients experience a gradually progressive relapsing remitting disease course, the clinical course of MS varies greatly between individuals and can range from being limited to several mild attacks over a lifetime to fulminant chronic progressive disease. A quantitative increase in myelin-autoreactive T cells with the capacity to secrete IFN-gamma is associated with the pathogenesis of MS and EAE.

Autoantigens associated with the autoimmune response in autoimmune demyelinating diseases, such as multiple sclerosis and experimental autoimmune encephalomyelitis (EAE), may comprise epitopes from proteolipid protein (PLP); myelin basic protein (MBP); myelin oligodendrocyte glycoprotein (MOG); cyclic nucleotide phosphodiesterase (CNPase); myelin-associated glycoprotein (MAG), and myelin-associated oligodendrocytic basic protein (MBOP); alpha-B-crystalin (a heat shock protein); viral and bacterial mimicry peptides, e.g. influenza, herpes viruses, hepatitis B virus, etc.; OSP (oligodendrocyte specific-protein); citrulline-modified MBP (the C8 isoform of MBP in which 6 arginines have been de-imminated to citrulline); etc. The integral membrane protein PLP is a dominant autoantigen of myelin. Determinants of PLP antigenicity have been identified in several mouse strains, and include residues 139-151, 103-116, 215-232, 43-64 and 178-191. At least 26 MBP epitopes have been reported (Meinl et al., J Clin Invest 92, 2633-43, 1993). Notable are residues 1-11, 59-76 and 87-99. Immunodominant MOG epitopes that have been identified in several mouse strains include residues 1-22, 35-55, 64-96. As used herein the term "epitope" is understood to mean a portion of an autoantigen having a particular shape or structure that is recognized by either B-cells or T-cells of the animal's immune system.

In human MS patients the following myelin proteins and epitopes were identified as targets of the autoimmune T and B cell response. Antibody eluted from MS brain plaques recognized myelin basic protein (MBP) peptide 83-97 (Wucherpfennig et al., J Clin Invest 100:1114-1122, 1997). Another study found approximately 50% of MS patients having peripheral blood lymphocyte (PBL) T cell reactivity against myelin oligodendrocyte glycoprotein (MOG) (6-10% control), 20% reactive against MBP (8-12% control), 8% reactive against PLP (0% control), 0% reactive MAG (0% control). In this study 7 of 10 MOG reactive patients had T cell proliferative responses focused on one of 3 peptide epitopes, including MOG 1-22, MOG 34-56, MOG 64-96 (Kerlero de Rosbo et al., Eur J Immunol 27, 3059-69, 1997). T and B cell (brain lesion-eluted Ab) response focused on MBP 87-99 (Oksenberg et al., Nature 362, 68-70, 1993). In MBP 87-99, the amino acid motif HFFK is a dominant target of both the T and B cell response (Wucherpfennig et al., J Clin Invest 100, 1114-22, 1997). Another study observed lymphocyte reactivity against myelin-associated oligodendrocytic basic protein (MOBP), including residues MOBP 21-39 and MOBP 37-60 (Holz et al., J Immunol 164, 1103-9, 2000). Using immunogold conjugates of MOG and MBP peptides to stain MS and control brains both MBP and MOG peptides were recognized by MS plaque-bound Abs (Genain and Hauser, Methods 10, 420-34, 1996).

*Rheumatoid Arthritis*

Rheumatoid arthritis (RA) is a chronic autoimmune inflammatory synovitis affecting 0.8% of the world population. It is characterized by chronic inflammatory synovitis that causes erosive joint destruction. RA is mediated by T cells, B cells and macrophages.

Evidence that T cells play a critical role in RA includes the (1) predominance of CD4[+] T cells infiltrating the synovium, (2) clinical improvement associated with suppression of T cell function with drugs such as cyclosporine, and (3) the association of RA with certain HLA-DR alleles. The HLA-DR alleles associated with RA contain a similar sequence of amino acids at positions 67-74 in the third hypervariable region of the α-chain that are involved in peptide binding and presentation to T cells. RA is mediated by autoreactive T cells that recognize an autoantigen present in synovial joints. Autoantigens associated with RA include epitopes from type II collagen; hnRNP; A2/RA33; Sa; filaggrin; keratin; citrulline; cartilage proteins including gp39; collagens type I, III, IV, V, IX, XI; HSP-65/60; IgM (rheumatoid factor); RNA polymerase; hnRNP-B1; hnRNP-D; cardiolipin; aldolase A; citrulline-modified filaggrin and fibrin. Autoantibodies that recognize filaggrin peptides containing a modified arginine residue (de-iminated to form citrulline) have been identified in the serum of a high proportion of RA patients. Autoreactive T and B cell responses are both directed against the same immunodominant type II collagen (CII) peptide 257-270 in some patients.

*Insulin Dependent Diabetes Mellitus*

Insulin Dependent Diabetes Mellitus Human type I or insulin-dependent diabetes mellitus (IDDM) is characterized by autoimmune destruction of beta cells in the pancreatic islets of Langerhans. The depletion of beta cells results in an inability to regulate levels of glucose in the blood. Overt diabetes occurs when the level of glucose in the blood rises above a specific level, usually about 250 mg/dl. In humans a long presymptomatic period precedes the onset of diabetes. During this period there is a gradual loss of pancreatic beta cell function. The development of disease is implicated by the presence of autoantibodies against insulin, glutamic acid decarboxylase, and the tyrosine phosphatase IA2 (IA2), each an example of an autoantigen associated with IDDM and employed in the subject invention.

Markers that may be evaluated during the presymptomatic stage are the presence of insulitis in the pancreas, the level and frequency of islet cell antibodies, islet cell surface antibodies, aberrant expression of Class II MHC molecules on pancreatic beta cells, glucose concentration in the blood, and the plasma concentration of insulin. An increase in the number of T lymphocytes in the pancreas, islet cell antibodies and blood glucose is indicative of the disease, as is a decrease in insulin concentration.

The Non-Obese Diabetic (NOD) mouse is an animal model with many clinical, immunological, and histopathological features in common with human IDDM. NOD mice spontaneously develop inflammation of the islets and destruction of the beta cells; which leads to hyperglycemia and overt diabetes. Both CD4[+] and CD8[+] T cells are required for diabetes to develop, although the roles of each remain unclear. As noted above, it has been shown that administration of insulin or GAD, as proteins or in the form of DNA vaccines, under tolerizing conditions to NOD mice may prevent disease and can down regulate responses to the other autoantigens.

The presence of combinations of autoantibodies with various specificities in serum are highly sensitive and specific for human type I diabetes mellitus. For example, the presence of autoantibodies against GAD and/or IA-2 is approximately 98% sensitive and 99% specific for identifying type I diabetes mellitus from control serum. In non-diabetic first degree relatives of type I diabetes patients, the presence of autoantibodies specific for two of the three autoantigens including GAD, insulin and IA-2 conveys a positive predictive value of >90% for development of type I DM within 5 years.

Autoantigens associated with human insulin dependent diabetes mellitus may include the tyrosine phosphatase IA-2; IA-2.ß.; glutamic acid decarboxylase (GAD) both the 65 kDa and 67 kDa forms; carboxypeptidase H; insulin; proinsulin; heat shock proteins (HSP); glima 38; islet cell antigen 69 KDa (ICA69); p52; two ganglioside antigens (GT3 and GM2-1); and an islet cell glucose transporter (GLUT 2).

Human IDDM is currently treated by monitoring blood glucose levels to guide injection, or pump-based delivery, of recombinant insulin. Diet and exercise regimens contribute to achieving adequate blood glucose control.

*Autoimmune Uveitis*

Autoimmune uveitis is an autoimmune disease of the eye that is estimated to affect 400,000 people, with an incidence of 43,000 new cases per year in the U.S. Autoimmune uveitis is currently treated with steroids, immunosuppressive agents such as methotrexate and cyclosporin, intravenous immunoglobulin, and TNFα-antagonists.

Experimental autoimmune uveitis (EAU) is a T cell-mediated autoimmune disease that targets neural retina, uvea, and related tissues in the eye. EAU shares many clinical and immunological features with human autoimmune uveitis, and is induced by peripheral administration of uveitogenic peptide emulsified in Complete Freund's Adjuvant (CFA).

Autoantigens associated with the autoimmune response in human autoimmune uveitis may include S-antigen, interphotoreceptor retinoid binding protein (IRBP), rhodopsin, and recoverin.

*Primary Billiary Cirrhosis*

Primary Biliary Cirrhosis (PBC) is an organ-specific autoimmune disease that predominantly affects women between 40-60 years of age. The prevalence reported among this group approaches 1 per 1,000. PBC is characterized by progressive destruction of intrahepatic biliary epithelial cells (IBEC) lining the small intrahepatic bile ducts. This leads to obstruction and interference with bile secretion, causing eventual cirrhosis. Association with other autoimmune diseases characterized by epithelium lining/secretory system damage has been reported, including Sjogren's Syndrome, CREST Syndrome, autoimmune thyroid disease and rheumatoid arthritis. Attention regarding the driving antigen(s) has focused on the mitochondria for over 50 years, leading to the discovery of the antimitochondrial antibody (AMA) (Gershwin et al., Immunol Rev 174:210-225, 2000); (Mackay et al., Immunol Rev 174:226-237, 2000). AMA soon became a cornerstone for laboratory diagnosis of PBC, present in serum of 90-95% patients long before clinical symptoms appear. Autoantigenic reactivities in the mitochondria were designated as M1 and M2. M2 reactivity is directed against a family of components of 48-74 kDa. M2 represents multiple autoantigenic subunits of enzymes of the 2-oxoacid dehydrogenase complex (2-OADC) and is another exemplary autoantigen of the instant invention. Studies identifying the role of pyruvate dehydrogenase complex (PDC) antigens in the etiopathogenesis of PBC support the concept that PDC plays a central role in the induction of the disease (Gershwin et al., Immunol Rev 174:210-225, 2000); (Mackay et al., Immunol Rev 174:226-237, 2000). The most frequent reactivity in 95% of cases of PBC is the E2 74 kDa subunit, belonging to the PDC-E2. There exist related but distinct complexes including: 2-oxoglutarate dehydrogenase complex (OGDC) and branched-chain (BC) 2-OADC. Three constituent enzymes (E1,2,3) contribute to the catalytic function which is to transform the 2-oxoacid substrate to acyl co-enzyme A CoA), with reduction of NAD$^+$ to NADH. Mammalian PDC contains an additional component, termed protein X or E-3 Binding protein (E3BP). In PBC patients; the major antigenic response is directed against PDC-E2 and E3BP. The E2 polypeptide contains two tandemly repeated lipoyl domains, while E3BP has a single lipoyl domain. The lipoyl domain is found in a number of autoantigen targets of PBC and is referred to herein as the "PBC lipoyl domain." PBC is currently treated with glucocorticoids and immunosuppressive agents including methotrexate and cyclosporin A.

A murine model of experimental autoimmune cholangitis (EAC) uses intraperitoneal (i.p.) sensitization with mammalian PDC in female SJUJ mice, inducing non-suppurative destructive cholangitis (NSDC) and production of AMA (Jones, J Clin Pathol 53:813-21, 2000).

*Other Autoimmune Diseases And Associated Autoantigens*

Autoantigens associated with myasthenia gravis may include epitopes within the acetylcholine receptor. Autoantigens associated with pemphigus vulgaris may include desmoglein-3. Sjogren's syndrome antigens may include SSA (Ro); SSB (La); and fodrin. The dominant autoantigen for pemphigus vulgaris may include desmoglein-3. Autoantigens associated with myositis may include tRNA synthetases (e.g., threonyl, histidyl, alanyl, isoleucyl, and glycyl); Ku; Scl; SSA; U1 Sn ribonuclear protein; Mi-1; Mi-1; Jo-1; Ku; and SRP. Autoantigens associated with scieroderma may include Scl-70; centromere; U1 ribonuclear proteins; and fibrillarin. Autoantigens associated with pernicious anemia may include intrinsic factor; and glycoprotein beta subunit of gastric H/K ATPase. Autoantigens associated with systemic lupus erythematosus (SLE) may include DNA; phospholipids; nuclear antigens; Ro; La; U1 ribonucleoprotein; Ro60(SS-A); Ro52 (SS-A); La (SS-B); calreticulin; Grp78; Scl-70; histone; Sm protein; and chromatin, etc. For Grave's disease autoantigens may include the Na+/I- symporter; thyrotropin receptor; Tg; and TPO.

*Neurodegenerative Diseases*

In addition, several neurodegenerative diseases are associated with autoantigens present in the host at levels different from levels present in well or non-afflicted patients. Examples of these are set forth in Table 3.

**TABLE 3**

| Neurodegenerative Disease | Pathologic Deformity | Autoantigen Present Non-Physiologically |
|---|---|---|
| Alzheimer's disease | senile plaques | amyloid β protein |
| Parkinson's disease | Lewy bodies | α-synuclein |
| Huntington's disease | intranuclear inclusions | Huntingtin protein |
| Prion disease | Prion protein inclusions | Prion protein |

*Alzheimer's Disease*

Alzheimer's disease (AD) is the most common neurodegenerative disease in the population (Cummings et al., Neurology 51, S2-17; discussion S65-7, 1998). AD affects approximately 10% of people over age 65 and almost 50% of people over age 85. It is estimated that by the year 2025, about 22 million individuals will be afflicted with AD. AD is characterized by a slowly progressive dementia. The definitive diagnosis of AD is made if the triad of dementia, neurofibrillary tangles, and senile plaques are found post-mortem. Senile plaques are invariably found in the brains of patients with Alzheimer disease. The principal constituent of senile plaques is amyloid beta protein (Aβ) (Iwatsubo et al., Neuron 13:45-53, 1994) (Lippa et al., Lancet 352:1117-1118, 1998) another example of a auto-antigen of this invention. Aβ is a 42 amino acid peptide that is derived from the amyloid precursor protein (APP), which is a transmembrane glycoprotein with a variety of physiologic roles, including cell proliferation, adhesion, cell signaling, and neurite outgrowth (Sinha et al., Ann N Y Acad Sci 920:206-8, 2000). APP is normally cleaved within the Aβ domain to generate a secreted fragment. However, alternative processing leads to the cleavage of APP to generate soluble Aβ that can accumulate within senile plaques.

The current therapies for AD are limited in efficacy and are not targeted to the Aβ accumulation. The available drugs are central cholinesterase inhibitors aimed at increasing the concentration of postsynaptic acetylcholine in the brain (Farlow and Evans, Neurology 51, S36-44; discussion S65-7, 1998); (Hake, Cleve Clin J Med 68, 608-9:613-4, 616, 2001). These drugs provide minimal clinical benefit in only a few cognitive parameters. A mouse transgenic for human Aβ has been shown to have many features in common with human AD (Games et al., Nature 373:523-527, 1995); (Hsiao et al., Science 274:99-102, 1996). In these transgenic mice, immunization with the Aβ peptide has demonstrated efficacy in terms of cognitive improvement and reduced histopathology (Morgan et al., Nature 408:982-985, 2000); (Schenk et al., Nature 400:173-177, 1999). Studies have also shown that creating an antibody response against Aβ with a peptide vaccine in animal models of Alzheimer disease can reverse the abnormal histopathology as well as the behavioral changes observed in these models (Bard et al., Nat Med 6:916-19, 2000); (DeMattos et al., Proc Natl Acad Sci U S A 98:8850-8855, 2001).

*Parkinson's Disease*

Parkinson's disease is a neurodegenerative disease of the extrapyramidal motor system that has a very high prevalence of 128-168 per 100,000 (Schrag et al., Bmj 321:21-22, 2000). The cardinal clinical features are resting tremor, bradykinesia, rigidity, and postural instability. Dementia also occurs in the majority of cases in its late stages. The pathophysiologic hallmark is the loss of neurons within the extrapyramidal system of the brain and especially within the substantia nigra. Many neurons within the brains of patients with Parkinson's disease have an intracellular inclusion known as a Lewy body (Fomo and Norville, Acta Neuropathol (Berl) 34:183-197, 1976). It has been found that the major constituent of Lewy bodies is a protein known as α-synuclein, another example of an autoantigen of this invention (Dickson, Curr Opin Neurol 14:423-432, 2001). The accumulation of Lewy bodies containing α-synuclein has been correlated with the disease phenotype.

Current therapies for Parkinson's disease are directed at managing the resultant symptoms of the disease but not the underlying cause (Jankovic, Neurology 55:S2-6, 2000). The available drugs for Parkinson's disease are classified as dopaminergic agents (e.g., carbidopa/levodopa and selegiline), dopamine agonists (e.g, pergolide and ropinirole), and catechol-o-methyl-transferase or COMT inhibitors (e.g., entacapone and tolcapone). All of these therapies are directed at increasing the amount of dopamine available in the affected neurons. As a whole, these drugs are initially effective in most patients at reducing some of the motor symptoms such as tremor and rigiditiy, but are not effective in attenuating the progression of the neurodegenerative process that leads to destruction of the neurons of the substantia nigra.

*Huntington's Disease*

Huntington's disease is a genetic disorder inherited in an autosomal dominant fashion and linked to an abnormal expansion in the length of a CAG trinucleotide repeat contained within a gene called huntingtin (Cell 72, 971-983 1993). The predominant clinical features consist of an abnormal uncontrollable movement called chorea and a progressive dementia. Pathophysiologically there is selective neuronal death and degeneration within the corpus striatum and cerebral cortex. The neurons within these regions have been shown to accumulate intracellular aggregates of mutant protein, huntingtin, another autoantigen of this invention and this accumulation is correlated with disease phenotype (DiFiglia et al., Science 277: 1990-1993, 1997); (Scherzinger et al., Cell 90:549-558, 1997); (Davies et al., Cell 90:537-548, 1997).

There are currently no available treatments for either the symptoms of or the etiologic cause of Huntington's disease. As a result, these patients slowly progress to inevitable death on average 17 years after the first onset of symptoms.

*Prion Disease*

Prion disease, also known as transmissible spongiform encephalopathy, is a potentially infectious disease which affects animals and humans and is characterized by a sponge-like degeneration of the brain (Prusiner, Proc Natl Acad Sci U S A 95, 13363-83, 1998). The most common form of this disorder is also termed Creutzfeldt-Jakob disease. Another form of the disease called new-variant Creutzfeldt-Jakob disease has major public health implications because it is felt to occur by cross-species transmission, for example from cattle to man. The clinical features of this group of disorders includes a rapidly progressive dementia, myoclonus, weakness, and ataxia. Pathophysiologically, it has been reported in the literature that a conformational change in the normal prion protein, another autoantigen of this invention, causes the accumulation of the prion protein into a beta sheet type structure, leading to the degeneration seen within the central nervous system. Presently there are no treatments available for prion disease. The clinical course is rapid with inevitable death usually within two years of diagnosis and no intervention has been able to alter this course.

In addition, several other diseases are associated with autoantigen present in the host at levels different from levels present in well or non-afflicted patients. Examples of these are set forth in Table 4.

**TABLE 4**

| Disease | Abnormality | Autoantigens Associated with Disease and Present Non-Physiologically |
|---|---|---|
| Obesity | weight gain due to energy intake exceeds expenditure | syndecan-3, perilipin, Orexin, Galanin, glucogon-like peptide receptor |
| Osteoarthritis | cartilage degeneration | cathepsins, plasmin, collagenases, metalloproteinases |
| Spinal cord injury | inhibition of regeneration | Nogo-1 |
| Hypertension | persistent high blood pressure | angiotensin-converting enzyme |
| Peptic ulcer disease | excess stomach acid | $H^+/K^+$ ATPase, gastrin |
| Aging | | superoxide dismutase |
| Depression | excessive serotonin | serotonin 5HT2 receptor, $\alpha_1$-adrenergic receptor |
| Gout | Excess uric acid | Xanthine oxidase |
| Migraine headaches | vasospasm | serotonin $5HT_{1B}$ and $5HT_{1D}$ receptors |
| Hyperlipidemia | elevated lipids | HMG CoA-reductase, apolipoproteins A, B-100 |
| Coronary artery disease | obstruction of coronary arteries restricting blood flow | Angiotensin-converting enzyme, apolipoproteins A, B-100 |

*Osteoarthritis and Degenerative Joint Diseases*

Osteoarthritis (OA) affects 30% of people over 60 years of age, and is the most common joint disease of humans. Osteoarthritis represents the degeneration and failure of synovial joints, and involves breakdown of the articular cartilage.

Cartilage is composed primarily of proteoglycans, which provide stiffness and ability to withstand load, and collagens that provide tensile and resistance to sheer strength. Chondrocytes turnover and remodel normal cartilage by producing and secreting latent collagenases, latent stromelysin, latent gelatinase, tissue plasminogen activator and other associated enzymes, each of which are autoantigens for use in this invention. Several inhibitors, including tissue inhibitor of metalloproteinase (TIMP) and plasminogen activator inhibitor (PAI-1), are also produced by chondrocytes and limit the degradative activity of neutral metalloproteinases, tissue plasminogen activator, and other enzymes. These degradative enzymes and inhibitors, are also autoantigens for use in this invention. These degradative enzymes and inhibitors coordinate remodeling and maintenance of normal cartilage. In OA, dysregulation of this process results in the deterioration and degradation of cartilage.

In early OA there are abnormal alterations in the arrangement and size of collagen fibers. Metalloproteinases, cathepsins, and plasmin, alone or in combination are auto-antigen(s) of this invention, cause significant cartilage matrix loss. Initially increased chondrocyte production of proteoglycans and cartilage results in the articular cartilage being thicker than normal. The articular cartilage then thins and softens as a result of the action of degradative enzymes including collagenases, stromelysin, gelatinase, tissue plasminogen activator and other related enzymes, alone or in combination are autoantigen(s) of this invention. IL-1, cathepsins, and plasmin may promote the degeneration and breakdown of cartilage alone or in combination and are auto-antigen(s) of this invention. The softer and thinner cartilage is much more susceptible to damage by mechanical stress. These factors lead to the breakdown of the cartilage surface and the formation of vertical clefts (fibrillation). Erosions in the cartilage surface form, and extend to bone in end-stage disease. Chondrocytes initially replicate and form clusters, and at end stage the cartilage is hypocelluar. Remodeling and hypertrophy of bone are significant features of OA.

Current therapies for OA include rest, physical therapy to strengthen muscles supporting the joint, braces and other supportive devices to stabilize the joint, non-steroidal anti-inflammatory agents and other analgesics. In end-stage bone-on-bone OA of joints critical for activities of daily living, such as the knees or hips, surgical joint replacement is often performed.

*Obesity*

Obesity is a major health problem facing the United States and other industrialized countries. It is estimated that obesity affects 20% of the U.S. population. Obesity is the excess of adipose tissue. When prolonged energy intake exceeds expenditure for prolonged periods, excess calories are stored as adipose tissue resulting in obesity. Obesity can thus result from increased intake and/or decreased expenditure. Intake is dependent on eating behavior, which is a complex process controlled by the cerebral cortex. Discrete regions of the hypothalamus, including the feeding center and the satiety center send signals to the cerebral cortex to facilitate the regulation of feeding. Blood glucose, insulin, glycerol and other levels may be detected by the feeding and satiety centers in the hypothalamus to help regulate feeding behavior.

Humans can partially adapt to excessive intake of calories by several mechanisms. Excess intake of carbohydrate and protein can be, in part, compensated for by increasing the resting metabolic rate through mechanisms that increase plasma levels of triiodothyronine (T3) and decrease levels of reverse T3 (rT3). Increased central or peripheral sympathetic outflow also increase catecholamine-induced caloric usage and heat production. Dietary thermogenesis, or the body's thermal response to food involves increased heat and metabolic expenditure above the resting metabolic rate for several hours following ingestion of a meal and is greater for protein, than for carbohydrate or fat based meals.

Feeding behavior and adipogenesis are controlled by complex mechanisms. Molecules including syndecan-3 regulates feeding and increases feeding behavior in the hypothalamus (Reizes et al, Cell 106:105-116, 2001). Other molecules and receptors that impact food intake and metabolism include Orexin, Galanin, corticotrophin-releasing factor, melanin-concentrating hormone, leptin, cholecystokinin, somatostatin, enterostating, glucagons-like peptides 1 and 2, and bombesin, all of which either alone or in combination are autoantigen(s) for use in this invention. (Chiesi et al, Trends Pharmacological Sciences, 22:247-54, 2001). In animal models of obesity, antagonists or agonists of several of these molecules have demonstrated efficacy in weight reduction (Chiesi et al, Trends Pharmacological Sciences, 22:247-54, 2001). Perilipin coats lipid droplets of adipocytes and regulates triacylglycerol hydrolysis, and interference with perilipin resulted in mice resistant to diet-induced obesity but with normal glucose tolerance (Tansey et-al, Proc. Natl. Acad. Sci. USA, 98:6494-99).

When obesity is secondary to a secondary metabolic or other disease state, that secondary cause is treated. Primary obesity is treated by diet regimens and eating behavior modification to reduce caloric intake, and exercise regimens to increase expenditure. Anorexiants (amphetimine-like agents), thyroid hormone drugs, and human chorionic gonadotrophin have been used to treat obesity. Surgical small bowel bypass (jujunoileal shunts) is also used to treat severe cases of morbid obesity. '

*Spinal Cord Injury*

It is estimated that there are approximately 11,000 new cases of spinal cord injury every year in the U.S. and that the overall prevalence is a total of 183,000 to 230,000 cases in the U.S. presently (Stover et al., Arch Phys Med Rehabil 80, 1365-71,1999). Recovery from spinal cord injury is very poor and results in devastating irreversible neurologic disability. Current treatment of acute spinal cord injury consists of mechanical stabilization of the injury site, for example by surgical intervention, and the administration of parenteral steroids. These interventions have done little to reduce the incidence of permanent paralysis following spinal cord injury. Treatment of-chronic spinal cord injury is focused on maintenance of quality of life such as the management of pain, spasticity, and bladder function. No currently available treatment addresses the recovery of neurologic function.

One of the factors responsible for such poor recovery after spinal cord injury is the presence of axonal regrowth inhibitors in the myelin sheath. These factors are released shortly after injury and prevent axons from growing across the lesion to re-establish functional connections. One of these axonal regrowth inhibitors is a protein called Nogo-A, an autoantigen of this invention (Huber and Schwab, Biol Chem 381, 407-19., 2000; Reilly, J Neurol 247, 239-40, 2000; Chen et al., Nature 403, 434-9, 2000). Nogo-A has been shown in vitro to inhibit neurite outgrowth, and neutralizing antibodies against Nogo-A have been shown to reverse this growth inhibitory property. Furthermore, monoclonal antibodies against Nogo-A have been shown to promote axonal regrowth in vivo in animal models of spinal cord injury (Raineteau et al., Proc Natl Acad Sci U S A 98, 6929-34., 2001; Merkler et al., J Neurosci 21, 3665-73, 2001; Blochlinger et al., J Comp Neurol 433, 426-36, 2001; Brosamle et al., J Neurosci 20, 8061-8, 2000). Nogo-A is a transmembrane protein expressed mainly in oligodendrocytes within the cerebral cortex and spinal cord. Two regions of the Nogo-A molecule the have been identified as potentially responsible for the inhibitory capacity of this molecule, namely an extracellular 66 amino acid loop and an intracytoplasmic C-terminal region termed AS472.

*Molecular mimicry*

Infectious agents also are known to affect the ability of T-cells to detect autoantigens, a result of molecular mimicry. Without being bound by theory, it is thought that antigens from infectious pathogens that are closely related to self antigens may induce an immune response against the self antigens resulting in an apparent autoimmune disease. Accordingly, such antigens from infectious agents also find use when provide with CD-8 as described herein Examples of infectious agents causing autoimmune disease as a result of molecular mimicry are set forth in Nature Medicine, 7: 8 (Aug, 2001), pp: 899-905..

In some embodiments it is not necessary to co-express a full-length autoantigen with an immunomodulatory molecule of the invention. In this embodiment at least one epitope of the autoantigen is co-expressed with the immunomodulatory molecule of the invention. In a preferred embodiment the epitope is at least about 3, preferably at least about 5 and most preferably at least about 8 amino acids in length. In addition, other truncations of the full-length autoantigen can be co-expressed with the immunomodulatory molecule. When expressing a portion or fragment of an autoantigen it may be desirable to express the fragment as a fusion protein to ensure proper targeting and/or processing of the autoantigen epitope, depending on the desired target cell. In some embodiments, it may be necessary to express a fusion protein that includes the fragment fused to a signal or leader peptide as described herein, for example, to ensure that the polypeptide including the at least one epitope reaches the plasma membrane of the target cell. The signal sequence typically encodes a signal peptide comprised of hydrophobic amino acids which direct the secretion of the protein from the cell, as is well known in the art. In addition, and again depending on the desired target cell, it may be desirable to include a transmembrane region or other membrane anchoring domain in the fusion protein to ensure that the fusion protein remains associated with the target cell expressing it. In one embodiment the transmembrane need not be native to the autoantigen. In this embodiment the "synthetic transmembrane domain" contains from around 20 to 25 hydrophobic amino acids followed by at least one and preferably two charged amino acids.

If the immunomodulatory molecule is encoded by a gene contained in a vector that is separate from the vector comprising and expressing the therapeutic transgene, e.g. the autoantigen, the vector comprising the immunomodulatory molecule can be brought into contact with the cell prior to, simultaneously with, or subsequent to contact of the cell with the vector comprising and expressing the gene, as long as similar or identical types of vectors are used and the timing of the contact effects is sufficient to inhibit an immune response to the vectors brought into contact with the cell. In the present invention, however, the vector encodes both the immunomodulatory molecule and the sequence encoding the autoantigen or the epitope or other fragment thereof. When both the immunomodulatory molecule and therapeutic transgene are expressed in the same vector, preferably the respective inserts are under the control of separate control elements. In this embodiment, the expression system contains either two expression cassettes or a single cassette allowing the simultaneous expression of two nucleic acids (bicistronic unit). When the system comprises two expression cassettes, these can use identical or different promoters.

When the expression system includes the immunomodulatory molecule and therapeutic transgene within a vector, promoters of identical or similar strength may be used, and an identical or similar number of copies of nucleic acids. Generally, the respective quantity of the two transgenes produced *in vivo* is sufficiently close. However, it may be preferable in certain situations to produce different quantities of each transgene. In this case, it is possible to use either promoters of different strength, or a system in which numbers of copies of different genes are present, or to vary the doses administered.

In one embodiment, the therapeutic molecule, e.g. the autoantigen, is delivered to the target cell in the form of a protein or polypeptide for processing via the Class I or Class II MHC pathway for presentation on the surface of the target cell. Either the full length protein or an immunogenic fragment thereof is delivered to the target cell. In this embodiment, the immunomodulatory molecule is preferably delivered to the target cell in an expression system as described herein.

In one embodiment, the therapeutic autoantigen is provided as a fusion protein with MHC class I and/or class II molecules, or is a nucleic acid encoding such a fusion protein. In this embodiment, autoantigen and MHC class I and II molecules are engineered such that the autoantigen is linked, preferably covalently, to the MHC molecule. In some embodiments the autoantigen is directly linked, e.g. adjacent, to the MHC molecule or molecules, as is known in the art. *See, e.g.* Mottez et al., J. Exp. Med. 181 :493-502 (1995). In another embodiment a linker, including but not limited to a peptide linker, is included between the autoantigen and the MHC molecule, a technique also known in the art. *See, e.g.* Kozono et al., nature 369:151-154 (1994); White et al., J. Immunol. 162:2671-76 (1999). Such fusion proteins are described in more detail in U.S. Patent No. 6,211,342.

A "target cell" can be present as a single entity, or can be part of a larger collection of cells. Such a "larger collection of cells" may comprise, for instance, a cell culture (either mixed or pure), a tissue (e.g., epithelial or other tissue), an organ (e.g., heart, lung, liver, gallbladder, urinary bladder, eye or other organ), an organ system (e.g., circulatory system, respiratory system, gastrointestinal system, urinary system, nervous system, integumentary system or other organ system), or an organism (e.g., a bird, mammal, particularly a human, or the like). Preferably, the organs/tissues/cells being targeted are of hematopoietic origin (e.g., including, but not limited to dendritic cells, macrophages, erythrocytes, leukocytes, T and B lymphocytes, and the like) and/or may be hematopoietic or other tissue-specific stem cells. Methods of culturing and using stem cells are disclosed in more detail in U.S. Patent Nos. 5,672,346, 6,143,292 and 6,534,052.

In particular, a target cell with which an expression vector such as a viral vector or plasmid is contacted differs from another cell in that the contacted target cell comprises a particular cell-surface binding site that can be targeted by the expression vector. By "particular cell-surface binding site" is meant any site (*i.e.*, molecule or combination of molecules) present on the surface of a cell with which the vector, *e.g.,* adenoviral vector, can interact in order to attach to the cell and, thereby, enter the cell. A particular cellsurface binding site, therefore, encompasses a cell-surface receptor and, preferably, is a protein (including a modified protein), a carbohydrate, a glycoprotein, a proteoglycan, a lipid, a mucin molecule or mucoprotein, and the like. Examples of potential cell-surface binding sites include, but are not limited to: heparin and chondroitin sulfate moieties found on glycosaminoglycans; sialic acid moieties found on mucins, glycoproteins, and gangliosides; major histocompatability complex I (MHC I) glycoproteins; common carbohydrate molecules found in membrane glycoproteins, including mannose, N-acetyl-galactosamine, N-acetylglucosamine, fucose, and galactose; glycoproteins, such as ICAM-1, VCAM, E-selectin, P-selectin, L-selectin, and integrin molecules; and tumor-specific antigens present on cancerous cells, such as, for instance, MUC-1 tumor-specific epitopes. However, targeting an expression vector such as an adenovirus to a cell is not limited to any specific mechanism of cellular interaction (*i.e.*, interaction with a given cell-surface binding site).

In one embodiment the target cell specificity of an expression vector is altered relative to the wild-type vector by pseudotyping the vector. By pseudotyping is meant that other target cell specific binding moieties are included in the expression vector that confer target cell specificity on the vector. Such target cell specific binding moieties include, but are not limited to, genes encoding proteins that target different cells relative to the wild-type vector, antibody fragments or other binding molecules, such as a leucine zipper, or a biotinavidin binding site. In some embodiments the target cell specific binding moiety is integrated into the viral capsid so that other targeting molecules can be easily attached, e.g. using anticapsid antibodies to attach cell surface molecules or cell surface binding antibodies. Additional examples of pseudotyping vectors are set forth in U.S. Patent Nos. 6,734,014, 6,783,981 and 6,762,031. The expression vectors and methods of the present invention may advantageously include such pseudotyping modifications as well as other targeting modifications and techniques well known to the skilled artisan. *See generally* Curiel and Douglas, Eds., Vector Targeting for Therapeutic Gene Delivery (Wiley-Uss, Inc. 2002).

In a preferred embodiment the vectors are administered systemically and expression of the immunomodulatory molecule and autoantigen epitope can occur in any cell. In this embodiment, when treating autoimmune disease, it is not necessary that the vectors contact the cell or cells subjected to the autoimmune response. Rather, the vector can be administered to or expressed in any cell.

In one embodiment, dendritic cells are used to present antigen to a patient's immune system. Dendritic cells express MHC class I and II, B7 costimulator, and IL-12, and are thus highly specialized antigen presenting cells. Dendritic cells can be used to present peptides to T cells in the context of MHC class I and II molecules. In one embodiment, autologous dendritic cells are pulsed with peptides capable of binding to MHC molecules. In another embodiment, dendritic cells are pulsed with the complete protein. Yet another embodiment involves engineering the overexpression of the gene encoding the antigen in dendritic cells using various implementing vectors known in the art and described herein, such as adenovirus (Arthur et al., 1997, Cancer Gene Ther. 4: 17-25), retrovirus (Henderson et al., 1996, Cancer Res. 56: 3763-3770), lentivirus, adeno-associated virus, DNA transfection (Ribas et al., 1997, Cancer Res. 57: 2865-2869), and tumor-derived RNA transfection (Ashley et al., 1997, J. Exp. Med. 186: 1177-1182), all of which are expressly incorporated herein by reference. Additional examples of such dendritic cell vaccines are found in U.S. Patent No. 6,440,735.

As used herein and further defined below, "polynucleotide" or "nucleic acid" may refer to either DNA or RNA, or molecules which contain both deoxy- and ribonucleotides. The nucleic acids include genomic DNA, cDNA and oligonucleotides including sense and antisense nucleic acids. Such nucleic acids may also contain modifications in the ribosephosphate backbone to increase stability and half-life of such molecules in physiological environments.

The nucleic acid may be double stranded, single stranded, or contain portions of both double stranded or single stranded sequence. As will be appreciated by those in the art, the depiction of a single strand ("Watson") also defines the sequence of the other strand ("Crick"); thus the sequences depicted in the Figures also include the complement of the sequence. By the term "recombinant nucleic acid" herein is meant nucleic acid, originally formed in vitro, in general, by the manipulation of nucleic acid by endonucleases, in a form not normally found in nature. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it may replicate non-recombinantly, i.e. using the in vivo cellular machinery of the host cell rather than in vitro or extrachromosomal manipulations; however, such nucleic acids, once produced recombinantly, although subsequently replicated non-recombinantly, are still considered recombinant for the purposes of the invention.

The terms "polypeptide" and "protein" may be used interchangeably throughout this application and mean at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and norleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard *in vivo* degradation. Alterations of native amino acid sequences to produce variant proteins and peptides for targeting or expression as a transgene, for example, can be done by a variety of means known to those skilled in the art. A variant peptide is a peptide that is substantially homologous to a given peptide, but which has an amino acid sequence that differs from that peptide. The degree of homology (i.e., percent identity) can be determined, for instance, by comparing sequence information using a computer program optimized for such comparison (e.g., using the GAP computer program, version 6.0 or a higher version, described by Devereux et al. (Nucleic Acids Res., 12, 387 (1984)), and freely available from the University of Wisconsin Genetics Computer Group (UWGCG)). The activity of the variant proteins and/or peptides can be assessed using other methods known to those skilled in the art.

In terms of amino acid residues that are not identical between the variant protein (peptide) and the reference protein (peptide), the variant proteins (peptides) preferably comprise conservative amino acid substitutions, i.e., such that a given amino acid is substituted by another amino acid of similar size, charge density, hydrophobicity/hydrophilicity, and/or configuration (e.g., Val for Phe). The variant site-specific mutations can be introduced by ligating into an expression vector a synthesized oligonucleotide comprising the modified site. Alternately, oligonucleotide-directed site-specific mutagenesis procedures can be used, such as those disclosed in Walder et al., Gene, 42:133 (1986); Bauer et al., Gene, 37:73 (1985); Craik, Biotechniques, January 1995, pp. 12-19; and U.S. Patent Nos. 4,518,584 and 4,737,462.

*IMMUNOMODULATORY MOLECULES*

In the context of the present specification, an "immunomodulatory molecule" is a polypeptide molecule that modulates, *i.e,* increases or decreases, a cellular and/or humoral host immune response directed to a target cell in an antigen-specific fashion, and preferably is one that decreases the host immune response. Generally, in accordance with the teachings of the present invention the immunomodulatory molecule(s) will be associated with the target cell surface membrane, *e.g.,* inserted into the cell surface membrane or covalently or non-covalently bound thereto, after expression from the vectors described herein.

The immunomodulatory molecule may comprise all or a functional portion of a CD8 protein, for example all or a functional portion of the CD8 α-chain. For human CD8 coding sequences, see Leahy, Faseb J. 9:17-25 (1995); Leahy et al., Cell 68:1145-62 (1992); Nakayama et al., Immunogenetics 30:393-7 (1989). By "functional portion" with respect to CD8 proteins and polypeptides is meant that portion of the CD8 α-chain retaining veto activity as described herein, more particularly that portion retaining the HLA-binding activity of the CD8 α-chain, and specifically the Ig-like domain in the extracellular region of the CD8 α-chain. Exemplary variant CD8 polypeptides are described in Gao and Jakobsen, Immunology Today 21:630-636 (2000). In some embodiments, the full length CD8 α-chain is used. However, in some embodiments the cytoplasmic domain is deleted. Preferably the transmembrane domain and extracellular domain are retained.

As will be appreciated by those of skill in the art the transmembrane domain of the CD8 α-chain can be exchanged with transmembrane domains of other molecules, if necessary, to modify association of the extracellular domain with the target cell surface. In this embodiment the nucleic acid encoding the extracellular domain of CD8 α-chain is operably linked to a nucleic acid encoding a transmembrane domain. Transmembrane domains of any transmembrane protein can be used in the invention. Alternatively a transmembrane not known to be found in transmembrane proteins may be employed. In this embodiment the "synthetic transmembrane domain" contains from around 20 to 25 hydrophobic amino acids followed by at least one and preferably two charged amino acids. In some embodiments the CD8 extracellular domain is linked to the target cell membrane by conventional techniques in the art. Preferred CD8 α-chain sequences are set forth in the following table and include the accession numbers for the full length sequences of either the amino acid sequence or nucleic acid sequence encoding a full length CD8 α-chain from species including human, mouse, rat, orangutan, spider monkey, guinea pig, cow, Hispid cotton rat, domestic pig and cat. The sequences included at the accession numbers are expressly incorporated herein by reference.

### CD8 α-chain Sequences

| Species | Accession Number |
| --- | --- |
| Homo sapiens | NM_001768, M27161 and NM_171827 |
| Pongo pygmaeus (Orangutan) | X60223 |
| Mus musculus (Mouse) | XM_132621, BC030679 and U34881 |
| Rattus norveticus (Rat) | NM_031538 |
| Cavia porcellus (Guinea Pig) | AY303773 |
| Bos Taurus (Cow) | NM_174015 |
| Sus scrofa (Domestic Pig) | AY517855 |
| Felis catus (Cat) | D16536 |
| Sigmodon hispidus (Hispid cotton rat) | AY065643 |
| Saimiri sciureus (Common squirrel monkey) | AJ130818 |

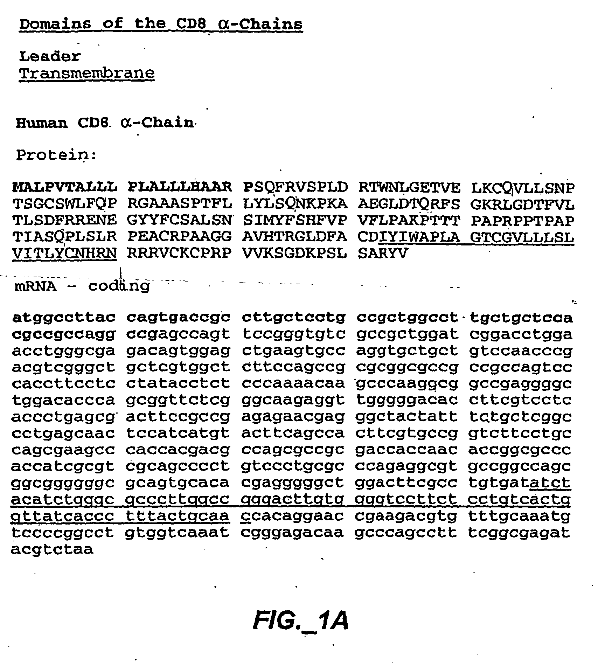

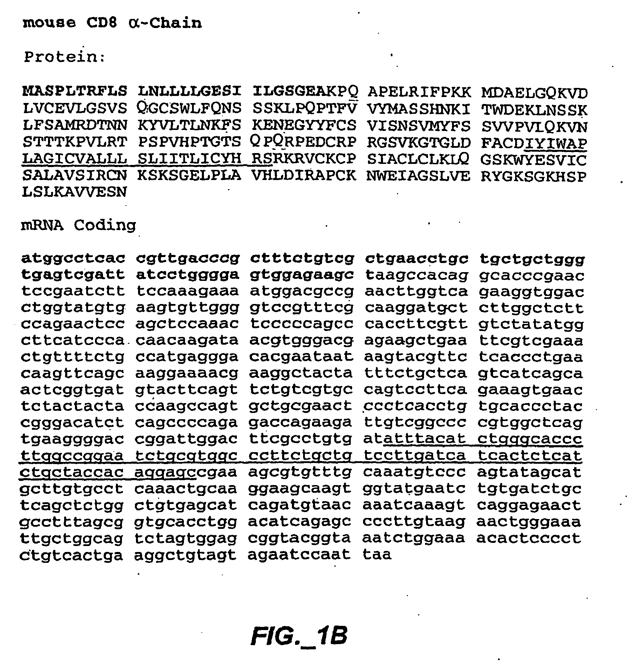

In a preferred embodiment the CD8 α-chain is not a fusion protein, but rather is a truncation protein wherein the intracellular domain is deleted. As depicted in Figures 1A-B, the human CD8 α-chain gene expresses a protein of 235 amino acids. The protein can be considered to be divided into the following domains (starting at the amino terminal and ending at the carboxy terminal of the polypeptide): a signal peptide (amino acids 1 to 21); immunoglobulin (Ig-like domain (approximately amino acids 22-136); membrane proximal stalk region (amino acids 137-181); transmembrane domain (amino acids 183-210) and cytoplasmic domain (amino acids 211-235). The nucleotides of the coding sequence that encode these different domains include 1-63 encoding the signal peptide, 64-546 encoding the extracellular domain, about 547-621 encoding the intracellular domain and about 622-708 encoding the intracellular domain. Likewise, the mouse sequences can be divided into domains as follows. The polypeptide can be divided into a signal sequence including amino acids 1-27, an extracellular domain including about amino acids 28 to 194, a transmembrane domain including about amino acids 195-222 and an intracellular domain including about amino acids 223-310. Similarly, the nucleotides of the coding sequence encoding these domain include nucleic acid 1-81 encoding signal peptide, about 82-582 encoding extracellular domain, about 583-666 encoding transmembrane domain and about 667-923 encoding the extracellular domain.

In some embodiments nucleic acid encoding the full length protein is included in the gene delivery vehicle. In other embodiments, nucleic acids encoding the intracellular domain are not included in the polynucleotide in the gene delivery vehicle resulting in a membrane anchored protein lacking the intracellular domain. Corresponding domains also can be identified in other species, including in preferred embodiments the mouse.

One skilled in the art will also appreciate that immunomodulatory molecules having substantial homology to the afore-mentioned polypeptides may find advantageous use in the invention. Accordingly, for example, also encompassed by "CD8 polypeptides" are homologous polypeptides having at least about 80% sequence identity, usually at least about 85% sequence identity, preferably at least about 90% sequence identity, more preferably at least about 95% sequence identity and most preferably at least about 98% sequence identity with the polypeptide encoded by nucleotides shown in Figure 1A.

By "nucleic acid molecules encoding CD8", and grammatical equivalents thereof is meant the nucleotide sequence of human CD8 as shown in Figure 1A as well as nucleotide sequences having at least about 80% sequence identity, usually at least about 85% sequence identity, preferably at least about 90% sequence identity, more preferably at least about 95% sequence identity and most preferably at least about 98% sequence identity with nucleotides shown in Figure 1A and which encode a polypeptide having the sequence shown in Figure 1A.

As noted previously, a number of different programs can be used to identify whether a protein or nucleic acid has sequence identity or similarity to a known sequence. Sequence identity and/or similarity is determined using standard techniques known in the art, including, but not limited to, the local sequence identity algorithm of Smith & Waterman, Adv. Appl. Math. 2:482 (1981), by the sequence identity alignment algorithm of Needleman & Wunsch, J. Mol. Biol. 48: 443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387-395 (1984), preferably using the default settings, or by inspection. Preferably, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351-360 (1987); the method is similar to that described by Higgins & Sharp CABIOS 5:151-153 (1989). Useful PILEUP parameters including a default gap weight of 3.00, a default gap length weight of 0.10, and weighted end gaps.

Another example of a useful algorithm is the BLAST algorithm, described in Altschul et al., J. Mol. Biol. 215, 403-410, (1990) and Karlin et al., PNAS USA 90:5873-5787 (1993). A particularly useful BLAST program is the WU-BLAST-2 program which was obtained from Altschul et al., Methods in Enzymology, 266: 460-480 (1996); http://blast.wustl/edu/blast/ README.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al. Nucleic Acids Res. 25:3389-3402. Gapped BLAST uses BLOSUM-62 substitution scores; threshold T parameter set to 9; the two-hit method to trigger ungapped extensions; charges gap lengths of k a cost of 10+k; Xu set to 16, and Xg set to 40 for database search stage and to 67 for the output stage of the algorithms. Gapped alignments are triggered by a score corresponding to -22 bits.

A % amino acid or nucleic acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

The alignment may include the introduction of gaps in the sequences to be aligned. In addition, for sequences which contain either more or fewer amino acids than the amino acid sequence of the polypeptide encoded by nucleotides shown in Figure 1A, it is understood that in one embodiment, the percentage of sequence identity will be determined based on the number of identical amino acids in relation to the total number of amino acids. Thus, for example, sequence identity of sequences shorter than that of the polypeptide encoded by nucleotides in Figure 1A, as discussed below, will be determined using the number of amino acids in the shorter sequence, in one embodiment. In percent identity calculations relative weight is not assigned to various manifestations of sequence variation, such as, insertions, deletions, substitutions, etc.

In one embodiment, only identities are scored positively (+1) and all forms of sequence variation including gaps are assigned a value of "0", which obviates the need for a weighted scale or parameters as described below for sequence similarity calculations. Percent sequence identity can be calculated, for example, by dividing the number of matching identical residues by the total number of residues of the "shorter" sequence in the aligned region and multiplying by 100. The "longer" sequence is the one having the most actual residues in the aligned region.

CD8 having less than 100% sequence identity with the polypeptide encoded by nucleotides in Figure 1A will generally be produced from native CD8 nucleotide sequences from species other than human and variants of native CD8 nucleotide sequences from human or non-human sources. In this regard, it is noted that many techniques are well known in the art and may be routinely employed to produce nucleotide sequence variants of native CD8 sequences and assaying the polypeptide products of those variants for the presence of at least one activity that is normally associated with a native CD8 polypeptide. In a preferred embodiment the CD8 α-chain is from human but as shown in TABLE 2, CD8 α-chain from rat, mouse, and primates are known and find use in the invention.

Polypeptides having CD8 activity may be shorter or longer than the polypeptide encoded by nucleotides depicted in Figure 1A. Thus, in a preferred embodiment, included within the definition of CD8 polypeptide are portions or fragments of the polypeptide encoded by nucleotides in Figure 1A. In one embodiment herein, fragments of the polypeptide encoded by nucleotides in Figure 1A are considered CD8 polypeptides if a) they have at least the indicated sequence identity; and b) preferably have a biological activity of naturally occurring CDB, as described above.

In addition, as is more fully outlined below, CD8 α-chain can be made longer than the polypeptide encoded by nucleotides in Figure 1A; for example, by the addition of other fusion sequences, or the elucidation of additional coding and non-coding sequences.

The CD8 polypeptides are preferably recombinant. A "recombinant polypeptide" is a polypeptide made using recombinant techniques, i.e. through the expression of a recombinant nucleic acid as described below. In a preferred embodiment, CD8 of the invention is made through the expression of nucleic acid sequence shown in Figure 1A, or fragment thereof. A recombinant polypeptide is distinguished from naturally occurring protein by at least one or more characteristics. For example, the polypeptide may be isolated or purified away from some or all of the proteins and compounds with which it is normally associated in its wild type host, and thus may be substantially pure. For example, an isolated polypeptide is unaccompanied by at least some of the material with which it is normally associated in its natural state, preferably constituting at least about 0.5%, more preferably at least about 5% by weight of the total protein in a given sample. A substantially pure polypeptide comprises at least about 75% by weight of the total polypeptide, with at least about 80% being preferred, and at least about 90% being particularly preferred. The definition includes the production of a CD8 polypeptide from one organism in a different organism or host cell.

Alternatively, the polypeptide may be made at a significantly higher concentration than is normally seen, through the use of a inducible promoter or high expression promoter, such that the polypeptide is made at increased concentration levels. Alternatively, the polypeptide may be in a form not normally found in nature, as in the addition of amino acid substitutions, insertions and deletions, as discussed below.

Nucleic acid CD8 variants fall into one or more of three classes: substitutional, insertional or deletional variants. These variants ordinarily are prepared by site specific mutagenesis of nucleotides in nucleotides of Figure 1A, using cassette or PCR mutagenesis or other techniques well known in the art, to produce DNA encoding the variant, including the variant in a gene therapy vector and thereafter expressing the DNA. Amino acid sequence variants are characterized by the predetermined nature of the variation, a feature that sets them apart from naturally occurring allelic or interspecies variation of CD8 amino acid sequence. The variants typically exhibit the same qualitative biological activity as the naturally occurring analogue, although variants can also be selected which have modified characteristics as will be more fully outlined below.

While the site or region for introducing a sequence variation is predetermined, the mutation per se need not be predetermined. For example, in order to optimize the performance of a mutation at a given site, random mutagenesis may be conducted at the target codon or region and the expressed variants screened for the optimal desired activity. Techniques for making substitution mutations at predetermined sites in DNA having a known sequence are well known, for example, M13 primer mutagenesis and PCR mutagenesis. Another example of a technique for making variants is the method of gene shuffling, whereby fragments of similar variants of a nucleotide sequence are allowed to recombine to produce new variant combinations. Examples of such techniques are found in U.S. Patent Nos. 5,605,703; 5,811,238; 5,873,458; 5,830,696; 5,939,250; 5,763,239; 5,965,408; and 5,945,325.

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger and may include the cytoplasmic domain or fragments thereof.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the CD8 are desired, substitutions are generally made in accordance with the following chart:

**Chart 1**

| Original Residue | Exemplary Substitutions |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |

| Leu | Ile, Val |
|-----|----------|
| Lys | Arq, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Substantial changes in function or immunological identity are made by selecting substitutions that are less conservative than those shown in Chart 1. For example, substitutions may be made which more significantly affect: the structure of the polypeptide backbone in the area of the alteration, for example the alpha-helical or beta-sheet structure; the charge or hydrophobicity of the molecule at the target site; or the bulk of the side chain. The substitutions which in general are expected to produce the greatest changes in the polypeptide's properties are those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine.

The variants typically exhibit the same qualitative biological activity and will elicit the same immune response as the naturally occurring analogue, although variants also are selected to modify the characteristics of the CD8 as needed. Alternatively, the variant may be designed such that the biological activity of the protein is altered.

One type of covalent modification of a polypeptide included within the scope of this invention comprises altering the native glycosylation pattern of the polypeptide. "Altering the native glycosylation pattern" is intended for purposes herein to mean deleting one or more carbohydrate moieties found in native sequence CD8 polypeptide, and/or adding one or more glycosylation sites that are not present in the native sequence polypeptide.

Addition of glycosylation sites to polypeptides may be accomplished by altering the amino acid sequence thereof. The alteration may be made, for example, by the addition of, or substitution by, one or more serine or threonine residues to the native sequence polypeptide (for O-linked glycosylation sites). The amino acid sequence may optionally be altered through changes at the DNA level, particularly by mutating the DNA encoding the polypeptide at preselected bases such that codons are generated that will translate into the desired amino acids.

Removal of carbohydrate moieties present on the polypeptide may be accomplished by mutational substitution of codons encoding for amino acid residues that serve as targets for glycosylation.

Once isolated from its natural source, e.g., contained within a plasmid or other vector or excised therefrom as a linear nucleic acid segment, the recombinant nucleic acid can be further-used as a probe to identify and isolate other nucleic acids. It can also be used as a "precursor" nucleic acid to make modified or variant nucleic acids and proteins. It also can be incorporated into a vector or other delivery vehicle for treating target cells as described herein.

*Expression Vectors*

In the context of the present invention, any suitable expression vector can be used. A "vector" is a vehicle for gene transfer as that term is understood by those of skill in the art. The expression vectors according to the invention include, but are not limited to, plasmids, phages, viruses, liposomes, and the like. An expression vector according to the invention preferably comprises additional sequences and mutations. In particular, an expression vector according to the invention comprises a nucleic acid comprising a transgene encoding an immunomodulatory molecule, particularly a CD8 α-chain, as defined herein. In addition, the vector includes sequences encoding at least one epitope comprising an autoantigen as described herein. The nucleic acid may comprise a wholly or partially synthetically made coding or other genetic sequence or a genomic or complementary DNA (cDNA) sequence, and can be provided in the form of either DNA or RNA.

A gene encoding for an autoantigen can be moved to or from a viral vector or into a baculovirus or a suitable prokaryotic or eukaryotic expression vector for expression of mRNA and production of protein, and for evaluation of other biochemical characteristics.

In terms of the production of vectors according to the invention (including recombinant adenoviral vectors and transfer vectors), such vectors can be constructed using standard molecular and genetic techniques, such as those known to those skilled in the art. Vectors comprising virions or viral particles (*e.g.*, recombinant adenoviral vectors) can be produced using viral vectors in the appropriate cell lines. Similarly, particles comprising one or more chimeric coat proteins can be produced in standard cell lines, *e.g.,* those currently used for adenoviral vectors. These resultant particles then can be targeted to specific cells, if desired.

Any appropriate expression vector *(e.g.,* as described in Pouwels et al., Cloning Vectors: A Laboratory Manual (Elsevior, N.Y.: 1985)) and corresponding suitable host cell can be employed for production of a recombinant peptide or protein in a host cell. Expression hosts include, but are not limited to, bacterial species within the genera Escherichia, Bacillus, Pseudomonas, Salmonella, mammalian or insect host cell systems, including baculoviral systems (*e.g.*, as described by Luckow et al., Bio/Technology, 6, 47 (1988)), and established cell lines, such as COS-7, C127, 3T3, CHO, HeLa, BHK, and the like. An especially preferred expression system for preparing chimeric proteins (peptides) according to the invention is the baculoviral expression system wherein Trichoplusia ni, Tn 5B1-4 insect cells, or other appropriate insect cells, are used to produce high levels of recombinant proteins. The ordinary skilled artisan is, of course, aware that the choice of expression host has ramifications for the type of peptide produced. For instance, the glycosylation of peptides produced in yeast or mammalian cells (e.g., COS-7 cells) will differ from that of peptides produced in bacterial cells, such as Escherichia coli.

In a preferred embodiment, the proteins are expressed in mammalian cells. Mammalian expression systems are also known in the art, and include retroviral and lentiviral systems. A mammalian promoter is any DNA sequence capable of binding mammalian RNA polymerase and initiating the downstream (3') transcription of a coding sequence for a protein into mRNA. A promoter will have a transcription initiating region, which is usually placed proximal to the 5' end of the coding sequence, and a TATA box, using a located 25-30 base pairs upstream of the transcription initiation site. The TATA box is thought to direct RNA polymerase II to begin RNA synthesis at the correct site. A mammalian promoter will also contain an upstream promoter element (enhancer element), typically located within 100 to 200 base pairs upstream of the TATA box. An upstream promoter element determines the rate at which transcription is initiated and can act in either orientation. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter.

Typically, transcription termination and polyadenylation sequences recognized by mammalian cells are regulatory regions located 3' to the translation stop codon and thus, together with the promoter elements, flank the coding sequence of the inserts for both the immunomodulatory molecule and the epitope of the auto-antigen. The 3' terminus of the mature mRNA is formed by site-specific post-translational cleavage and polyadenylation. Examples of transcription terminator and polyadenlytion signals include those derived form SV40.

The methods of introducing exogenous nucleic acid into mammalian hosts, as well as other hosts, is well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

The protein may also be made as a fusion protein, using techniques well known in the art. Thus, for example, the protein may be made as a fusion protein to increase expression, or for other reasons. For example, when the protein is a peptide, the nucleic acid encoding the peptide may be linked to other nucleic acid for expression purposes, as described herein. In a preferred embodiment when expressing an epitope of the autoantigen, the protein is expressed as a fusion protein to ensure appropriate targeting and processing. That is, the polypeptide comprising the epitope is expressed as a fusion protein that may include such processing sequences as a signal sequence to target the polypeptide to the secretory pathway. The fusion protein also may include a transmembrane domain to immobilize the polypeptide comprising the epitope of the autoantigen to the cell membrane such that both the immunomodulatory molecule and the epitope of the autoantigen are co-expressed on the cell surface.

To test for CD8 or an autoantigen, the protein is purified or isolated after expression. Proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the CD8 protein may be purified using a standard anti-CD8 antibody column. Ultrafiltration and diafiltration techniques, in conjunction with protein concentration, are also useful. For general guidance in suitable purification techniques, see Scopes, R., Protein Purification, Springer-Verlag, NY (1982). The degree of purification necessary will vary depending on the use of the CD8 protein. In some instances no purification will be necessary. In some instances CD8 and/or autoantigen expression is detected on the cell surface, for example by antibody binding and detection via fluorescence or by Fluorescence Activated Cell Sorting (FACS).

Nucleic acid molecules encoding CD8 and an autoantigen as well as any nucleic acid molecule derived from either the coding or non-coding strand of a CD8 nucleic acid molecule may be contacted with cells of an target in a variety of ways that are known and routinely employed in the art, wherein the contacting may be *ex vivo* or *in vivo*.

Viral attachment, entry and gene expression can be evaluated initially by using the adenoviral vector containing the insert of interest to generate a recombinant virus expressing the desired protein or RNA and a marker gene, such as ß-galactosidase. ß-galactosidase expression in cells infected with adenovirus containing the ß-galactosidase gene (Ad-LacZ) can be detected as early as two hours after adding Ad-Gluc to cells. This procedure provides a quick and efficient analysis of cell entry of the recombinant virus and gene expression, and is implemented readily by an artisan of ordinary skill using conventional techniques.

Using the nucleic acids of the present invention that encode a protein, a variety of expression vectors can be made. The expression vectors may be either self-replicating extrachromosomal vectors or vectors which integrate into a host genome. Generally, these expression vectors include transcriptional and translational regulatory nucleic acid operably linked to the nucleic acid encoding the protein. The term "control sequences" refers to transcriptional and translational regulatory nucleic acid sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. As another example, operably linked refers to DNA sequences linked so as to be contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. The transcriptional and translational regulatory nucleic acid will generally be appropriate to the host cell used to express the CD8 and autoantigen; for example, human transcriptional and translational regulatory nucleic acid sequences are preferably used to express the CD8 and autoantigen in human cells. Numerous types of appropriate expression vectors, and suitable regulatory sequences are known in the art for a variety of host cells.

In general, the transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences encode either constitutive or inducible promoters. The promoters may be either naturally occurring promoters or hybrid promoters. Hybrid promoters, which combine elements of more than one promoter, are also known in the art, and are useful in the present invention.

In addition, the expression vector may comprise additional elements. For example, the expression vector may have two replication systems, thus allowing it to be maintained in two organisms, for example in mammalian or insect cells for expression and in a procaryotic host for cloning and amplification. Furthermore, for integrating expression vectors, the expression vector contains at least one sequence homologous to the host cell genome, and preferably two homologous sequences which flank the expression construct. The integrating vector may be directed to a specific locus in the host cell by selecting the appropriate homologous sequence for inclusion in the vector. Constructs for integrating vectors are well known in the art.

In a further embodiment, the expression vector may contain a selectable marker gene to allow the selection of transformed host cells. Selection genes are well known in the art and will vary with the host cell used.

Preferably, the vector is a viral vector, such as an adenoviral vector, an adeno-associated viral vector, a herpes vector or a retroviral vector, among others. Most preferably, the viral vector is an adenoviral vector. An adenoviral vector can be derived from any adenovirus. An "adenovirus" is any virus of the family Adenoviridae, and desirably is of the genus Mastadenovirus (e.g., mammalian adenoviruses) or Aviadenovirus (e.g., avian adenoviruses). The adenovirus is of any serotype. Adenoviral stocks that can be employed as a source of adenovirus can be amplified from the adenoviral serotypes 1 through 47, which are currently available from the American Type Culture Collection (ATCC, Rockville, Md.), or from any other serotype of adenovirus available from any other source. For instance, an adenovirus can be of subgroup A (e.g., serotypes 12, 18, and 31), subgroup B (e.g., serotypes 3, 7, 11, 14, 16, 21, 34, and 35), subgroup C (e.g., serotypes 1, 2, 5, and 6), subgroup D (e.g., serotypes 8, 9, 10, 13, 15, 17, 19, 20, 22-30, 32, 33, 36-39, and 42-47), subgroup E (serotype 4), subgroup F (serotypes 40 and 41), or any other adenoviral serotype. Preferably, however, an adenovirus is of serotypes 2, 5 or 9. Desirably, an adenovirus comprises coat proteins (e.g., penton base, hexon, and/or fiber) of the same serotype. However, also preferably, one or more coat proteins can be chimeric, in the sense, for example, that all or a part of a given coat protein can be from another serotype.

Although the viral vector, which is preferably an adenoviral vector, can be replication-competent, preferably, the viral vector is replication-deficient or conditionally replication-deficient. For example, the viral vector which is preferably an adenoviral vector, comprises a genome with at least one modification that renders the virus replication-deficient. The modification to the viral genome includes, but is not limited to, deletion of a DNA segment, addition of a DNA segment, rearrangement of a DNA segment, replacement of a DNA segment, or introduction of a DNA lesion. A DNA segment can be as small as one nucleotide or as large as 36 kilobase pairs, i.e., the approximate size of the adenoviral genome, or 38 kilobase pairs, which is the maximum amount that can be packaged into an adenoviral virion.

Preferred modifications to the viral, in particular adenoviral, genome include, in addition to a modification that renders the virus replication-deficient, the insertion of a transgene encoding for an immunomodulatory molecule as defined herein and, additionally and preferably, at least one transgene encoding for a therapeutic molecule of interest. A virus, such as an adenovirus, also preferably can be a cointegrate, i.e., a ligation of viral, such as adenoviral, genomic sequences with other sequences, such as those of a plasmid, phage or other virus.

In terms of an adenoviral vector (particularly a replication-deficient adenoviral vector), such a vector can comprise either complete capsids (i.e., including a viral genome, such as an adenoviral genome) or empty capsids (i.e., in which a viral genome is lacking, or is degraded, e.g., by physical or chemical means). Preferably, the viral vector comprises complete capsids, i.e., as a means of carrying the transgene encoding for the immunomodulatory molecule and, optionally and preferably, at least one transgene encoding an inhibiting means. Alternatively, preferably, the transgenes may be carried into a cell on the outside of the adenoviral capsid.

To the extent that it is preferable or desirable to target a virus, such as an adenovirus, to a particular cell, the virus can be employed essentially as an endosomolytic agent in the transfer into a cell of plasmid DNA, which contains a marker gene and is complexed and condensed with polylysine covalently linked to a cell-binding ligand, such as transferrin (Cotten et al., PNAS (USA), 89, 6094-6098 (1992); and Curiel et al., PNAS (USA), 88, 8850-8854 (1991)). It has been demonstrated that coupling of the transferrin-polylysine/DNA complex and adenovirus (e.g., by means of an adenovirus-directed antibody, with transglutaminase, or via a biotin/streptavidin bridge) substantially enhances gene transfer (Wagner et al., PNAS (USA), 89, 6099-6103 (1992)).

Alternatively, one or more viral coat proteins, such as the adenoviral fiber, can be modified, for example, either by incorporation of sequences for a ligand to a cell-surface receptor or sequences that allow binding to a bispecific antibody (i.e., a molecule with one end having specificity for the fiber, and the other end having specificity for a cell-surface receptor) (PCT international patent application no. WO 95/26412 (the '412 application) and Watkins et al., "Targeting Adenovirus-Mediated Gene Delivery with Recombinant Antibodies," Abst. No. 336). In both cases, the typical fiber/cell-surface receptor interactions are abrogated, and the virus, such as an adenovirus, is redirected to a new cell-surface receptor by means of its fiber.

Alternatively, a targeting element, which is capable of binding specifically to a selected cell type, can be coupled to a first molecule of a high affinity binding pair and administered to a host cell (PCT international patent application no. WO 95/31566). Then, a gene delivery vehicle coupled to a second molecule of the high affinity binding pair can be administered to the host cell, wherein the second molecule is capable of specifically binding to the first molecule, such that the gene delivery vehicle is targeted to the selected cell type.

Along the same lines, since methods (e.g., electroporation, transformation, conjugation of triparental mating, (co-)transfection, (co-) infection, membrane fusion, use of microprojectiles, incubation with calcium phosphate-DNA precipitate, direct microinjection; etc.) are available for transferring viruses, plasmids, and phages in the form of their nucleic acid sequences (i.e., RNA or DNA), a vector similarly can comprise RNA or DNA, in the absence of any associated protein, such as capsid protein, and in the absence of any envelope lipid.

Similarly, since liposomes effect cell entry by fusing with cell membranes, a vector can comprise liposomes, with constitutive nucleic acids encoding the coat protein. Such liposomes are commercially available, for instance, from Life Technologies, Bethesda, Md., and can be used according to the recommendation of the manufacturer. Moreover, a liposome can be used to effect gene delivery and liposomes having increased transfer capacity and/or reduced toxicity *in vivo* can be used. The soluble chimeric coat protein (as produced using methods described herein) can be added to the liposomes either after the liposomes are prepared according to the manufacturer's instructions, or during the preparation of the liposomes.

The vectors according to the invention are not limited to those that can be employed in the method of the invention, but also include intermediary-type vectors (e.g., "transfer vectors") that can be employed in the construction of gene transfer vectors.

One of the preferred methods for *in vivo* delivery of one or more nucleic acid sequences involves the use of an adenovirus expression vector. "Adenovirus expression vector" is meant to include those constructs containing adenovirus sequences sufficient (a) to support packaging of the construct and (b) to express a polynucleotide that has been cloned therein in a sense or antisense orientation. Of course, in the context of an antisense construct, expression does not require that the gene product be synthesized.

The expression vector comprises a genetically engineered form of an adenovirus. Knowledge of the genetic organization of adenovirus, a 36 kb, linear, double-stranded DNA virus, allows substitution of large pieces of adenoviral DNA with foreign sequences up to 7 kb (Grunhaus and Horwitz, 1992). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

Adenovirus is particularly suitable for use as a gene transfer vector because of its mid-sized genome, ease of manipulation, high titer, wide target-cell range and high infectivity. Both ends of the viral genome contain 100-200 base pair inverted repeats (ITRs), which are cis elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off (Renan, 1990). The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNA's issued from this promoter possess a 5'-tripartite leader (TPL) sequence which makes them preferred mRNA's for translation.

In a current system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the adenovirus vectors, which are replication deficient, depend on a unique helper cell line. In nature, adenovirus can package approximately 105% of the wild-type genome (Ghosh-Choudhury et al., 1987), providing capacity for about 2 extra kB of DNA. Combined with the approximately 5.5 kB of DNA that is replaceable in the E1 and E3 regions, the maximum capacity of the current adenovirus vector is under 7.5 kB, or about 15% of the total length of the vector. More than 80% of the adenovirus viral genome remains in the vector backbone and is the source of vector-bome cytotoxicity. Also, the replication deficiency of the E1-deleted virus is incomplete. For example, leakage of viral gene expression has been observed with the currently available vectors at high multiplicities of infection (MOI) (Mulligan, 1993).

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, e.g., Vero cells or other monkey embryonic mesenchymal or epithelial cells. As stated above, the currently preferred helper cell line is 293.

Recently, Racher et al. (1995) disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for 1 to 4 h. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 h.

In a preferred embodiment the adenovirus is a "gutless" adenovirus as is known in the art. The "gutless" adenovirus vector is a recently developed system for adenoviral gene delivery. The replication of the adenovirus requires a helper virus and a special human 293 cell line expressing both E 1a and Cre, a condition that does not exist in natural environment. In the most efficient system to date, an E1-deleted helper virus is used with a packaging signal that is flanked by bacteriophage P1 loxP sites ("floxed"). Infection of the helper cells that express Cre recombinase with the gutless virus together with the helper virus with a floxed packaging signal should only yield gutless rAV, as the packaging signal is deleted from the DNA of the helper virus. However, if 293-based helper cells are used, the helper virus DNA can recombine with the Ad5 DNA that is integrated in the helper cell DNA. As a result, a wild-type packaging signal, as well as the E1 region, is regained. Thus, also production of gutless rAV on 293- (or 911-) based helper cells can result in the generation of RCA, if an E1-deleted helper virus is used.

The vector is deprived of all viral genes. Thus the vector is non-immunogenic and may be used repeatedly, if necessary. The "gutless" adenovirus vector also contains 36 kb space for accommodating transgenes, thus allowing co-delivery of a large number of genes into cells. Specific sequence motifs such as the RGD motif may be inserted into the H-I loop of an adenovirus vector to enhance its infectivity. An adenovirus recombinant is constructed by cloning specific transgenes or fragments of transgenes into any of the adenovirus vectors such as those described herein and known in the art. The adenovirus recombinant can be used to transduce epidermal cells of a vertebrate in a non-invasive mode for use as an immunizing agent.

Use of the "gutless" adenoviruses is particularly advantageous for insertion of large inserts of heterologous DNA (for a review, see Yeh. and Perricaudet, FASEB J. 11 :615 (1997)). In addition, gutless adenoviral vectors and methods of making and using them are described in more detail in U.S. Patent No. 6,156,497 and 6,228,646.

Other than the requirement that the adenovirus vector be replication defective, or at least conditionally defective, the nature of the adenovirus vector is not believed to be crucial to the successful practice of the invention. The adenovirus may be of any of the 42 different known serotypes or subgroups A-F. Adenovirus type 5 of subgroup C is the preferred starting material in order to obtain a conditional replication-defective adenovirus vector for use in the present invention, since Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E1 region. Thus, it will be most 'convenient to introduce the transgene encoding the immunomodulatory molecule and/or additional therapeutic protein of interest at the position from which the E1-coding sequences have been removed. However, the position of insertion of the expression construct within the adenovirus sequences is not critical to the invention. The transgene(s) of interest may also be inserted in lieu of the deleted E3 region in E3 replacement vectors as described by Karlsson et al. (1986) or in the E4 region where a helper cell line or helper virus complements the E4 defect.

Adenovirus is easy to grow and manipulate and exhibits broad host range in vitro and in vivo. This group of viruses can be obtained in high titers, e.g., $10^9$ -$10^{11}$ plaqueforming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus (Couch et al., 1963; Top et al., 1971), demonstrating their safety and therapeutic potential as in vivo gene transfer vectors.

Adenovirus vectors have been used in eukaryotic gene expression (Levrero et al., 1991; Gomez-Foix et al., 1992) and vaccine development (Grunhaus and Horwitz, 1992; Graham and Prevec, 1992). Recently, animal studies suggested that recombinant adenovirus could be used for gene therapy (Stratford-Perricaudet and Perricaudet, 1991; Stratford-Perricaudet et al., 1990; Rich et al., 1993). Studies in administering recombinant adenovirus to different tissues include trachea instillation (Rosenfeld et al., 1991; Rosenfeld et al., 1992), muscle injection (Ragot et al., 1993), peripheral intravenous injections (Herz and Gerard, 1993) and stereotactic inoculation into the brain (Le Gal La Salle et al., 1993).

Accordingly, in a preferred embodiment, the expression vectors used herein are adenoviral vectors. Suitable adenoviral vectors include modifications of human adenoviruses such as Ad2 or Ad5, wherein genetic elements necessary for the virus to replicate *in vivo* have been removed; e.g. the E 1 region, and an expression cassette coding for the exogenous gene of interest inserted into the adenoviral genome.

In addition, as described above, a preferred expression vector system is a retroviral vector system such as is generally described in PCT/US97/01019 and PCT/US97/01048.

The retroviruses are a group of single-stranded RNA viruses characterized by an ability to convert their RNA to double-stranded DNA in infected cells by a process of reverse-transcription (Coffin, 1990). The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome (Coffin, 1990).

In order to construct a retroviral vector, a nucleic acid encoding one or more oligonucleotide or polynucleotide sequences of interest is inserted into the viral genome in the place of certain viral sequences to produce a virus that is replication-defective. In order to produce virions, a packaging cell line containing the gag, pol, and env genes but without the LTR and packaging components is constructed (Mann et al., 1983). When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann et al., 1983). The media containing the recombinant retroviruses is then collected, optionally concentrated, and used for gene transfer. Retroviral vectors are able to infect a broad variety of cell types. However, integration and stable expression require the division of host cells (Paskind et al., 1975).

A novel approach designed to allow specific targeting of retrovirus vectors was recently developed based on the chemical modification of a retrovirus by the chemical addition of lactose residues to the viral envelope. This modification could permit the specific infection of hepatocytes via sialoglycoprotein receptors.

A different approach to targeting of recombinant retroviruses was designed in which, biotinylated antibodies against a retroviral envelope protein and against a specific cell receptor were used. The antibodies were coupled via the biotin components by using streptavidin (Roux et al., 1989). Using antibodies against major histocompatibility complex class I and class II antigens, they demonstrated the infection of a variety of human cells that bore those surface antigens with an ecotropic virus *in vitro* (Roux et al., 1989). Suitable retroviral vectors include LNL6, LXSN, and LNCX (see Byun et al., Gene Ther. 3(9):780-8 (1996 for review).

AAV (Ridgeway, 1988; Hermonat and Muzycska, 1984) is a parvovirus, discovered as a contamination of adenoviral stocks. It is a ubiquitous virus (antibodies are present in 85% of the US human population) that has not been linked to any disease. It is also classified as a dependovirus, because its replication is dependent on the presence of a helper virus, such as adenovirus. Five serotypes have been isolated, of which AAV-2 is the best characterized. AAV has a single-stranded linear DNA that is encapsidated into capsid proteins VP1, VP2 and VP3 to form an icosahedral virion of 20 to 24 nm in diameter (Muzyczka and McLaughlin, 1988).

The AAV DNA is approximately 4.7 kilobases long. It contains two open reading frames and is flanked by two ITRs. There are two major genes in the AAV genome: rep and cap. The rep gene codes for proteins responsible for viral replications, whereas cap codes for capsid protein VP1-3. Each ITR forms a T-shaped hairpin structure. These terminal repeats are the only essential cis components of the AAV for chromosomal integration. Therefore, the AA V can be used as a vector with all viral coding sequences removed and replaced by the cassette of genes for delivery. Three viral promoters have been identified and named p5, p19, and p40, according to their map position. Transcription from p5 and p19 results in production of rep proteins, and transcription from p40 produces the capsid proteins (Hermonat and Muzyczka, 1984).

AAV is also a good choice of delivery vehicles due to its safety. There is a relatively complicated rescue mechanism: not only wild type adenovirus but also AAV genes are required to mobilize rAAV. Likewise, AAV is not pathogenic and not associated with any disease. The removal of viral coding sequences minimizes immune reactions to viral gene expression, and therefore, rAAV does not evoke an inflammatory response. Other disclosure related to AAV is set forth in U.S. Patent No. 6,531,456.

Other viral vectors may be employed as expression vectors in the present invention for the delivery of immunomodulatory molecules to a host cell. Vectors derived from viruses such as vaccinia virus (Ridgeway, 1988; Coupar et al, 1988), lentiviruses, polio viruses and herpes viruses may be employed. They offer several attractive features for various mammalian cells (Friedmann, 1989; Ridgeway, 1988; Coupar et al., 1988; Horwich et al., 1990).

*Delivery of Expression Vectors*

In order to effect expression of the immunomodulatory molecule (e.g. CD8 $\alpha$-chain) and/or additional therapeutic protein, e.g. the polypeptide comprising at least one epitope of an autoantigen, the expression vectors must be delivered into a cell. This delivery may be accomplished *in vitro,* as in laboratory procedures for transforming cells lines, or *in vivo* or *ex vivo,* as in the treatment of certain disease states. As described above, one preferred mechanism for delivery is via infection where the nucleic acid is encapsulated in a recombinant viral particle. Delivery may be accomplished by systemic administration of the vector to the patient such as by injection or intravenous administration. Systemic administration may result in co-expression of the immunomodulatory molecule and polypeptide comprising the epitope in any cell. That is expression is not targeted to a particular cell type.

Once the expression vector has been delivered into the host cell the nucleic acid encoding the desired oligonucleotide or polynucleotide sequences may be positioned and expressed at different sites. The nucleic acid encoding the construct may be stably integrated into the genome of the cell. This integration may be in the specific location and orientation via homologous recombination (gene replacement) or it may be integrated in a random, non-specific location (gene augmentation). The nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression vector employed.

In certain embodiments of the invention, the expression vector may simply consist of naked recombinant DNA or plasmids. Transfer of the vector may be performed by any of the methods mentioned above which physically or chemically permeabilize the cell membrane. This is particularly applicable for transfer *in vitro* but it may be applied to *in vivo* use as well. Dubensky et al. (1984) successfully injected polyomavirus DNA in the form of calcium phosphate precipitates into liver and spleen of adult and newborn mice demonstrating active viral replication and acute infection. Benvenisty and Reshef (1986) also demonstrated that direct intraperitoneal injection of calcium phosphate-precipitated plasmids results in expression of the transfected genes. It is envisioned that DNA encoding a gene of interest may also be transferred in a similar manner *in vivo* and express the gene product.

Another embodiment of the invention for transferring a naked DNA expression construct into cells may involve particle bombardment. This method depends on the ability to accelerate DNA-coated microprojectiles to a high velocity allowing them to pierce cell membranes and enter cells without killing them (Klein et al., 1987). Several devices for accelerating small particles have been developed. One such device relies on a high voltage discharge to generate an electrical current, which in turn provides the motive force (Yang et al., 1990). The microprojectiles used have generally consisted of biologically inert substances such as tungsten or gold beads.

Selected organs including the liver, skin, and muscle tissue of rats and mice have been bombarded *in vivo* (Yang et al., 1990; Zelenin et al., 1991). This may require surgical exposure of the tissue or cells, to eliminate any intervening tissue between the gun and the target organ, i.e. *ex vivo* treatment. Again, DNA encoding a particular gene may be delivered via this method and still be incorporated by the present invention.

In one embodiment of the present invention, the nucleic acid molecule is introduced into target cells, by liposome-mediated nucleic acid transfer. In this regard, many liposomebased reagents are well known in the art, are commercially available and may be routinely employed for introducing a nucleic acid molecule into cells of the target. Certain embodiments of the present invention will employ cationic lipid transfer vehicles such as Lipofectamine or Lipofectin (Life Technologies), dioleoylphosphatidylethanolamine (DOPE) together with a cationic cholesterol derivative (DC cholesterol), N[1-(2,3-dioleyloxy)propyl]N,N,N-trimethylammonium chloride (DOTMA) (Sioud et al., J. Mol. Biol. 242:831-835 (199)), DOSPA:DOPE, DOTAP, DMRIE:cholesterol, DDAB:DOPE, and the like. Production of liposome-encapsulated nucleic acid is well known in the art and typically involves the combination of lipid and nucleic acid in a ratio of about 1:1.

*Uses of the Present Invention*

As detailed above, the methods and compositions described and enabled herein find general utility in inhibiting a cellular and/or humoral autoimmune response to autoantigens. According to the present invention, survival of cells expressing the autoantigen can be extended without the need for chronic general immunosuppressive agents by conditioning host cells to express a CD8 polypeptide, particularly the CD8 α-chain and a polypeptide comprising at least one epitope of an autoantigen. Expression of the CD8 polypeptide and auto-antigen epitope as described herein results in effective and specific inhibition of the auto-immune response directed to auto-antigens.

Without being bound by theory, it is thought that expression of CD8 on cells confers on the cells the ability to induce the "veto effect" on a host immune system. That is, as described above, when cells expressing CD8 are contacted with host T cells, the T cells are downregulated or killed. Accordingly, by "veto" or "veto effect" is meant the ability of a target cell to downregulate the immune response against the target cell or antigens expressed on the target cell. It is thought that CD8 is necessary for induction or transfer of the veto effect, and in particular, the CD8 α-chain. By "transfer of the veto effect" is meant that the veto effect is transferred to a cell that normally would not induce the veto effect. That is, the ability to reduce or downregulate the immune response to an antigen on a target cell is conferred upon the target cell by induced or increased expression of CD8. As reported for the first time herein, it has now been surprisingly discovered that the presence of CD8 α-chain on target cells in conjunction with induced autoantigen expression or presentation can "veto" the activity of auto reactive CD4+ T-cells as well as CD8+ cells, and thus both the cellular and humoral components of the immune response may be inhibited thereby.

Accordingly, the invention finds use in reducing the immune response to target cells by inducing the veto effect. This results in the down regulation and deletion of T cells that would otherwise recognize the target cell. When the target cell is a cell expressing an autoimmune antigen, inducing the veto effect protects against a host autoimmune response.

Generally, when expression of CD8 is used in an autoimmune scenario, the life of cells expressing the autoantigen will be extended for a significant amount of time beyond what could normally be anticipated in the absence of the subject nucleic acids, more usually at least five days, more preferably at least about 30 days, and even more preferably about 3 months and most preferably about 6 months to one year. The actual amount of time the life of autoimmune antigen expressing cells is extended will vary with the various conditions of the procedure, particularly depending on the cells expressing the autoantigen. Also, treatment with the delivery vehicle containing the CD8 nucleic acid can be repeated if CD8 expression declines such that the target cell is recognized by the immune response.

*In vivo* delivery includes, but is not limited to direct injection into muscle, an organ, via catheter, or by other means of perfusion. The nucleic acid may be administered intravascularly or administered systemically. One of ordinary skill in the art will recognize the advantages and disadvantages of each mode of delivery. For instance, direct injection may produce the greatest titer of nucleic acid in the patient, but distribution of the nucleic acid will likely be uneven throughout the body. Systemic administration is a preferred method, as is intramuscular injection. The nucleic acids may be introduced in a single administration, or several administrations. The skilled artisan will be able to determine a satisfactory means of delivery and delivery regimen without undue experimentation.

The subject nucleic acids may be used with a wide variety of hosts, particularly primates, more particularly humans, or with domestic animals.

Other applications of the method and compositions of the present invention will be apparent to those skilled in the art.

*Formulations and Dosing of Expression Vectors*

One skilled in the art will appreciate that many suitable methods of administering an expression vector (particularly an adenoviral vector) to an animal (see, for example, Rosenfeld et al., Science, 252, 431-434 (1991); Jaffe et al., Clin. Res., 39(2), 302A (1991); Rosenfeld et al., Clin. Res., 39(2), 311A (1991); Berkner, BioTechniques, 6, 616-629 (1988)) are available, and, although more than one route can be used for administration, a particular route can provide a more immediate and more effective reaction than another route. Pharmaceutically acceptable excipients for use in administering the expression vector and/or means of inhibiting an immune response also are well-known to those who are skilled in the art, and are readily available. The choice of excipient will be determined in part by the particular method used to administer the expression vector and for means of inhibiting an immune response. There are a wide variety of suitable formulations for use in the context of the compositions of the present invention. In particular, the present invention provides a therapeutic composition for inhibiting an autoreactive T cell response against a target cell comprising an expression vector encoding a CD8 polypeptide comprising all or a functional portion of a CD8 $\alpha$-chain, wherein said CD8 $\alpha$-chain includes a transmembrane domain for associating said CD8 $\alpha$-chain on a surface of said target cell, and at least one epitope of an autoantigen associated with said autoreactive T cell response, wherein said expression of the CD8 polypeptide is separate from said, expression of the autoantigen. In alternative embodiments, the expression vector further encodes a further therapeutic molecule or protein of interest such as, e.g., an antiinflammatory molecule. Such compositions can further comprise other active agents, such as therapeutic or prophylactic agents and/or immunosuppressive agents as are known in the art. The following methods and excipients are merely exemplary and are in no way limiting.

Formulations suitable for oral administration can consist of (a) liquid solutions, such as an effective amount of the compound dissolved in diluents, such as water, saline, or orange juice; (b) capsules, sachets or tablets, each containing a predetermined amount of the active ingredient, as solids or granules; (c) suspensions in an appropriate liquid; and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, emulsions, gels, and the like containing, in addition to the active ingredient, such excipients as are known in the art.

Aerosol formulations can be made for administration via inhalation. These aerosol formulations can be placed into pressurized acceptable propellants, such as dichlorodifluoromethane, propane, nitrogen, and the like. They also can be formulated as pharmaceuticals for non-pressurized preparations, such as in a nebulizer or an atomizer.

Formulations suitable for parenteral administration include aqueous and nonaqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Additionally, suppositories can be made with the use of a variety of bases, such as emulsifying bases or water-soluble bases. Formulations suitable for vaginal administration can be presented as pessaries, tampons, creams, gels, pastes, foams, or spray formulas containing, in addition to the active ingredient, such carriers as are known in the art to be appropriate.

The dose administered to an animal, particularly a human, in the context of the present invention will vary with the therapeutic transgene of interest, source of vector and/or the nature of the immunomodulatory molecule, the composition employed, the method of administration, and the particular site and organism being treated. However, preferably, a dose corresponding to an effective amount of a vector (e.g., an adenoviral vector according to the invention) is employed. An "effective amount" is one that is sufficient to produce the desired effect in a host, which can be monitored using several end-points known to those skilled in the art. For instance, one desired effect is nucleic acid transfer to a host cell. Such transfer can be monitored by a variety of means, including, but not limited to, a therapeutic effect (e.g., alleviation of some symptom associated with the disease, condition, disorder or syndrome being treated), or by evidence of the transferred gene or coding sequence or its expression within the host (e.g., using the polymerase chain reaction, Northern or Southern hybridizations, or transcription assays to detect the nucleic acid in host cells, or using immunoblot analysis, antibody-mediated detection, or particularized assays to detect protein or polypeptide encoded by the transferred nucleic acid, or impacted in level or function due to such transfer). These methods described are by no means all-inclusive, and further methods to suit the specific application will be apparent to the ordinary skilled artisan. In this regard, it should be noted that the response of a host to the introduction of a vector, such as a viral vector, in particular an adenoviral vector, as well as a vector encoding a means of inhibiting an immune response, can vary depending on the dose of virus administered, the site of delivery, and the genetic makeup of the vector as well as the transgene and the means of inhibiting an immune response.

Generally, to ensure effective transfer of the vectors it is preferable that about 1 to about 5,000 copies of the vector be employed per cell to be contacted, based on an approximate number of cells to be contacted in view of the given route of administration, and it is even more preferable that about 3 to about 300 pfu enter each cell. However, this is merely a general guideline, which by no means precludes use of a higher or lower amount, as might be warranted in a particular application, either *in vitro* or *in vivo.* Similarly, the amount of a means of inhibiting an immune response, if in the form of a composition comprising a protein, should be sufficient to inhibit an immune response to the recombinant vector comprising the transgene. For example, the actual dose and schedule can vary depending on whether the composition is administered in combination with other pharmaceutical compositions, or depending on interindividual differences in pharmacokinetics, drug disposition, and metabolism. Similarly, amounts can vary in *in vitro* applications, depending on the particular cell type targeted or the means by which the vector is transferred. One skilled in the art easily can make any necessary adjustments in accordance with the necessities of the particular situation.

## Example 1: Studies with Veto Vectors

### a. Use of Plasmid Expression Vectors to Engineer Fibroblasts as Veto Cells

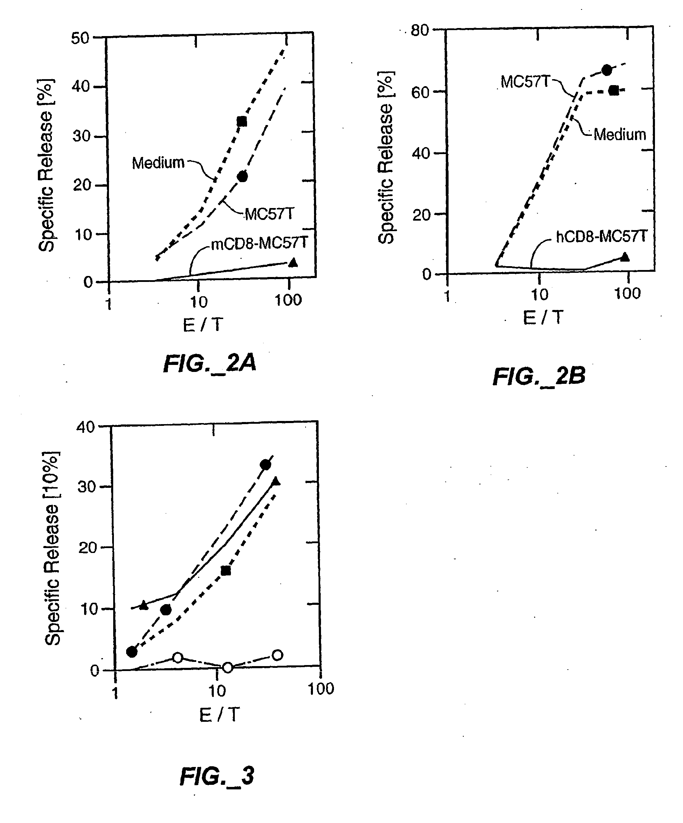

Fibroblasts were engineered to express either human or mouse CD8 $\alpha$-chain on their surface. Fibroblasts were transfected with the pCMVhCD8$\alpha$ plasmid or pCMVmCD8$\alpha$ plasmid in which expression of the CD8 $\alpha$-chain is driven by the CMV immediate early promotor/enhancer (Invitrogen). When the CD8 $\alpha$-chain transfected fibroblasts (H-2$^b$) were added to mixed lymphocyte cultures (Balb/c; H-2$^d$ anti-C57BL/6; H-2$^b$), only the CD8 $\alpha$-chain expressing line suppressed CTL responses. As depicted in Figures 2A and B, the addition of MC57T fibroblasts expressing either the mouse or human CD8 $\alpha$-chain completely suppressed the induction of CTLs. In contrast, the addition of non-transfected fibroblasts did not affect T-lymphocyte activation. In addition to establishing the inhibitory function of a CD8 $\alpha$-chain, these experiments also demonstrated that mouse T-lymphocytes could be veto-ed with the human CD8 $\alpha$-chain. Therefore, the mouse model will be useful in examining veto designed for clinical use.

### *In vivo* Function of Engineered Veto Cells

It was determined whether engineered veto functioned in the animal. C57BL/6 (H- 2$^b$)-derived fibroblasts transfected to express the CD8 $\alpha$-chain were injected into Balb/c (H-2$^d$) mice. Control animals were injected with non-transfected fibroblasts. Spleen cells were harvested after 8 to 40 days and introduced into MLCs cultures with C57BL/6 (H- 2$^b$) spleen cells as stimulator cells. After 5 days, cultures were harvested and tested for their ability to lyse EL4 (C57BL/6, H- 2$^b$) target cells. Induction of anti-H- 2$^b$ CTL responses was completely suppressed in animals that had been injected with CD8 $\alpha$-chain expressing fibroblasts (Figure 3). Inhibition of anti-H- 2$^b$ T cells was highly specific. T cells from these mice still mounted responses to third party H-2$^k$ allo-MHC molecules. These experiments confirmed that engineered veto cells specifically suppressed immune responses *in vivo* similar to conventional veto cells and that non-classical veto cells could be engineered to become veto cells. In other words, engineered cells negatively immunized animals to antigens carried on these cells.

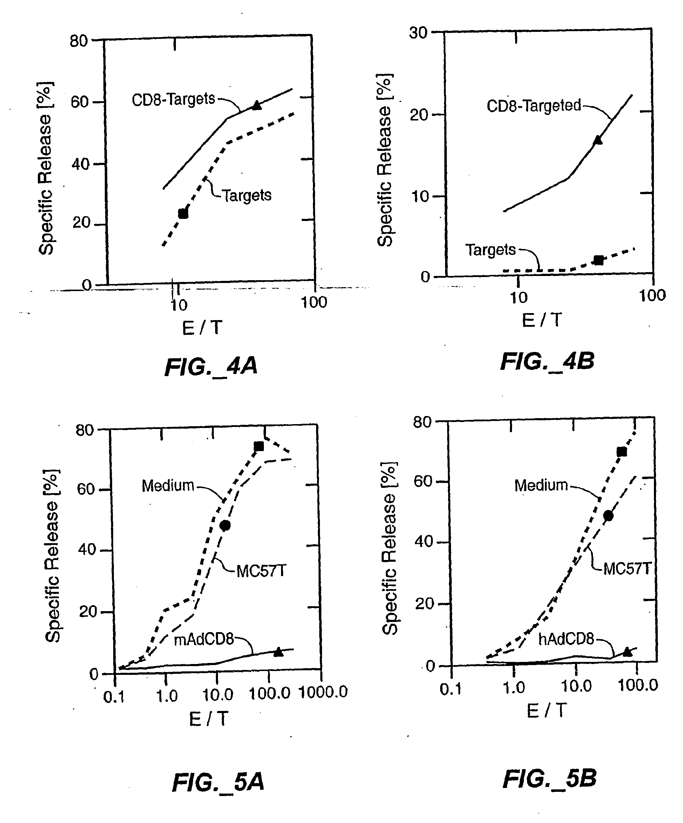

It was tested whether expression of the CD8 $\alpha$-chain interfered with the function of fully activated T cells. For this purpose, target cells expressing CD8 $\alpha$-chains were tested for their susceptibility to lysis by fully activated CTLs. Two different T cell populations were chosen for these studies, allo-reactive CTLs stimulated in a MLCs and activated peptide-specific CTLs. As depicted in Figures 4A-B, targets expressing the CD8 $\alpha$-chain were lysed efficiently by populations of alloreactive T cells, but not by antigen-specific T cells. These results suggested that engineered veto was able to interfere even with on-going antigen specific immune responses, such as those found in autoimmune responses.

### b. Viral Transfer Vectors to Engineer Fibroblasts as Veto Cells

**Veto function of the Adenoviral Transfer Vector m-CD8:** A replication-deficient vector Adenoviral Transfer Vector (mAdCD8$\alpha$) was developed that carried the mouse CD8 $\alpha$-chain. Mouse fibroblasts (MC57) that had been infected with the mAdCDB veto transfer vector expressed high levels of the mouse CD8 $\alpha$-chain on day 2. In these fast proliferating cells, expression of the mouse CD8 $\alpha$-chain is significantly reduced by day 5. mAdCD8 also infected other mouse cell lines, such as EL4, albeit with lower efficiency (data not shown).

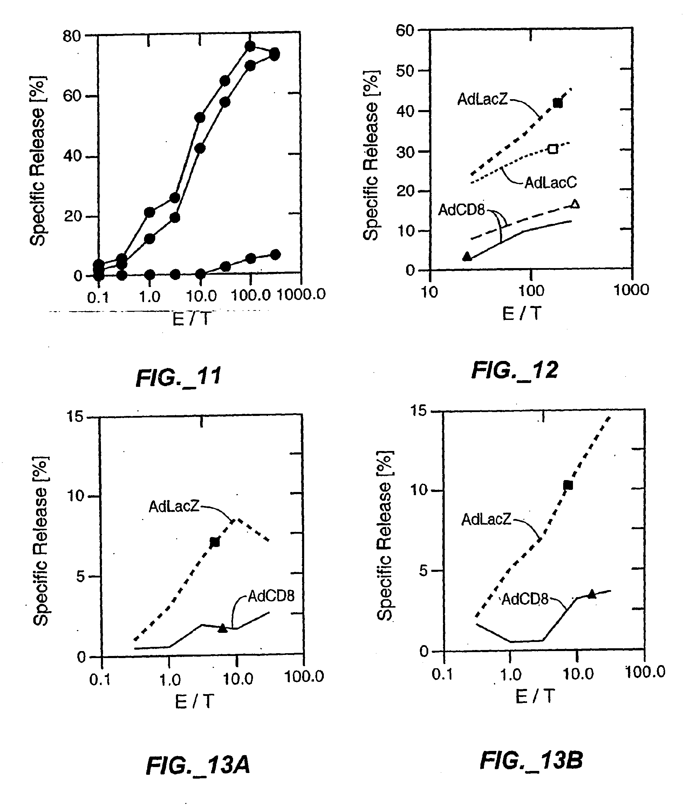

In subsequent experiments, mAdCD8 $\alpha$-infected MC57 fibroblasts (H-2$^b$) were added to Balb/C (H-2$^d$) anti-C57B1/6 (H-2$^b$) MLCs. After 5 days, the cultures were harvested and tested for the presence of anti-H-2$^b$ CTLs. MLCs to which infected fibroblasts had been added, no longer contained anti- H-2$^b$ CTLs (Figure 11). These experiments established the ability of a veto transfer vector to mediate immune suppression.

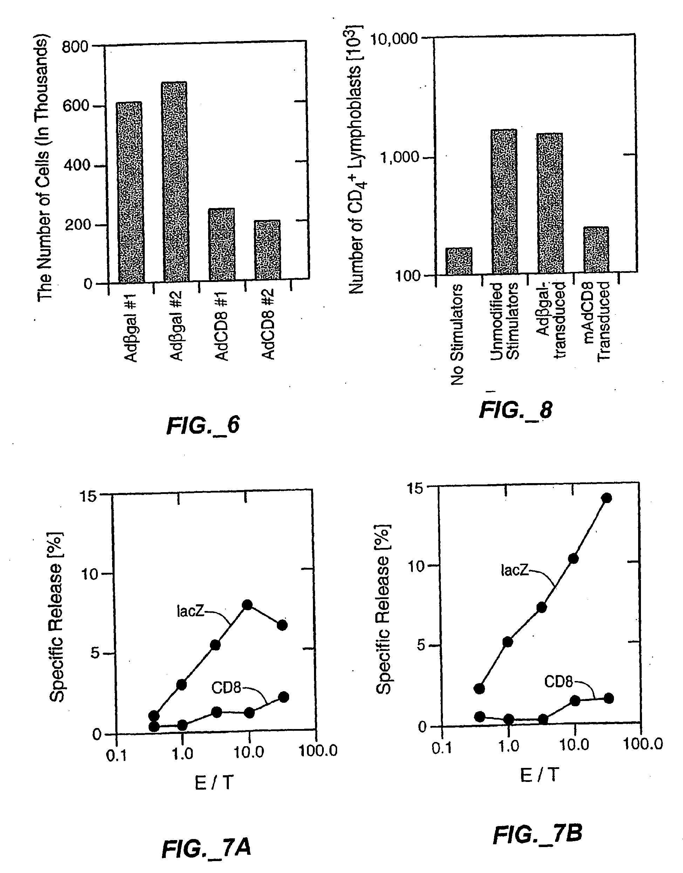

In addition, the human CD8-version of the Adenoviral vectors have been produced. Also, Adenoviral Associated Viruses that expressed mouse CD8 α-chain have been produced. It has been demonstrated that these viruses induce expression of the respective CD8 chains. Adenoviral vectors expressing either the mouse or the human CD8 α-chain mediated the complete inhibition of the induction of killer T cells (see Figure 6).

**Negative immunization with the mAdCD8 Veto Transfer Vector:** Two different experiments were set up to determine whether mAdCD8 suppressed immune responses *in vivo.* In the first experiment, C57Bl/6 mice were infected with equivalent doses of either the mAdCD8 veto transfer vector or a similar adenoviral control vector coding for ß-galactosidase, instead of the mouse CD8 α-chain (Adβgal). Seven days after immunization, these animals were sacrificed. Single cell suspensions of their spleen cells were cultured in the presence of Adβgal viruses for 5 days. Then the cultures were harvested and their ability to proliferate was evaluated. As depicted in Figure 6, T cells proliferated vigorously to Adβgal harvested from mice immunized with Adβgal indicative of the presence of the highly proliferative CD4+ T cells. In contrast, T cells harvested from mAdCD8-injected animals failed to expand.

In a second step, we tested whether these cultures contained functional CD8+ CTLs testing them for their ability to lyse Adβgal-infected target cells (EL4, H-2^b). CTLs could only be revealed in cultures established from mice injected with Adβgal (Figure 8). This first experiment suggested that AdCD8α did not induce responses to the adenoviral antigens possibly due to the expression of the CD8 α-chain. However, it was possible that AdCD8 failed to induce immune responses for different reasons. AdCD8 was non-functional in some undefined way, or the mice could only react with the ß-galactosidase protein not found in mAdCD8.

To test the validity of the different conclusions, C57Bl/6 mice were injected once with either mAdCD8 or Adβgal followed by a second infusion with Adβgal after 7 days. Seven days later, mice were sacrificed, and 5-day spleen cell cultures were established in the presence of Adβgal. The responding T cells were tested for their lytic ability towards Adβgal-infected target cells (Figures 7A-B). Indeed, two exposures to Adβgal led to improved immunization. These studies also showed that after an AdCD8 injection, mice no longer responded to Adβgal and that Adβgal primarily, if not exclusively induced CTL responses towards the adenoviral proteins common to both vectors. This set of experiments strongly suggests that it will be possible to produce a gene therapy viral vector able to negatively immunize against responses towards genes carried on these vectors.

**Inhibition of CD4+ T lymphocytes by veto**: To examine whether veto transfer vectors can be used to inhibit the induction of CD4+ T lymphocytes, the following experimental system was established. C57Bl/6-derived fibroblast stimulator were transformed to express an allogeneic MHC class II molecule (H-2E^k) and the immune stimulatory CD80. These slow-proliferating fibroblasts non-irradiated to preserve their full stimulatory capacity, were transduced with either the mAdCD8 or the Adβgal transfer vectors and added to unselected C57B1/6 spleen cells. After 4 days, these cultures were harvested and analyzed by surface immunofluorescence for the presence of activated, i.e. blasting, CD4+ T lymphocytes (Figure 8). It was found that unselected C57B1/6 spleen cells cultured with normal or Adβgal-transduced stimulator cells had high numbers of CD4+ T lymphoblasts. In contrast, cultures to which mAdCD8-infected stimulators had been added, only few CD4+T lymphoblasts were detected. These studies confirmed that veto inhibited CD4+ T lymphocytes and in addition that a viral veto transfer vector could be used for this purpose.

Surface Expression of the mouse and human CD8 α-chains after infection with the different virus constructs

Staining Protocols:

mAdCD8:

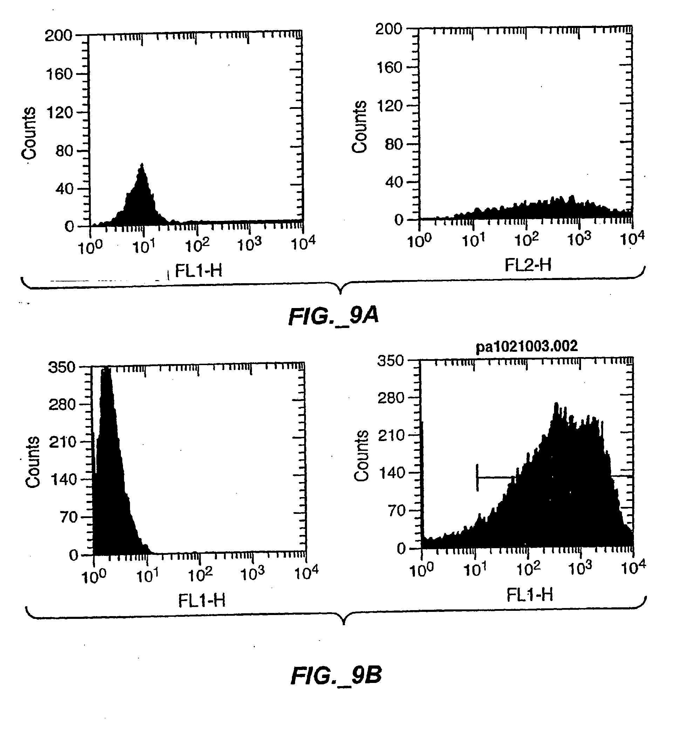

MC57T were mock-infected or infected with mAdCD8 at a multiplicity of infection of approximately $10^4$ for 3 days in modified IMDM. The infected cells were harvested and stained for the surface expression of the CD8 α-chain with the anti-mouse CD8 α-chain antibody directly labeled with FITC (Pharmingen). The extent of surface fluorescence was measured on a fluorescent activated cell analyzer (FACScan, Beckton-Dickinson) (Figure 9B).

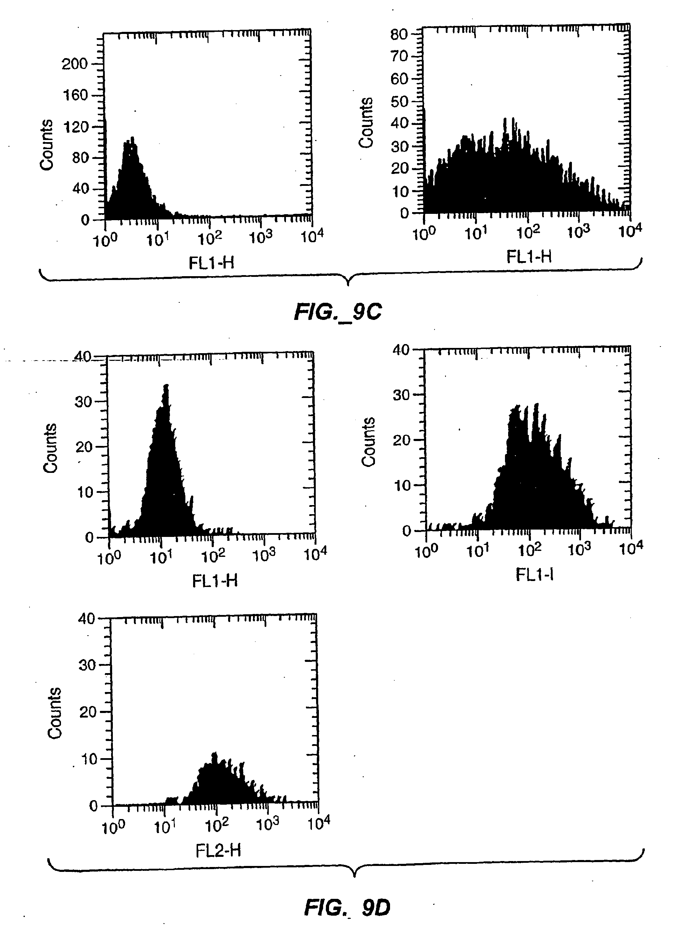

Bone marrow cells were harvested from the cavity of femoral bones of Balb/c mice. The cells were infected with a ß-galactosidase expressing Adenoviral control vector (AdLacZ) or with mAdCD8 at a multiplicity of infection of $10^4$ for 3 days cultures in modified IMDM. The infected cells were harvested and stained for the surface expression of the CD8 α-chain with the anti-mouse CD8 α-chain antibody directly labeled with FITC. The extent of surface fluorescence was measured (Figure 9C). In addition, it was determined that several cell types including CD34+ bone marrow cells, i.e. cells within the stem cell pool, were transduced efficiently (Table 5).

**TABLE 5**

| Marker | Cell Type | Positive | Staining |
|--------|-----------|----------|----------|
| CD11a | Leukocytes | 29.3% | 31.5% |
| CD34 | Hematopoietic Lineages | 13.8% | 10.5% |
| CD19 | B Lymphocytes | 0.6% | 7.7% |
| CD3 | T Lymphocytes | 0.6% | nd |

hAdCD8:

MC57T were mock-infected. The viral titer of the hAdCD8 is not known. 100$\mu$l of its stock solution was used to infect $3 \times 10^5$ cells for 3 days. The infected cells were harvested and stained for the surface expression of the CD8 $\alpha$-chain with the anti-human CD8 $\alpha$-chain antibody directly labeled with FITC (Pharmingen). The extent of surface fluorescence was measured on a fluorescent activated cell analyzer (Figure 9A).

**AAV-Based Vectors:** AAV-based vectors were produced in parallel using a Strategene/Avigen system. In these constructs, the human and mouse CD8 $\alpha$-chains were driven from the same CMV intermediate early promotor/enhancer. The two viruses, mAAVCD8 and hAAVCD8 were packaged in the HEK 293 packaging cell line. The system employed is free of helper virus. mAAVCD8 and hAAVCD8 efficiently infected mouse fibroblasts (MC57T) and drove high levels of expression of the mouse or human CD8 $\alpha$-chains, respectively. The extent of fluorescence was measured on a fluorescent activated cell analyzer (Figure 9D). It is interesting to note that high levels of CD8 $\alpha$-chain expression was seen within 36 hours after transduction. This finding was in contrast to observation by others. They had found that AAV-driven gene expression took several days to reach significant levels (PH Schmelck, PrimeBiotech). Additional studies with AAV vectors reiterated our previous findings that they could be used to suppress immune responses. Here, the standard MLC protocol was used (Figures 5A-B).

**Example 2: _In vitro_ Inhibition Studies - Mixed Lymphocyte Cultures**

Spleen cells were harvested from Balb/c (H-2$^d$) and C57BU6 (H-2$^b$) mice. Single cell suspensions were prepared. The C57BU6 spleen cells were irradiated with 3,000 rad (Mark 1 Cesium Irradiator). $4 \times 10^6$ Balb/c spleen cells (responder/effector cells) were cultured together with $4 \times 10^6$ irradiated C57BU6 spleen cells (stimulator cells) per well in 24-well plates (TPP, Midwest Scientific, Inc.) in IMDM (Sigma) that contained 10% fetal calf serum (FCS) (Sigma), HEPES, penicillin G, streptomycin sulfate, gentamycine sulfate, L-glutamine, 2-mercaptoethanol, non-essential amino acids (Sigma), sodium pyruvate and sodium bicarbonate (modified IMDM). After 5 days of culture in a $CO_2$ incubator (Forma Scientific), the cultures were harvested in their entirety and tested for the ability to lyse C57BU6-derived target cells (H-2$^b$). To some of these cultures $4 \times 10^5$ MC57T fibroblasts (H-2$^d$) were added that had been irradiated with 12,000 rad. In inhibition cultures, $4 \times 10^5$ MC57T cells were included that had been infected with mAdCD8 at a multiplicity of infection of approximately $10^4$ to 1 for 2 days.

**Cytotoxic T Lymphocyte Killer Assays**: Cells harvested from the mixed lymphocyte cultures were counted for the number of blast.cells, as an indicator of activated T lymphocytes. These effector cells were added to a single well in a U-bottomed 96-well plate. The number of effectors per well was titrated in 3-fold titration steps starting from $3 \times 10^6$ or $1 \times 10^5$ effectors per well. To these effector cells $1 \times 10^4$ target cells EL4 (H-2$^b$), MC57T (H-2$^b$) or P815 (H-2$^d$) per well were added. The target cells had previously been labeled with $^{51}$Cr (Na-Chromate, Perkin-Elmer). $1 \times 10^6$ target cells had been incubated with 100 $\mu$Ci in a modified IMDM in a volume of approximately 500$\mu$l for 90 min. Thereafter, the non-incorporated $^{51}$Cr was removed by multiple washes with modified IMDM.

The effector and target cells were incubated in a total volume of 200 $\mu$l for 4 hrs in a $CO_2$ incubator. Thereafter, the plates were spun in centrifuge (Centra CJ35R, International Equipment Company) at 1,500 rpm for 3 min. 100 ml of medium was removed from each well and the amount of $^{51}$Cr released from the target cells was counted in a Model 4000 Gamma counter (Beckman Instruments). Control cultures were set, in which effector cells were omitted to determine the background release. Total $^{51}$Cr incorporation into target cells was determined in wells, in which a 1% solution (w/v) of Triton X100 (Sigma) was substituted for the effector cells.

The amount of specific lysis was determined as:

in % = (specific release - background release) / (total release - background release) x 100

**Activity of mAdCD8 in vitro:** Mixed lymphocyte cultures were set up (Balb/c antiC57BU6). To these cultures MC57T fibroblasts were added (as indicated) that had been irradiated with 12,000 rad and had been infected with mAdCD8. After 5 days of culture, the cultures were harvested and tested for their ability to lyse EL4 (H-2$^b$) target cells at different effector-to-target (E/T) ratios (see Figure 3). As can be seen, even in the mixed lymphocyte culture, the cells expressing CD8 inhibited the induction of lytic T lymphocytes.

**Production of mAdCD8 and hAdCD8:** Both Adenoviral vectors were produced with the help of the AdEasy™ system from Biogene. Here the mouse and human CD8 α-chain cDNA is incorporated into the Transfer Vector (Step 1). Recombination with the Ad5ΔE1/ΔE3 vector is achieved in BJ5183 EC bacteria (Step 2). The recombinant vector is then transferred into the QBI-HEK 293A cells that contain the E1A and E1B Adenovirus 5 viral genes, which complement the deletion of this essential region in the recombinant adenovirus. The hAdCD8 and mAdCD8 produced in these cells are thus replication deficient.

As control vector expressing the bacterial LacZ gene (ß-galactosidase) the Qbiogene provided QBI-Infect+ Viral Particle (Ad5.CMVLacZΔE1/ΔE3). Mouse CD8 α-chain sequence used. This sequence is similar to the published mouse sequence:

ACTUAL SEQUENCE: MASPLTRFLS **LNLLLMGESI** ILGSGEAKPQAPELRIFPKK

MDAELGQKVD LVCEVLGSVS QGCSWLFQNS SSKLPQPTFVVYMASSHNKI

TWDEKLNSSK LFSAVRDTNN KYVLTLNKFS KENEGYYFCSVISNSVMYFS

SVVPVLQKVN STTTKPVLRT PSPVHPTGTS QPQRPEDCRPRGSVKGTGLD

FACDIYIWAP LAGICVAPLL SLIITLICYH RSRKRVCKCPRPLVRQEGKP RPSEKIV

Human CD8 α-chain sequence used. This sequence has a silent mutation compared to the published human sequence as indicated.

ACTUAL SEQUENCE: MALPVTALLL PLALLLHAAR

PSQFRVSPLDRTWNLGWTVE LKCQVLLSNP TSGCSWLFQP RGAAASPTFL

LYLSQNKPKAAEGLDTQRFS GKRLGDTFVL TLSDFRRENE GYYFCSALSN

SIMYFSHFVPVFLPAKPTTT PAPRPPTPAP TIASQPLSLR PEACRPAAGG

AGNRRRVCKCPRPVVKSGDK PSLARYV

**Production of pAAV-mCD8 and pAAV-hCD8:** These vectors were produced with the help of the AAV Helper-Free System from Stratagene. The system works by inserting the mouse and human sequences into the pAAV-MCS cloning vector. This plasmid is then co-transfected into HEK 293 cells together with a helper plasmid (containing the necessary Adenoviral proteins) and the pAAV-RC vector (containing the capsid genes) to produce the recombinant AAV particles.

## Example 3: Engineered Veto in Animal Models

We investigated how animals responded to the injection of large doses of the mAdCD8. In the first set of experiments, Balb/c mice (two mice in each group) were injected i.v. with equivalent doses of mAdCD8 or an Adenoviral control vector coding for ß-galactosidase (AdLacZ). After seven days the animals were sacrificed. Their spleen cells were cultured in the presence of AdLacZ for five days. They were then tested for their ability to lyse AdLacZ-infected target cells (P815, Balb/c-derived). As depicted in Figure 13A, CTLs with specific lytic ability could be expanded from Balb/c mice that had been immunized with AdLacZ, but not from mice that had received the mAdCD8. This result suggested that AdCD8 did not induce immune responses to Adenoviral antigens due to the expression of the CD8 α-chain.

In a second set-up, C57Bl/6 mice were immunized with equivalent doses of mAdCD8 (2 mice) or AdLacZ (2 mice). Seven days after immunization, one animal of each group was sacrificed. Their spleen cells were cultured in cell suspension in the presence of AdLacZ for five days. They were then tested for their ability to specifically lyse AdLacZ-infected target cells (EL-4, C57Bl/6-derived). Again, injection of AdLacZ had induced the development of specific killer cells albeit at a low frequency, whereas mAdCD8 had failed to do so (Figure 13A).

In the second phase of this experiment, the remaining C57BU6 mice that had received either mAdCD8 or AdLacZ received a second dose of AdLacZ seven days after their first viral injection. Seven days later, mice were sacrificed, and five-day spleen cell cultures were established in the presence of AdLacZ. The responding T cells were again tested for their lytic ability towards AdLacZ-infected EL4-target cells (Figure 13B). Indeed, two exposures to AdLacZ led to a somewhat improved immunization. However, the animal that had previously received mAdCD8 still failed to mount a response. These experiments suggest that AdCD8 not only failed to induce immune responses, but prevented the induction of immune responses directed against itself. Thus, mAdCD8 evaded the immune system.

## Example 4: Production of Vectors

**Production of Adenoviral Vector**: The full-length mouse CD8 $\alpha$-chain cDNA is linked to the ovalbumin cDNA by a short IRES taken from the Hepatitis C virus. This bi-cistronic construct produced by PCR is moved into the Adenoviral Transfer plasmid, in which it is placed behind the non-specific CMV immediate early promotor/enhancer. A poly-adenylation is also provided in this vector. This plasmid will is in homologous bacterial recombination together with the plasmid (pAdEasy-1) containing the DE1-DE2 Adenovirus 5 genome, to generate a new plasmid, in which the expression cassette is inserted into the original E1 region of the Adenovirus genome. This resulting vector plasmid is then transfected into QBI-293A cells to generate the recombinant Adenoviral vector, Ad/CMV/CD8/Ova, expressing the CD8 $\alpha$-chain and ovalbumin under the control of the CMV promotor. Control vector only expressing ovalbumin is produced using the same system. A second set of Adenoviral vectors is produced, in which the CMV promotor region is exchanged for a mouse MHC class II promotor that has been found to direct gene expression to all cells naturally expressing MHC class II (Ceman, J Immunol. 1992 Aug 1;149(3):754-61; Martin, J Immunogenet. 1990 Feb-Apr;17(1-2):151-9). The Adenoviral vector particles are produced in QBI-293A cells by standard means and purified by gradient purification. The number of infectious particles in each preparation is determined in a standard plaque assay.

**Production of AAV-based Vector:** The bi-cistronic expression cassettes described above is packaged into AAV type 2 capsids using the helper virus-free Strategene AAV-vector system. In this vector system, the gene of interest, in our case the bi-cistronic construct, is moved into the pCMV-MCS cloning vector via a multiple cloning site. After cloning, the expression cassette is moved to pAAV-LacZ replication-deficient AAV vector. As a consequence the LacZ gene is removed. The recombinant vector is co-transfected into a viral packaging cell line 293 for production of recombinant, replication-deficient AAV virions together with the pAAV-RC vector that harbors the cap and rep genes and the pHelper vector that carries the adenovirus genes (E2A, E4 and VA RNAs) required for inducing the lytic phase of AAV. Again the virions are purified using standard purification protocols. In addition, ovalbumin only vectors are produced.

**Helper Virus-Free Production of Gutless Adenoviruses**: To package gutless Adenoviruses, the necessary Adenoviral genes have to be provided in *trans.* Most published protocol use helper Adenovirus that has to be removed from the final therapeutic preparations. Cre-Lox recombination systems have been used to limit the packaging of additional helper viruses (Hardy, 1996; Sakhuja, 2003). As an alternative to infectious Adenoviral helper viruses, a system has been described, in which the helper genome was delivered by an Adenovirus/Baculovirus hybrid genome construct (Cheshenko, 2001). This hybrid genome construct avoided the use of Adenoviral helper viruses. It however allowed the recombination of replication-competent Adenoviruses via a homologous recombination between ΔE1 fragments in the hybrid genome and the E1 in the 293A packaging cells. The helper genome used had a relatively short deletion within the E1 gene.

The strategy to prevent recombinants is to eliminate sequence overlap between E1 sequences present in the genome of the packaging cell and in the helper genome (Nichols, 2002). A potential hurdle to achieve this is the way the E1B and pIX genes are regulated. They use the same poly-adenylation site. Furthermore, the pIX gene has to be carried in the helper genome to allow its expression. Therefore, a new Adenovirus/Baculovirus hybrid genome will be constructed, in which E1A and E1B, but not the pIX expression cassette will be deleted. The packaging cell will express E1A and E1B, but will be devoid of pIX. The E1 region will be deleted between position 460 and 3509. This will lead to a truncation of the E1 b, but will leave pIX intact. Otherwise the Adenovirus/Baculovirus hybrid genome will be constructed as described before (Cheshenko, 2001). It will be designed to carry a functional genome of and Adenovirus type 5 deleted of the packaging signals and E1 and E3. It will also contain fused ITRs acting as an Adenoviral origin of replication. The Adenoviral genome will be flanked by *loxP* sites, allowing its excision with the help of the Cre recombinase. It was shown that pseudotyping of the baculovirus with the VSV glycoprotein provided a more efficient transduction of mammalian cells (Cheshenko, 2001). The vesicular stomatitis virus (VSV) glycoprotein envelope protein will be placed into the baculovirus genome of this construct. Driven by the baculovirus polyhydrin promotor it will provide selective expression in insect cells. The Adenovirus/Baculovirus hybrid genome will be rescued in insect cells as recombinant Baculovirus. Upon infection of Cre-expressing packaging cells (*see below*), the circular Adenovirus genome will be excised from the hybrid genome. Two circular molecules will be created, one containing the Adenovirus genome (ΔE1, ΔE3) and the other containing the baculovirus genome. The Adenovirus genome will provide all helper functions necessary for the propagation of the insert DNA construct, *i.e.* the expression cassette (*design see below*). Since the Adenovirus genome released from the hybrid through Cre-Lox recombination lacks the Adenovirus packaging signal, only the CD8 α-chain expression cassette will be packaged resulting in helper-free vector preparation.

Establishing of New Packaging Cell Lines: Human embryonic retinoblasts (HER) will be used. They can be immortalized by the Adenoviral E1 gene (Graham, 1977), and they are very efficient in producing Adenoviruses (Gallimore, 1986). To be used in the production of gutless Adenoviruses, HER cells will have to be modified in the following manner: (i) They will express Cre, so that the circular Adenovirus genome is excised from the hybrid molecule. Therefore, they will be transfected with a Cre-gene modified with the SV40 T-antigen nuclear localization signal (Lieber, 1996). Cre will be driven from an actin promotor. An immediate-early CMV promotor will not be used to avoid any sequence overlap with the viral expression cassette. (ii) The HER cells will have to provide the complimentary E1 genes missing from the Adenovirus/Baculovirus hybrid genome. The E1 promotor and the E1 poly-adenylation sequences will be obtained from non-viral sources, such as the human housekeeping gene phosphoglycerate kinase (PGK) promotor and the hepatitis virus poly-adenylation site (Valerio, 1985; Simonsen, 1983). (iii) The HER cells will be co-transfected with the Cre and the E1-expression cassettes. Immortalization of the Her cell will be taken as evidence for the integration of E1. Clones of continuously growing HER (CE-HER) cells will then be further analyzed for the presence and expression of the full-length Cre- and E1 constructs by PCR.

**Design of the CD8 α-Chain/Insulin Expression Cassette and the Non-Viral dmVTV**: The following expression cassette will be assembled. The full-length mouse CD8 α-chain cDNA will be linked to the (pre pro)insulin or (pro)insulin cDNA by a short IRES taken from the Hepaptitis C-virus. The immediate-early CMV promotor/enhancer employed in mAdCD8 will be used to drive the coordinate expression of both genes. This bi-cistronic construct will be placed into stuffer DNA sequences between two inverted copies of the left Adenoviral ITRs and a packaging signal. Restriction sites will be placed so that the expression cassette can be excised from the construction plasmid. Control vectors expressing only one of the two genes will be produced. The expression cassette devoid of the Adenoviral ITRs and the packaging signals will form the basis of the non-viral dmVTV. The non-viral dmVTV will be designed that antibiotic selection sequences and other non-essential DNA segments can be excised prior to use. The non-viral DNA construct will also be used to test the expression of the two different genes. For this purpose, different cell lines will be transfected with this expression cassette. The DNA integration and the levels of specific mRNA will be tested by PCR. The levels of surface expression of the CD8 α-chain will be determined by surface immunofluorescence, those of insulin released into the supernatant will be studied with the help of a previously established specific ELISA.

**Production of Viral and Non-Viral dmVTVs:** In the case of gutless Adenoviral dmVTVs, the CD-HER cells will be transfected with the enzymatically excised CD8/insulin/ITR construct and then transduced with the Adenovirus/Baculovirus helper genome. Crude lysates from the transduced CE-HER cells will be harvested and used to transduce 293 cells or tissue culture fibroblasts. The frequency of CD8-expressing cells will be used to determine the 'functional' titer of the dmVTVs. Determining the number of 293 plaques will provide us with a test for recombinant replication-efficient viruses. Adenoviral dmVTVs will be expanded in additional passages in CE-HER cells using the Adenovirus/Baculovirus helper genome. In addition to determining the dmVTV titers and the presence of recombinant viruses, the integrity of the dmVTV will be studied with the help of restriction enzyme mapping, PCR studies and DNA sequencing of dmVTV. Non-viral dmVTVs consisting of the bi-cistronic expression cassette will be used to transfect different cell lines, such fibroblasts. The selection marker will be provided by separate plasmids. CD8 α-chain and insulin expression will be studied.

## Example 5: Functional Testing of Vectors

**Transduction Efficiency of the Different Vectors**: Establish standard tissue culture cell lines of different phenotypes, such as fibroblasts, lymphomas, macrophages etc., freshly harvested and differentiated cells, such as macrophages and DCs, are tested. BM cells from C57Bl/6 are harvested and cultured in the presence of murine GM-CSF (harvested from transfected J558L cells, provided by U.D. Staerz). Cell cultures are washed repeatedly to remove non-adherent cells. After about 4 days, the adherent DCs pre-dominantly express an immature phenotype. The DCs are dislodged, re-plated and re-cultured. The resulting cells are analyzed phenotypically (Kamath, 2000; Bell, 1999; Liu, 2002; Mellman, 2001; Mellman, 1998). They are activated by the addition of LPS (10ng/ml, Sigma-Aldrich). Macrophages are harvested from the peritoneal cavity of C57Bl/6 mice either without or with previous proteose peptone (preparation provided by U.D. Staerz) i.p. injection. They are enriched by their ability to adhere to plastic and are stained (Leenen, 1994). Alternatively, macrophages are grown out of the BM in the presence of M-CSF. Macrophages are activated in culture by the addition of IFN-γ and LPS.

The different cell populations are infected with the different vectors at increasing multiplicities of infections (MOI) most likely starting at MOIs of 10-to-1 and reaching to levels of MOIs of $10^4$-to-1. The cell population are exposed to vectors from 30 minutes to 24 hours. The shorter exposure times are followed by an incubation period to allow expression of the transduced genes. The entire culture period will be 24 hours in these experiments. Transduced cells are studied for viability. They are stained for propidium iodide as a measure of overall DNA content and annexin-5 as an early measure of membrane orientation. The cells are then be analyzed on a fluorescence activate cell analyzer (FACSCalibur, Becton-Dickinson).

In parallel experiments, the expression levels of both the CD8 α-chain and the immunogen (ovalbumin) are determined. RNA is purified from these cells and the level of gene expression for the CD8 α-chain and the immunogen (ovalbumin) are determined by RT-PCR standardized to the levels of actin RNA. These studies are followed by the determination of protein levels. In the case of the CD8 α-chain, transduced cells are stained for surface expression of the mouse CD8 α-chain (FITC-linked anti- CD8 α-chain mAb 53-6-75). Interference of the staining with non-specific antibody binding is prevented by the use of F(ab)'$_2$ preparations of the antibody. The extent of mAb binding and thus CD8 α-chain expression is determined on a fluorescence activated cell analyzer.

The levels of ovalbumin production is determined in two ways. As ovalbumin is secreted by transfected cells, the amount of ovalbumin in the culture supernatant is determined by a sandwich ELISA with the help of a rat anti-ovalbumin mAb and purified rabbit anti-ovalbumin antibodies provided by U.D Staerz. In addition, it is determined whether ovalbumin can be presented in an immunologically relevant form on the MHC molecules on the surface of these cells. For this purpose, T lymphocytes are harvested from the T cell receptor transgenic mice, OT-I and D011.10, and are activated *in vitro.* They are then exposed to the different transduced cell populations expressing the appropriate MHC molecules. In standard activation experiments, it is tested to determine whether they are able to specifically lyse target cells (OTOI) or secrete IL-2 (DO11.10). Control experiments are performed with cells that have been transduced with vectors that solely carry the CD8 α-chain. Exogenous ovalbumin peptides are added. Here, we determine whether veto inhibits effector T cells. Anti-CD8 mAbs are added to inhibit target cell CD8. In addition, vectors that only carry ovalbumin are used.

*In vitro* **Inhibitory Function of Vectors:** Having optimized the transduction protocols, we determine whether the different vectors inhibit MHC class I- and MHC class II-restricted immune responses in a specific manner. From the previous experiments, we know which of the different vectors is the most promising and which cell line expresses the CD8 α-chain and efficiently presents ovalbumin. As a first study, the function of the CD8 α-chain is tested in MLCs, as described previously. In short, C57BU6 anti-Balb/c MLCs are established. They are provided with graded numbers of different transduced cell populations expressing the CD8 α-chain and cultured for five days. The development of allo-reactive CD8+ and CD4+ T cells are measured in subsequent CTL and re-stimulation assays.

Lines of insulin specific CD8+ and CD4+ T lymphocytes will be established from NOD mice possibly from harvested pancreatic islets. These lines will be maintained using established re-stimulation protocols. dmVTV transduced or transfected syngeneic cells will be added to killer assays (CD8+CTLs) and to cytokine release studies (CD4+ TH cells). Their ability to inhibit these responses will be examined. Different cell populations, including APCs, will be tested in this way. It will be studied whether veto-ed T cells are inhibited or killed in these cultured using overall DNA content and annexin-5 orientation (see *studies above*).

*In vivo* **Functional Testing of the Overall Veto Function of dmVTVs:** The overall veto function of the different dmVTVs will be tested with the help of allo-responses in an experimental set-up that previously demonstrated the *in vivo* activity of veto. C57BU6-derived cell lines transduced or stably transfected with the respective dmVTV will be injected into BALB/c animals. Ten days later the mice will be sacrificed, their spleen cells will be harvested, the resident T cells will be activated *in vitro* with C57BU6 stimulator cells. The presence of responding T cells will be examined in killer and cytokine-release assays. Since mice transgenic for T cell receptors specific for insulin are not available, the ability of dmVTVs to suppress insulin-specific immune responses will have to be assessed in studies in the NOD mouse (*see below*).

**Determine whether the different vectors can specifically inhibit the induction of the ovalbumin-specific TCR transgenic T lymphocytes:** In addition, determine whether different cell populations (see above) can be transformed into inhibitory cells with the help of the different vectors. OT-I and D011.10 T lymphocytes are harvested and stimulated with irradiated splenic stimulator cells conditioned with the respective ovalbumin peptides. To these cultures, graded numbers of cells (of different phenotypes) transduced with the different vectors are added. After approximately 5 days of culture, the T lymphocytes are harvested, counted and functionally tested. OT-I T lymphocytes are examined for their ability to specifically kill ovalbumin-coated target cells (OT-1). In the case of DO11.10, their proliferative response in the primary cultures are measured. In both situations, the fate of the responding T lymphocytes is determined. We want to determine whether they have undergone apoptosis. All studies are controlled with the different control vectors that either express only the CD8 α-chain or ovalbumin by itself.

**Determination of antigen-specific veto inhibition:** In these experiments, T lymphocytes from TCR transgenic mice (C10.4, AttM, MHC class Ib-restricted (provided by U. D. Staerz and AND, pigeon cytochrome C, MHC class II-restricted) are stimulated on peptide-coated stimulator cells in the presence of vector infected cells of the appropriate haplotype.

*In vivo* **Functional Testing of Vectors**: TCR transgenic animals represent the most straight-forward approach to surveying the activity and fate of T cells. Therefore, we chose two TCR transgenic mice, in which T cells are reactive with the model antigen, ovalbumin. One, OT-I, carries CD8+ H-2K$^b$-restricted CTLs specific for ovalbumin 257-264, the other DO11.10 CD4+ H-2A$^b$-restricted T helper cells specific for ovalbumin 329-339. The *in vivo* veto experiments are performed in chimeric mice, in which only a certain percentage of peripheral T cells is of the TCR transgenic type. These chimera are constructed in sub-lethally irradiated mice. The mice are injected with mixed BM, in which a certain number of precursors (5%) are from one or both TCR transgenic mice. A similar percentage of the peripheral T cells expressed the transgenic T cell receptor. This percentage is measured in the peripheral blood and confirmed in the lymph node that is surgically removed for this purpose. Biopsy the spleen to determine the frequency of transgenic T cells there. In addition to cell surface staining with mAbs (see above) or with MHC tetramers specific for the TCR transgene, the activation state of these T cells is determined with the help of mAbs, such as anti-CD62L, anti-CD44, anti-CD25, and anti-CD122. The functionality of these T cells are examined in standard *in vitro* activation assays, in which their ability to develop into specific CTLs or to specifically proliferate to their antigen is determined.

The different vectors and control vectors are injected most likely *i.v.* at increasing doses and possibly several times. After certain periods of time (possibly after one week and up to six months), the animals are bled. The frequency and the functional phenotype (resting versus resting) of the TCR transgenic T cells are determined. In addition, propidium iodide and annexin-5 staining are used to determine whether they are undergoing apoptosis. The animals are sacrificed at different time-points. Their spleens and lymph nodes are harvested. The number, the activation status and the viability of the recovered T lymphocytes are determined. In addition, it will be investigated whether these T cells can be induced to proliferate (DO11.10) or to develop into functional CTLs (OT-I) after specific in vitro stimulation. If the TCR transgenic T cells no longer respond, yet are still present, different T cell populations will be adoptively transferred into a secondary host of similar chimerism to determined whether regulatory T cells, such as CD4+CD25+ T cells, have been induced.

B lymphocytes, T lymphocytes, macrophages, DCs, granulocytes will the identified with the respective mAbs in conjunction with anti-CD8 α-chain mAbs to determine which cell populations have taken up the vectors and now express the CD8 α-chain. Besides spleen and lymph nodes, liver and lung are investigated. As an alternative approach to injecting the vectors directly into the chimeric mice, different cells, such as macrophages and DCs, are infected *ex vivo* and then re-injected to the mice. The T cell response to this treatment is determined as described above.

Mice that have received vectors will be injected simultaneously or with different time delays with a potent immunogen, such as the influenza virus PR/8 to determine whether the veto inhibition is specific. Approximately two weeks after the immunization, spleen cells and lymph nodes are harvested and re-stimulated *in vitro* against the virus either to examine whether proliferating CD4+ influenza-specific T cells have developed or whether influenza-specific CTLs can be induced.

### Example 6: Inhibition of the Development of Type I Diabetes in the NOD Mouse

**Production of CD8 α / Insulin 2 Vectors:** pBudCE4.1 vector (Invitrogen #V532-20) contained two multiple cloning sites (MCS): 1) CMV promoter, MCS, SV40 PA signal; 2) Elongation Factor One Alpha (EF-1 alpha), MCS, BGH PA signal. The gene for mouse CD8 α-chain was cloned into the pEF-1 alpha MCS with Kpn1 (5') and Xho1 (3') restriction sites using the following primers: Forward: 5' - CT TAT GGT ACC GCA ATG GCC TCA CCG TTG - 3'; Reverse: 5' - CG CTC CTC GAG TTA TTA CAC AAT TTT CTC - 3'. Mouse CD8 α-chain gene was first cloned into the pCR2.1 vector prior to cloning into the pBudCE4.1 vector. When the gene was cut out using Kpn1 and Xho1 enzymes, it was actually cut using the Kpn1 site from the pCR2.1 vector, resulting in the addition of around 30 nucleotides onto the 5' end of the mouse CD8 α-chain gene. These extra nucleotides were located after the promoter, but before the ATG start codon of the gene.

The gene for mouse Insulin 2 was cloned into the CMV MCS with Hind III (5') and Xba1 (3') restriction sites, using the following primers: Forward: 5' - GC TTG AAG CTT GCA ATG GCC CTG TGG ATG - 3'; Reverse: 5' - CG CTC TCT AGA TTA CTA GTT GCA GTA GTT C - 3'.

Both the mouse CD8 α-chain gene and the mouse Insulin 2 gene were sequenced and contained no mutations.

**Multiple Epitope Insulin Vectors**: In an alternative embodiment, the vector is prepared with the full-length mouse CD8 α-chain cDNA linked to the antigenic segments of the mouse insulin that encompass the major MHC class I (H-2$^d$ - B15-B23, B24-C36) and MHC class II (H-2A$^{g7}$ - B9-23)-restricted epitopes, *i.e.,* B9-C36 (Martinez, 2003; Chen, 2001; Wong, 1999; Wegmann, 1994; Wegmann, 1994; Wegmann, 1993) by a short IRES taken from the Hepatitis C virus. In addition, the respective peptides are synthesized.

In base-line experiments, it will be investigated whether T lymphocytes harvested from NOD mice at different disease stages respond to these insulin epitopes as described by others (Wegmann, 1993). In addition, it will be tested whether this insulin construct is appropriately presented. Transfer vectors that solely carry this construct are produced. They are used to transduce stimulator cells and the efficiency of recognition is determined. After these preliminary experiments have established that the respective insulin constructs are recognized, the respective vectors are produced.

**Prevention of Diabetes Development in NOD Mice:** In preliminary studies, we have found that approximately 70% of female NOD mice spontaneously develop diabetes in our animal colony within approximately 4 months. From the work of others, we know that full-blown diabetes is preceded by an insulitis at about 4 weeks of age. This insulitis is characterized by the immigration of T lymphocytes into the pancreatic islets at approximately 4 weeks of age.

In the present experiment, two groups of NOD mice were employed. Eleven mice in the control group, 15-weeks of age, were not treated. Four mice of the same age (NOD, female, Jackson Laboratories) were injected I.M. (Intramuscularly) with the pBudCE4.1 / CD8 α-chain / Insulin 2 veto vector. Injections were 50μl (1ml/mg) into both quadriceps muscles (or calf muscles) at different time points. Blood glucose levels were measured twice a week. Serum from 3 drops of blood was collected and spun out once a week to test for autoantibody production.

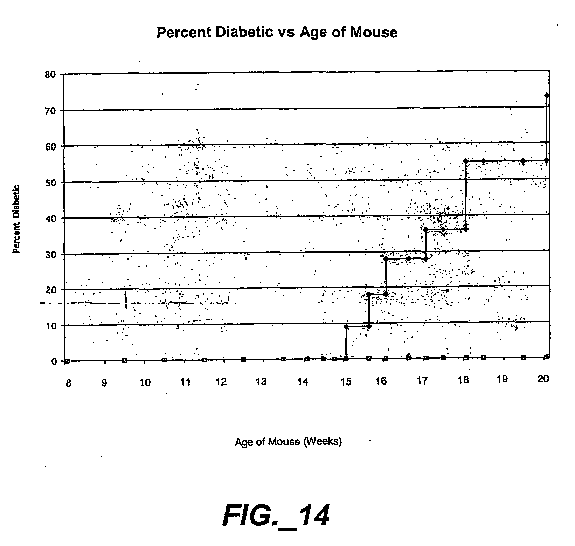

As shown in Figure 14, mice receiving the pBudCE4.1 / CD8 α-chain / Insulin 2 veto vector demonstrated a persistent reduction in the percentage of diabetic mice over a six-week period, comparing to the control group that was not treated. When the same vector was provided to NOD mice that have already developed full-blown diabetes, there were no changes in their diabetes status, suggesting that therapeutically effective insulin production via the vector is unlikely (data not shown). Further, published studies have shown that injection of vectors containing Insulin 2 and GAD past week 12 did not show any effect, whereas the instant CD8 α-chain / insulin 2 vectors were administered at week 15 and were effective. Therefore, we conclude that the co-expression of CD8 and insulin most likely on muscle cells and also on antigen presenting cells in accordance with the subject methods inhibits the activity of insulin-specific T cells required for destroying the insulin-producing islet cells.

To confirm this conclusion, the following experiment is performed, using pBudCE4.1 / CD8 α-chain / Insulin 2 veto vector (same as above) along with control plasmids pBudCE4.1 + mouseCD8 α-chain (EF-1 alpha promoter) only, PBudCE4.1 + mouse Insulin 2 (CMV Promoter) only, and empty pBudCE4.1 vector.

Female NOD mice are separated into the following groups (Table 6). They are injected with the respective vectors 50μl (1ml/mg) I.M. In contrast to the preliminary experiments, they are to receive two injections, 2 weeks apart. The blood glucose levels are measured twice a week. The level of anti-insulin antibodies are determined using RIA and ELISAs. At certain times, some mice are sacrificed. Their spleens and lymph nodes are harvested and the presence of insulin and GAD specific CD4+ and CD8+ T cells are determined using standard T cell activation assays. Histological studies are included to determine whether inflammatory processes have occurred in the pancreatic islets of these mice.

**TABLE 6**

| | Group | Treatment | Times of Treatment/weeks |
|---|---|---|---|
| 1 | Control | No Injection | - |
| 2 | CD8 Control | pBud / CD8 | 8 and 10 |
| 3 | CD8 Control | pBud / CD8 | 12 and 14 |
| 4 | Insulin | pBud / Insulin | 8 and 10 |
| 5 | Insulin | pBud / Insulin | 12 and 14 |
| 6 | Veto Vector | pBud / CD8 / Insulin | 8 and 10 |
| 7 | Veto Vector | pBud / CD8 / Insulin | 12 and 14 |

About 70 to 80% of untreated NOD mice develop diabetes within about 26 weeks of their life, a rate that was detected in the previous experiments. The injection of the veto vector by itself is not expected to significantly reduce this rate (Groups 2 and 3). Injection of Insulin 2 may reduce the incidence of diabetes to about 30% when given at weeks 8 and 10 (Group 4). When given at a later time point, group 5, no significant reduction in the incidence of diabetes is expected. When the veto vector is given early (Group 6), a significantly more pronounced reduction in the incidence of diabetes is expected as compared to Group 2. Less than 20% of animals are expected to develop diabetes. A delay in diabetes delivery is also expected. No more than 30% of animals in Group 7 (compared to more than 70% in Group 5) are expected to develop diabetes. A similar delay in diabetes onset is expected.

In another experiment, female NOD mice are injected with a CD8 α-chain / insulin vector prior to the disease onset, starting at two-weeks of age to determine whether the overt onset of diabetes is delayed, if not prevented in these animals. The peripheral blood glucose levels are measured. In addition, mice are sacrificed at different periods of time, and the pancreatic islets undergo histological examination for evidence of insulitis. The results are compared with studies in which the respective control vectors (insulin only, CD8 α-chain only) are injected.

The vector treatment is given at later stages of the disease to determine whether vectors can interfere with later stages of the disease development, such as insulitis. Finally, animals with evidence of overt diabetes are treated.

If the development of diabetes is prevented, studies are undertaken to investigate whether a concomitant inhibition of T lymphocytes will be observed. For this purpose T lymphocytes are harvested from these mice and studied for their ability to respond to insulin. Adoptive transfer experiments are included to look at any evidence of regulatory cell induction.

**Claims**

1.  A polynucleotide comprising:

    a. a first nucleic acid sequence encoding a CD8 polypeptide comprising a CD8 α-chain or a functional portion thereof having HLA binding activity, wherein said CD8 α-chain includes a transmembrane domain for associating said CD8 α-chain on a surface of said target cell;
    b. a second nucleic acid sequence encoding at least one epitope of an autoantigen associated with an autoreactive T cell response; and
    c. separate control sequences operably linked with said first and second nucleic acids for expression of said CD8 polypeptide and said autoantigen separately in the target cell.

2.  The polynucleotide according to Claim 1, wherein said first nucleic acid encodes the human CD8 α-chain.

3.  The polynucleotide according to Claims 1 or 2, wherein said autoantigen is selected from the group consisting of insulin, proteolipid protein PLP-1, myelin basic protein, myelin oligodendrocyte, glycoprotein, glutamic acid, decarboxylase 2, cholinergic receptor γ-chain, thyroglobulin, type II collagen, α1 matrix metalloproteinase, and MMP-1.

4.  A therapeutic composition for inhibiting an autoreactive T cell response against a target cell comprising an expression vector encoding a CD8 polypeptide comprising a CD8 α-chain or a functional portion thereof having HLA binding activity, wherein said CD8 α-chain includes a transmembrane domain for associating said CD8 α-chain on a surface of said target cell, and at least one epitope of an autoantigen associated with said autoreactive T cell response, wherein said expression of the CD8 polypeptide is separate from said expression of the autoantigen.

5.  The therapeutic composition according to Claim 4, wherein said expression vector encodes for multiple epitopes of said autoantigen.

6.  The therapeutic composition according to Claim 4, wherein the expression of said CD8 polypeptide and said at least one epitope of an autoantigen are under the control of the same promoter.

7.  The therapeutic composition according to any one of Claims 4 to 6, wherein said CD8 polypeptide is a human CD8 polypeptide.

8.  The therapeutic composition according to Claim 7, wherein said CD8 polypeptide consists essentially of the extracellular domain of the human CD8 α-chain and the transmembrane domain.

9.  The therapeutic composition according to any one of Claims 4 to 8, wherein said autoantigen is selected from the group consisting of insulin, proteolipid protein PLP-1, myelin basic protein, myelin oligodendrocyte, glycoprotein, glutamic acid, decarboxylase 2, cholinergic receptor γ-chain, thyroglobulin, type II collagen, α1 matrix metalloproteinase, and MMP-1.

10. An *ex vivo* method for conditioning a target cell to specifically inhibit an autoreactive immune response directed against an autoantigen expressed by the target cell comprising contacting the target cell *ex vivo* with a therapeutic composition according to any one of Claims 4 to 9.

11. Use of a therapeutic composition according to any one of Claims 4 to 9 in the manufacture of a medicament for inhibiting an autoimmune response to a target antigen.

12. The use according to Claim 11, wherein said target cell is a muscle cell.

13. The method or use according to Claim 10 or Claim 11, wherein said target cell is a hematopoietic cell.

14. The method or use according to Claim 13, wherein said target cell is a lymphocyte or an antigen-presenting cell.

15. Use of a therapeutic composition according to any one of Claims 4 to 9 in the manufacture of a medicament for preventing the development of, or for treating, an autoimmune disease in a host.

16. The therapeutic composition according to any one of Claims 4 to 9 for use in inhibiting an autoimmune response to a target antigen.

17. The therapeutic composition according to any one of Claims 4 to 9 for use in preventing the development of, or for treating, an autoimmune disease in a host.

**Patentansprüche**

1. Polynukleotid, umfassend:

   a. eine erste Nukleinsäuresequenz, die für ein CD8-Polypeptid kodiert, welches eine CD8-α-Kette oder einen funktionellen Teil davon mit HLA-bindender Aktivität umfasst, wobei die CD8-α-Kette eine transmembrane Domäne für ein Assoziieren der CD8-α-Kette auf einer Oberfläche der Zielzelle einschließt;
   b. eine zweite Nukleinsäuresequenz, die für wenigstens ein Epitop eines mit einer autoreaktiven T-Zellen-Reaktion assoziierten Autoantigens kodiert; und
   c. separate Steuerungssequenzen, die operativ mit der ersten und der zweiten Nukleinsäure verbunden sind, zur separaten Expression des CD8-Polypeptids und des Autoantigens in der Zielzelle.

2. Polynukleotid nach Anspruch 1, wobei die erste Nukleinsäure für die humane CD8-α-Kette kodiert.

3. Polynukleotid nach Anspruch 1 oder 2, wobei das Autoantigen aus der aus Insulin, Proteolipidprotein PLP-1, Myelin-basischem Protein, Myelin-Oligodendrozyt, Glykoprotein, Glutaminsäure, Decarboxylase 2, der γ-Kette des cholinergen Rezeptors, Thyroglobulin, Typ-II-Kollagen, α1-Matrixmetalloproteinase und MMP-1 bestehenden Gruppe ausgewählt ist.

4. Therapeutische Zusammensetzung zur Inhibierung einer autoreaktiven T-Zellen-Reaktion gegen eine Zielzelle, umfassend einen Expressionsvektor, welcher für ein CD8-Polypeptid kodiert, welches eine CD8-α-Kette oder einen funktionellen Teil davon mit HLA-bindender Aktivität umfasst, wobei die CD8-α-Kette eine transmembrane Domäne zum Assoziieren der CD8-α-Kette auf einer Oberfläche der Zielzelle und wenigstens ein Epitop eines mit der autoreaktiven T-Zellen-Reaktion assoziierten Autoantigens einschließt, wobei die Expression des CD8-Polypeptids getrennt von der Expression des Autoantigens erfolgt.

5. Therapeutische Zusammensetzung nach Anspruch 4, wobei der Expressionsvektor für mehrere Epitope des Autoantigens kodiert.

6. Therapeutische Zusammensetzung nach Anspruch 4, wobei die Expression des CD8-Polypeptids und des wenigstens einen Epitops eines.Autoantigens unter der Kontrolle des gleichen Promotors erfolgt.

7. Therapeutische Zusammensetzung nach einem der Ansprüche 4 bis 6, wobei es sich bei dem CD8-Polypeptid um ein humanes CD8-Polypeptid handelt.

8. Therapeutische Zusammensetzung nach Anspruch 7, wobei das CD8-Polypeptid im Wesentlichen aus der extrazellulären Domäne der humanen CD8-α-Kette und der transmembranen Domäne besteht.

9. Therapeutische Zusammensetzung nach einem der Ansprüche 4 bis 8, wobei das Autoantigen aus der aus Insulin, Proteolipidprotein PLP-1, Myelin-basischem Protein, Myelin-Oligodendrozyt, Glykoprotein, Glutaminsäure, Decarboxylase 2, der γ-Kette des cholinergen Rezeptors, Thyroglobulin, Typ-II-Kollagen, α1-Matrixmetalloproteinase und MMP-1 bestehenden Gruppe ausgewählt ist.

10. Ex-vivo-Verfahren zum Konditionieren einer Zielzelle zur spezifischen Inhibierung einer gegen ein durch die Zielzelle exprimiertes Autoantigen gerichteten autoreaktiven Immunreaktion, bei dem man die Zielzelle ex vivo mit einer therapeutischen Zusammensetzung nach einem der Ansprüche 4 bis 9 in Kontakt bringt.

11. Verwendung einer therapeutischen Zusammensetzung nach einem der Ansprüche 4 bis 9 bei der Herstellung eines Medikaments zur Inhibierung einer Autoimmunreaktion auf ein Zielantigen.

**12.** Verwendung nach Anspruch 11, wobei es sich bei der Zielzelle um eine Muskelzelle handelt.

**13.** Verfahren oder Verwendung nach Anspruch 10 oder 11, wobei es sich bei der Zielzelle um eine hämatopoietische Zelle handelt.

**14.** Verfahren nach Anspruch 13, wobei es sich bei der Zielzelle um einen Lymphozyten oder eine antigenpräsentierende Zelle handelt.

**15.** Verwendung einer therapeutischen Zusammensetzung nach einem der Ansprüche 4 bis 9 bei der Herstellung eines Medikaments zum Verhindern der Entstehung oder zur Behandlung einer Autoimmunkrankheit in einem Wirt.

**16.** Therapeutische Zusammensetzung nach einem der Ansprüche 4 bis 9 zur Verwendung bei der Inhibierung einer Autoimmunreaktion auf ein Zielantigen.

**17.** Therapeutische Zusammensetzung nach einem der Ansprüche 4 bis 9 zur Verwendung bei der Verhinderung der Entstehung oder zur Behandlung einer Autoimmunkrankheit in einem Wirt.

**Revendications**

**1.** Polynucléotide comprenant :

a. une première séquence d'acide nucléique codant pour un polypeptide CD8 comprenant une chaîne $\alpha$ du CD8 ou une portion fonctionnelle de cette dernière, ayant une activité de liaison à l'HLA, ladite chaîne $\alpha$ du CD8 comprenant un domaine transmembranaire pour association de ladite chaîne $\alpha$ du CD8 sur une surface de ladite cellule cible ;
b. une deuxième séquence d'acide nucléique codant pour au moins un épitope d'un auto-antigène associé à une réponse des cellules T auto-réactives ; et
c. des séquences régulatrices distinctes, liées d'une manière opérationnelle auxdits premier et deuxième acides nucléiques, pour expression, séparément dans la cellule cible, dudit polypeptide CD8 et dudit auto-antigène.

**2.** Polynucléotide selon la revendication 1, dans lequel le premier acide nucléique code pour la chaîne $\alpha$ du CD8 humain.

**3.** Polynucléotide selon la revendication 1 ou 2, dans lequel ledit auto-antigène est choisi dans le groupe consistant en l'insuline, la protéine protéolipide PLP-1, la protéine basique de la myéline, l'oligodendrocyte myéline, les glycoprotéines, l'acide glutamique, la décarboxylase 2, la chaîne $\gamma$ du récepteur cholinergique, la thyroglobuline, le collagène de type II, la métalloprotéinase de la matrice $\alpha$1 et le MMP-1.

**4.** Composition thérapeutique pour inhiber une réponse de cellules T auto-réactives vis-à-vis d'une cellule cible, comprenant un vecteur d'expression codant pour un polypeptide CD8 comprenant une chaîne $\alpha$ du CD8 ou une portion fonctionnelle de cette dernière ayant une activité de liaison à l'HLA, ladite chaîne $\alpha$ du CD8 comprenant un domaine transmembranaire pour association de ladite chaîne $\alpha$ du CD8 sur la surface de ladite cellule cible, et au moins un épitope d'un auto-antigène associé à ladite réponse des cellules T auto-réactives, ladite expression du polypeptide CD8 étant distincte de ladite expression de l'auto-antigène.

**5.** Composition thérapeutique selon la revendication 4, dans laquelle ledit vecteur d'expression code pour des épitopes multiples dudit auto-antigène.

**6.** Composition thérapeutique selon la revendication 4, dans laquelle l'expression dudit polypeptide CD8 et celle dudit au moins un épitope de l'auto-antigène sont sous le contrôle d'un seul et même promoteur.

**7.** Composition thérapeutique selon l'une quelconque des revendications 4 à 6, dans laquelle ledit polypeptide CD8 est un polypeptide CD8 humain.

**8.** Composition thérapeutique selon la revendication 7, dans laquelle ledit polypeptide CD8 consiste essentiellement en un domaine extracellulaire de la chaîne $\alpha$ du CD8 humain et du domaine transmembranaire.

**9.** Composition thérapeutique selon l'une quelconque des revendications 4 à 8, dans lequel ledit auto-antigène est choisi dans le groupe consistant en l'insuline, la protéine protéolipide PLP-1, la protéine basique de la myéline, l'oligodendrocyte myéline, les glycoprotéines, l'acide glutamique, la décarboxylase 2, la chaîne γ du récepteur cholinergique, la thyroglobuline, le collagène de type II, la métalloprotéinase de la matrice α1 et le MMP-1.

10. Procédé ex vivo pour le conditionnement d'une cellule cible, pour assurer une inhibition spécifique d'une réponse immune auto-réactive dirigée contre un auto-antigène exprimé par la cellule cible, comprenant la mise en contact de la cellule cible ex vivo avec une composition thérapeutique selon l'une quelconque des revendications 4 à 9.

11. Utilisation d'une composition thérapeutique selon l'une quelconque des revendications 4 à 9 pour fabriquer un médicament destiné à inhiber une réponse auto-immune à un antigène cible.

12. Utilisation selon la revendication 11, pour laquelle ladite cellule cible est une cellule musculaire.

13. Procédé ou utilisation selon la revendication 10 ou 11, ladite cellule cible étant une cellule hématopoïétique.

14. Procédé ou utilisation selon la revendication 13, ladite cellule cible étant un lymphocyte ou une cellule présentant l'antigène.

15. Utilisation d'une composition thérapeutique selon l'une quelconque des revendications 4 à 9 pour fabriquer un médicament destiné à prévenir le développement d'une maladie auto-immune chez un hôte, ou à traiter cette maladie auto-immune.

16. Composition thérapeutique selon l'une quelconque des revendications 4 à 9, pour utilisation dans le but d'inhiber une réponse auto-immune à un antigène cible.

17. Composition thérapeutique selon l'une quelconque des revendications 4 à 9, pour utilisation dans le but de prévenir le développement d'une maladie auto-immune chez un hôte ou de traiter cette maladie auto-immune.

Domains of the CD8 α-Chains

Leader
Transmembrane

Human CD8. α-Chain·

Protein:

```
MALPVTALLL PLALLLHAAR PSQFRVSPLD RTWNLGETVE LKCQVLLSNP
TSGCSWLFQP RGAAASPTFL LYLSQNKPKA AEGLDTQRFS GKRLGDTFVL
TLSDFRRENE GYYFCSALSN SIMYFSHFVP VFLPAKPTTT PAPRPPTPAP
TIASQPLSLR PEACRPAAGG AVHTRGLDFA CDIYIWAPLA GTCGVLLLSL
VITLYCNHRN RRRVCKCPRP VVKSGDKPSL SARYV
```

mRNA - coding

```
atggccttac cagtgaccgc cttgctcctg ccgctggcct·tgctgctcca
cgccgccagg ccgagccagt tccgggtgtc gccgctggat cggacctgga
acctgggcga gacagtggag ctgaagtgcc aggtgctgct gtccaacccg
acgtcgggct gctcgtggct cttccagccg cgcggcgccg ccgccagtcc
caccttcctc ctatacctct cccaaaacaa gcccaaggcg gccgaggggc
tggacaccca gcggttctcg ggcaagaggt tggggggacac cttcgtcctc
accctgagcg acttccgccg agagaacgag ggctactatt tctgctcggc
cctgagcaac tccatcatgt acttcagcca cttcgtgccg gtcttcctgc
cagcgaagcc caccacgacg ccagcgccgc gaccaccaac accggcgccc
accatcgcgt cgcagcccct gtccctgcgc ccagaggcgt gccggccagc
ggcggggggc gcagtgcaca cgagggggct ggacttcgcc tgtgatatct
acatctgggc gcccttggcc gggacttgtg gggtccttct cctgtcactg
gttatcaccc tttactgcaa ccacaggaac cgaagacgtg tttgcaaatg
tccccggcct gtggtcaaat cgggagacaa gcccagcctt tcggcgagat
acgtctaa
```

FIG._1A

**mouse CD8 α-Chain**

Protein:

```
MASPLTRFLS  LNLLLLGESI  ILGSGEAKPQ  APELRIFPKK  MDAELGQKVD
LVCEVLGSVS  QGCSWLFQNS  SSKLPQPTFV  VYMASSHNKI  TWDEKLNSSK
LFSAMRDTNN  KYVLTLNKFS  KENEGYYFCS  VISNSVMYFS  SVVPVLQKVN
STTTKPVLRT  PSPVHPTGTS  QPQRPEDCRP  RGSVKGTGLD  FACDIYIWAP
LAGICVALLL  SLIITLICYH  RSRKRVCKCP  SIACLCLKLQ  GSKWYESVIC
SALAVSIRCN  KSKSGELPLA  VHLDIRAPCK  NWEIAGSLVE  RYGKSGKHSP
LSLKAVVESN
```

mRNA Coding

```
atggcctcac  cgttgacccg  ctttctgtcg  ctgaacctgc  tgctgctggg
tgagtcgatt  atcctgggga  gtggagaagc  taagccacag  gcacccgaac
tccgaatctt  tccaaagaaa  atggacgccg  aacttggtca  gaaggtggac
ctggtatgtg  aagtgttggg  gtccgtttcg  caaggatgct  cttggctctt
ccagaactcc  agctccaaac  tcccccagcc  caccttcgtt  gtctatatgg
cttcatccca  caacaagata  acgtgggacg  agaagctgaa  ttcgtcgaaa
ctgttttctg  ccatgaggga  cacgaataat  aagtacgttc  tcaccctgaa
caagttcagc  aaggaaaacg  aaggctacta  tttctgctca  gtcatcagca
actcggtgat  gtacttcagt  tctgtcgtgc  cagtccttca  gaaagtgaac
tctactacta  ccaagccagt  gctgcgaact  ccctcacctg  tgcaccctac
cgggacatct  cagccccaga  gaccagaaga  ttgtcggccc  cgtggctcag
tgaaggggac  cggattggac  ttcgcctgtg  atatttacat  ctgggcaccc
ttggccggaa  tctgcgtggc  ccttctgctg  tccttgatca  tcactctcat
ctgctaccac  aggagccgaa  agcgtgtttg  caaatgtccc  agtatagcat
gcttgtgcct  caaactgcaa  ggaagcaagt  ggtatgaatc  tgtgatctgc
tcagctctgg  ctgtgagcat  cagatgtaac  aaatcaaagt  caggagaact
gcctttagcg  gtgcacctgg  acatcagagc  cccttgtaag  aactgggaaa
ttgctggcag  tctagtggag  cggtacggta  aatctggaaa  acactcccct
ctgtcactga  aggctgtagt  agaatccaat  taa
```

*FIG._1B*

FIG._2A

FIG._2B

FIG._3

FIG._4A

FIG._4B

FIG._5A

FIG._5B

FIG._6

FIG._8

FIG._7A

FIG._7B

*FIG._9A*

*FIG._9B*

*FIG._9C*

*FIG._9D*

*FIG._10A*

*FIG._10B*

*FIG._10C*

*FIG._10D*

*FIG._10E*

FIG._11

FIG._12

FIG._13A

FIG._13B

## Percent Diabetic vs Age of Mouse

Age of Mouse (Weeks)

## FIG._14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030148983 A1
- US 5242687 A
- US 5601828 A
- US 5623056 A
- WO 02102852 A
- US 6211342 B
- US 5672346 A
- US 6143292 A
- US 6534052 B
- US 6734014 B
- US 6783981 B
- US 6762031 B
- US 6440735 B
- US 4518584 A
- US 4737462 A

- US 5605703 A
- US 5811238 A
- US 5873458 A
- US 5830696 A
- US 5939250 A
- US 5763239 A
- US 5965408 A
- US 5945325 A
- WO 9526412 A
- WO 9531566 A
- US 6156497 A
- US 6228646 B
- US 9701019 W
- US 9701048 W
- US 6531456 B

### Non-patent literature cited in the description

- **BERG et al.** *J. Exp. Med.,* 1999, vol. 194, 427-38
- **ZEPP et al.** *Nature,* 1988, vol. 336, 473-5
- **JORDAN et al.** *Nat. Immunol.,* 2001, vol. 2, 301-6
- **STEPHENS et al.** *Eur. J. Immunol.,* 2003, vol. 33, 1282-91
- **ASSEMAN et al.** *J. Exp. Med.,* 1999, vol. 190, 995-1004
- **SEDDON et al.** *J. Exp. Med.,* 1999, vol. 189, 279-88
- **WICKER et al.** *Ann. Rev. Immunol.,* 1995, vol. 12, 179-200
- **MORA et al.** *J. Immuno/.,* 1996, vol. 162, 4576-88
- **RUIZ et al.** *J. Immunol.,* 1999, vol. 162, 3336-3341
- **GARREN et al.** *Immunity,* 2001, vol. 15, 15-22
- **ROBINSON et al.** *Nature Biotech.,* 2003, vol. 21, 1033-39
- **BOT et al.** *J. Immuno/.,* 2001, vol. 167, 2950-55
- **MILLER.** *Science,* 1991, vol. 252, 1424-1427
- **RAMMENSEE et al.** *Eur. J. Immunol.,* 1982, vol. 12, 930-934
- **FINK et al.** *J. Exp. Med.,* 1983, vol. 157, 141-154
- **RAMMENSEE et al.** *J. Immuno/.,* 1984, vol. 132, 668-672

- **HAMBOR et al.** *J. Immunol.,* 1990, vol. 145, 1646-1652
- **HAMBOR et al.** *Intern. Immuno/.,* 1990, vol. 2, 8856-8879
- **KAPLAN et al.** *Proc. Natl. Acad. Sci. USA,* 1989, vol. 86, 8512-8515
- **QI et al.** *J. Exp. Med.,* 1996, vol. 183, 1973-1980
- **STAERZ.** *Immunol Today,* 2000, vol. 21 (4), 172-6
- **MORTLOCK et al.** *Nuc. Acids. Res.,* 2003, vol. 31, 152
- **MIZUGUCHI et al.** *Hum. Gene Ther.,* 2003, vol. 14, 1265-77
- *Nature Medicine,* August 2001, vol. 7 (8), 899-905
- *J. Neurosci Res.,* 01 February 2001, vol. 63 (3), 290-302
- *J Neurosci Res.,* February 2001, vol. 63 (3), 290-302
- *J. Neurochem.,* 2004, vol. 88 (5), 1211-1219
- *J. Biol. Chem.,* 1994, vol. 269 (50), 31725-31730
- *J. Neuropathol. Exp. Neurol.,* 2003, vol. 62 (1), 25-33
- *J. Neurosci.,* 1989, vol. 24 (2), 137-142
- *J. Neurosci. Res.,* 2004, vol. 75 (4), 516-523
- *Proc Natl Acad Sci USA,* 18 August 2003, vol. 100 (18), 10376-81

- *J. Clin. Invest.,* 15 June 1996, vol. 97 (12), 2772-83
- *J. Biol. Chem,* 11 July 2003, vol. 278 (28), 26166-73
- *J. Autoimmun.,* August 1997, vol. 1 (4), 387-94
- *Ann NY Acad Sci.,* September 2003, vol. 998, 284-307
- *Int Rev Immunol.,* 2000, vol. 19 (6), 501-33
- *J Rheumatol.,* March 2000, vol. 27 (3), 589-93
- *Genomics,* 1997, vol. 43 (2), 221-225
- *J Rheumatol.,* June 2003, vol. 30 (6), 1147-56
- *J. Biol. Chem.,* 2002, vol. 277 (37), 34109-34116
- **MEINL et al.** *J Clin Invest,* 1993, vol. 92, 2633-43
- **WUCHERPFENNIG et al.** *J Clin Invest,* 1997, vol. 100, 1114-1122
- **KERLERO DE ROSBO et al.** *Eur J Immunol,* 1997, vol. 27, 3059-69
- **OKSENBERG et al.** *Nature,* 1993, vol. 362, 68-70
- **WUCHERPFENNIG et al.** *J Clin Invest,* 1997, vol. 100, 1114-22
- **HOLZ et al.** *J Immunol,* 2000, vol. 164, 1103-9
- **GENAIN ; HAUSER.** *Methods,* 1996, vol. 10, 420-34
- **GERSHWIN et al.** *Immunol Rev,* 2000, vol. 174, 210-225
- **MACKAY et al.** *Immunol Rev,* 2000, vol. 174, 226-237
- **JONES.** *J Clin Pathol,* 2000, vol. 53, 813-21
- **CUMMINGS et al.** *Neurology,* 1998, vol. 51, 2-1765-7
- **IWATSUBO et al.** *Neuron,* 1994, vol. 13, 45-53
- **LIPPA et al.** *Lancet,* 1998, vol. 352, 1117-1118
- **SINHA et al.** *Ann N Y Acad Sci,* 2000, vol. 920, 206-8
- **FARLOW ; EVANS.** *Neurology,* 1998, vol. 51, 36-4465-7
- **HAKE.** *Cleve Clin J Med,* 2001, vol. 68, 608-9613-4616
- **GAMES et al.** *Nature,* 1995, vol. 373, 523-527
- **HSIAO et al.** *Science,* 1996, vol. 274, 99-102
- **MORGAN et al.** *Nature,* 2000, vol. 408, 982-985
- **SCHENK et al.** *Nature,* 1999, vol. 400, 173-177
- **BARD et al.** *Nat Med,* 2000, vol. 6, 916-19
- **DEMATTOS et al.** *Proc Natl Acad Sci U S A,* 2001, vol. 98, 8850-8855
- **SCHRAG et al.** *Bmj,* 2000, vol. 321, 21-22
- **FOMO ; NORVILLE.** *Acta Neuropathol (Berl,* 1976, vol. 34, 183-197
- **DICKSON.** *Curr Opin Neurol,* 2001, vol. 14, 423-432
- *Cell,* 1993, vol. 72, 971-983
- **DIFIGLIA et al.** *Science,* 1997, vol. 277, 1990-1993
- **SCHERZINGER et al.** *Cell,* 1997, vol. 90, 549-558
- **DAVIES et al.** *Cell,* 1997, vol. 90, 537-548
- **PRUSINER.** *Proc Natl Acad Sci U S A,* 1998, vol. 95, 13363-83
- **REIZES et al.** *Cell,* 2001, vol. 106, 105-116
- **CHIESI et al.** *Trends Pharmacological Sciences,* 2001, vol. 22, 247-54
- **TANSEY et al.** *Proc. Natl. Acad. Sci. USA,* vol. 98, 6494-99
- **HUBER ; SCHWAB.** *Biol Chem,* 2000, vol. 381, 407-19
- **REILLY.** *J Neurol,* 2000, vol. 247, 239-40
- **CHEN et al.** *Nature,* 2000, vol. 403, 434-9
- **RAINETEAU et al.** *Proc Natl Acad Sci U S A,* 2001, vol. 98, 6929-34
- **MERKLER et al.** *J Neurosci,* 2001, vol. 21, 3665-73
- **BLOCHLINGER et al.** *J Comp Neurol,* 2001, vol. 433, 426-36
- **BROSAMLE et al.** *J Neurosci,* 2000, vol. 20, 8061-8
- **MOTTEZ et al.** *J. Exp. Med.,* 1995, vol. 181, 493-502
- **KOZONO et al.** *nature,* 1994, vol. 369, 151-154
- **WHITE et al.** *J. Immunol.,* 1999, vol. 162, 2671-76
- Vector Targeting for Therapeutic Gene Delivery. Wiley-Uss, Inc, 2002
- **ARTHUR et al.** *Cancer Gene Ther.,* 1997, vol. 4, 17-25
- **HENDERSON et al.** *Cancer Res.,* 1996, vol. 56, 3763-3770
- **RIBAS et al.** *Cancer Res.,* 1997, vol. 57, 2865-2869
- **ASHLEY et al.** *J. Exp. Med.,* 1997, vol. 186, 1177-1182
- **DEVEREUX et al.** *Nucleic Acids Res.,* 1984, vol. 12, 387
- **WALDER et al.** *Gene,* 1986, vol. 42, 133
- **BAUER et al.** *Gene,* 1985, vol. 37, 73
- **CRAIK.** *Biotechniques,* January 1995, 12-19
- **LEAHY.** *Faseb J.,* 1995, vol. 9, 17-25
- **LEAHY et al.** *Cell,* 1992, vol. 68, 1145-62
- **NAKAYAMA et al.** *Immunogenetics,* 1989, vol. 30, 393-7
- **GAO ; JAKOBSEN.** *Immunology Today,* 2000, vol. 21, 630-636
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *PNAS USA,* 1988, vol. 85, 2444

- **DEVEREUX et al.** *Nucl. Acid Res.,* 1984, vol. 12, 387-395
- Current Methods in Sequence Comparison and Analysis. Macromolecule Sequencing and Synthesis, Selected Methods and Applications. Alan R. Liss, Inc, 1988, 127-149
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *PNAS USA,* 1993, vol. 90, 5873-5787
- **ALTSCHUL et al.** *Methods in Enzymology,* 1996, vol. 266, 460-480, http://blast.wustl/edu/blast/ README.html
- **ALTSCHUL et al.** *Nucleic Acids Res.,* vol. 25, 3389-3402
- **LUCKOW et al.** *Bio/Technology,* 1988, vol. 6, 47
- **COTTEN et al.** *PNAS (USA,* 1992, vol. 89, 6094-6098
- **CURIEL et al.** *PNAS (USA,* 1991, vol. 88, 8850-8854
- **WAGNER et al.** *PNAS (USA,* 1992, vol. 89, 6099-6103
- **YEH ; PERRICAUDET.** *FASEB J.,* 1997, vol. 11, 615
- **BYUN et al.** *Gene Ther.,* 1996, vol. 3 (9), 780-8
- **SIOUD et al.** *J. Mol. Biol.,* vol. 242, 831-835
- **ROSENFELD et al.** *Science,* 1991, vol. 252, 431-434
- **JAFFE et al.** *Clin. Res.,* 1991, vol. 39 (2), 302A
- **ROSENFELD et al.** *Clin. Res.,* 1991, vol. 39 (2), 311A
- **BERKNER.** *BioTechniques,* 1988, vol. 6, 616-629
- **CEMAN.** *J Immunol.,* 01 August 1992, vol. 149 (3), 754-61
- **MARTIN.** *J Immunogenet.,* February 1990, vol. 17 (1-2), 151-9